# EUROPEAN PATENT SPECIFICATION

(11) **EP 3 280 418 B1**
(45) Date of publication and mention of the grant of the patent: **06.05.2020**
(21) Application number: 16777094.0
(22) Date of filing: 04.04.2016
(51) Int. Cl.: A61K 31/5377, A61K 31/55, C07D 487/04, A61K 31/541

(54) **AZACARBAZOLE BTK INHIBITORS**
AZACARBAZOL-BTK-HEMMER
INHIBITEURS DE BTK AZACARBAZOLE

(30) Priority: 08.04.2015 WO PCT/CN2015/076040
(43) Date of publication of application: 14.02.2018
(73) Proprietor: Merck Sharp & Dohme Corp., Rahway, NJ 07065-0907 (US)
(72) Inventor: LIU, Jian, Rahway, New Jersey 07065-0907 (US); KOZLOWSKI, Joseph, Rahway, New Jersey 07065-0907 (US); KIM, Ronald, Rahway, New Jersey 07065-0907 (US); GAO, Xiaolei, Rahway, New Jersey 07065-0907 (US); BOGA, Sobhana Babu, Rahway, New Jersey 07065-0907 (US); YU, Younong, Rahway, New Jersey 07065-0907 (US); WU, Hao, Shanghai 200131 (CN); LIU, Shilan, Shanghai 200131 (CN); YANG, Chundao, Shanghai 200131 (CN)
(74) Representative: Jaap, David Robert
(86) International application number: PCT/US2016/025808
(87) International publication number: WO 2016/164284

(56) References cited:
- WO-A1-2009/075830
- WO-A2-2007/061764
- US-A1- 2010 048 551
- US-A1- 2011 230 464
- US-A1- 2013 096 118

## Description

### FIELD OF THE INVENTION

The present invention relates to Btk inhibitor compounds, to pharmaceutical compositions comprising these compounds and to their use in therapy. In particular, the present invention relates to the use of Btk inhibitor compounds in the treatment of Bruton's Tyrosine Kinase (Btk) mediated disorders.

### BACKGROUND OF THE INVENTION

B lymphocyte activation is important in the generation of adaptive immune responses. Derailed B lymphocyte activation is a hallmark of many autoimmune diseases and modulation of this immune response is therefore of therapeutic interest. Recently the success of B cell therapies in autoimmune diseases has been established. Treatment of rheumatoid arthritis (RA) patients with Rituximab (anti-CD20 therapy) is an accepted clinical therapy by now. More recent clinical trial studies show that treatment with Rituximab also ameliorates disease symptoms in relapsing remitting multiple sclerosis (RRMS) and systemic lupus erythematosus (SLE) patients. This success supports the potential for future therapies in autoimmune diseases targeting B cell immunity.

Bruton tyrosine kinase (Btk) is a Tec family non-receptor protein kinase, expressed in B cells and myeloid cells. The function of Btk in signaling pathways activated by the engagement of the B cell receptor (BCR) and FcεR1 on mast cells is well established. In addition, a function for Btk as a downstream target in Toll-like receptor signaling was suggested. Functional mutations in Btk in human results in the primary immunodeficiency disease called XLA which is characterized by a defect in B cell development with a block between pro- and pre-B cell stages. This results in an almost complete absence of B lymphocytes in humans causing a pronounced reduction of serum immunoglobulin of all classes. These finding support the key role for Btk in the regulation of the production of auto-antibodies in autoimmune diseases. In addition, regulation of Btk may affect BCR-induced production of pro-inflammatory cytokines and chemokines by B cells, indicating a broad potential for Btk in the treatment of autoimmune diseases.

With the regulatory role reported for Btk in FcεR-mediated mast cell activation, Btk inhibitors may also show potential in the treatment of allergic responses [Gilfillan et al, Immunological Reviews 288 (2009) pp149-169].

Furthermore, Btk is also reported to be implicated in RANKL-induced osteoclast differentiation [Shinohara et al, Cell 132 (2008) pp794-806] and therefore may also be of interest for the treatment of bone resorption disorders.

Other diseases with an important role for dysfunctional B cells are B cell malignancies. Indeed anti-CD20 therapy is used effectively in the clinic for the treatment of follicular lymphoma, diffuse large B-cell lymphoma and chronic lymphocytic leukemia [Lim et al, Haematologica, 95 (2010) pp135-143]. The reported role for Btk in the regulation of proliferation and apoptosis of B cells indicates there is potential for Btk inhibitors in the treatment of B cell lymphomas as well. Inhibition of Btk seems to be relevant in particular for B cell lymphomas due to chronic active BCR signaling [Davis et al, Nature, 463 (2010) pp88-94].

Some classes of Btk inhibitor compounds have been described as kinase inhibitors, e.g.,Imidazo[1,5-f][1,2,4]triazine compounds have been described in WO2005097800 and WO2007064993. Imidazo[1,5-a]pyrazine compounds have been described in WO2005037836 and WO2001019828 as IGF-1R enzyme inhibitors.

Some of the Btk inhibitors reported are not selective over Src-family kinases. With dramatic adverse effects reported for knockouts of Src-family kinases, especially for double and triple knockouts, this is seen as prohibitive for the development of Btk inhibitors that are not selective over the Src-family kinases.

Both Lyn-deficient and Fyn-deficient mice exhibit autoimmunity mimicking the phenotype of human lupus nephritis. In addition, Fyn-deficient mice also show pronounced neurological defects. Lyn knockout mice also show an allergic-like phenotype, indicating Lyn as a broad negative regulator of the IgE-mediated allergic response by controlling mast cell responsiveness and allergy-associated traits [Odom et al, J. Exp. Med., 199 (2004) pp1491-1502]. Furthermore, aged Lyn knock-out mice develop severe splenomegaly (myeloid expansion) and disseminated monocyte/macrophage tumors [Harder et al, Immunity, 15 (2001) pp603-615]. These observations are in line with hyperresponsive B cells, mast cells and myeloid cells, and increased Ig levels observed in Lyn-deficient mice. Female Src knockout mice are infertile due to reduced follicle development and ovulation [Roby et al, Endocrine, 26 (2005) pp169-176]. The double knockouts Src^{-/-}Fyn^{-/-} and Src^{-/-}Yes^{-/-} show a severe phenotype with effects on movement and breathing. The triple knockouts Src^{-/-}Fyn^{-/-}Yes^{-/-} die at day 9.5 [Klinghoffer et al, EMBO J., 18 (1999) pp2459-2471]. For the double knockout Src^{-/-}Hck^{-/-}, two thirds of the mice die at birth, with surviving mice developing osteopetrosis, extramedullary hematopoiseis, anemia, leukopenia [Lowell et al, Blood, 87 (1996) pp1780-1792].

Hence, an inhibitor that inhibits multiple or all kinases of the Src-family kinases simultaneously may cause serious adverse effects.

### SUMMARY OF THE INVENTION

The present invention provides compounds which inhibit Btk activity, their use for treatment of Btk mediated diseases and disorders, in particular autoimmune diseases and inflammatory diseases, as well as pharmaceutical compositions comprising such compounds and pharmaceutical carriers.

### DETAILED DESCRIPTION

### Definitions

The terms used herein have their ordinary meaning and the meaning of such terms is independent at each occurrence thereof. That notwithstanding and except where stated otherwise, the following definitions apply throughout the specification and claims. Chemical names, common names, and chemical structures may be used interchangeably to describe the same structure. If a chemical compound is referred to using both a chemical structure and a chemical name, and an ambiguity exists between the structure and the name, the structure predominates. These definitions apply regardless of whether a term is used by itself or in combination with other terms, unless otherwise indicated. Hence, the definition of "alkyl" applies to "alkyl" as well as the "alkyl" portions of "hydroxyalkyl," "fluoroalkyl," "-O-alkyl," etc.

As used herein, and throughout this disclosure, the following terms, unless otherwise indicated, shall be understood to have the following meanings:
A "patient" is a human or non-human mammal. In one embodiment, a patient is a human. In another embodiment, a patient is a chimpanzee.

The term "therapeutically effective amount" as used herein refers to an amount of the compound of Formula (I) and/or an additional therapeutic agent, or a composition thereof that is effective in producing the desired therapeutic, ameliorative, inhibitory, or preventative effect when administered to a patient suffering from a disease or condition mediated by Btk. In the combination therapies of the present invention, a therapeutically effective amount can refer to each individual agent or to the combination as a whole, wherein the amounts of all agents administered are together effective, but wherein the component agent of the combination may not be present individually in an effective amount.

The term "preventing," as used herein with respect to cancer or an inflammatory disease or disorder, refers to reducing the likelihood of an autoimmune or inflammatory disease or disorder.

The term "alkyl," as used herein, refers to an aliphatic hydrocarbon group having one of its hydrogen atoms replaced with a bond having the specified number of carbon atoms. In different embodiments, an alkyl group contains from 1 to 6 carbon atoms (C₁₋₆alkyl) or from 1 to 3 carbon atoms (C₁₋₃alkyl). Non-limiting examples of alkyl groups include methyl, ethyl, *n-*propyl, isopropyl, *n*-butyl, sec-butyl, isobutyl, *tert*-butyl, *n*-pentyl, neopentyl, isopentyl, n-hexyl, isohexyl, and neohexyl. In one embodiment, an alkyl group is linear. In another embodiment, an alkyl group is branched.

The term "fluoroalkyl," as used herein, refers to an alkyl group as defined above, wherein one or more of the alkyl group's hydrogen atoms has been replaced with a fluorine. In one embodiment, a fluoroalkyl group has from 1 to 6 carbon atoms (C₁₋₆fluoroalkyl). In another embodiment, a fluoroalkyl group has from 1 to 3 carbon atoms (C₁₋₃fluoroalkyl). In another embodiment, a fluoroalkyl group is substituted with from 1 to 3 fluorine atoms. Non-limiting examples of fluoroalkyl groups include -CH₂F, -CHF₂, and -CF₃. The term "C₁-C₃ fluoroalkyl" refers to a fluoroalkyl group having from 1 to 3 carbon atoms.

The term "alkylene," as used herein, refers to an alkyl group, as defined above, wherein one of the alkyl group's hydrogen atoms has been replaced with a bond. Non-limiting examples of alkylene groups include -CH₂-, -CH₂CH₂-, -CH₂CH₂CH₂-, -CH₂CH₂CH₂CH₂-, -CH(CH₃)CH₂CH₂-, -CH(CH₃)-, and -CH₂CH(CH₃)CH₂-. In one embodiment, an alkylene group has from 1 to about 6 carbon atoms (C₁₋₆alkylene). In another embodiment, an alkylene group has from 1 to 3 carbon atoms (C₁₋₃alkylene). In another embodiment, an alkylene group is branched. In another embodiment, an alkylene group is linear. In one embodiment, an alkylene group is -CH₂-. Unless otherwise indicated, an alkylene group is unsubstituted.

The term "alkenyl," as used herein, refers to an aliphatic hydrocarbon group containing at least one carbon-carbon double bond and having one of its hydrogen atoms replaced with a bond. An alkenyl group may be straight or branched and contain from about 2 to about 15 carbon atoms. In one embodiment, an alkenyl group contains from about 2 to 4 carbon atoms. Non-limiting examples of alkenyl groups include ethenyl, propenyl, n-butenyl, 3-methylbut-2-enyl, n-pentenyl, octenyl, and decenyl. The term "C₂₋₆alkenyl" refers to an alkenyl group having from 2 to 6 carbon atoms. The term "C₂₋₄alkenyl" refers to an alkenyl group having from 2 to 4 carbon atoms. Unless otherwise indicated, an alkenyl group is unsubstituted.

The term "alkoxy," as used herein, refers to an -O-alkyl group, wherein an alkyl group is as defined above. Non-limiting examples of alkoxy groups include methoxy, ethoxy, n-propoxy, isopropoxy, *n*-butoxy, and *tert*-butoxy. An alkoxy group is bonded via its oxygen atom to the rest of the molecule.

The term "aryl," as used herein, refers to an aromatic monocyclic or multicyclic ring system comprising from about 6 to about 14 carbon atoms. In one embodiment, an aryl group contains from about 6 to 10 carbon atoms (C₆-C₁₀aryl). In another embodiment an aryl group is phenyl. Non-limiting examples of aryl groups include phenyl and naphthyl.

The term "cycloalkyl," as used herein, refers to a saturated ring containing the specified number of ring carbon atoms, and no heteroatom. In a like manner the term "C₃₋₆cycloalkyl" refers to a saturated ring having from 3 to 6 ring carbon atoms. Non-limiting examples of monocyclic cycloalkyls include cyclopropyl, cyclobutyl, cyclopentyl, and cyclohexyl.

The term "halo," as used herein, means -F, -Cl, -Br or -I. In one embodiment, a halo group is -F or -Cl. In another embodiment, a halo group is -F.

The term "heteroaryl," or "heteroaryl ring," as used herein, refers to an aromatic monocyclic or multicyclic ring system comprising about 5 to about 14 ring atoms, wherein from 1 to 3 of the ring atoms is independently N, O, or S and the remaining ring atoms are carbon atoms. In one embodiment, a heteroaryl group has 5 to 10 ring atoms. In another embodiment, a heteroaryl group is monocyclic ring system and has 5 or 6 ring atoms. In another embodiment, a heteroaryl group is a bicyclic ring system. A heteroaryl group is joined via a ring carbon atom. The term "heteroaryl" also includes a heteroaryl as defined above fused to a heterocycle as defined below. The term "heteroaryl" also encompasses a heteroaryl group, as defined above, which is fused to a benzene, a cyclohexadiene or a cyclohexene ring. Non-limiting examples of heteroaryls include pyridyl, pyrazinyl, furanyl, thienyl, pyrimidinyl, pyridone (including N-substituted pyridones), isoxazolyl, isothiazolyl, oxazolyl, oxadiazolyl, thiazolyl, pyrazolyl, furyl, pyrrolyl, triazolyl, 1,2,4-thiadiazolyl, pyrazinyl, pyridazinyl, indolyl, quinoxalinyl, phthalazinyl, oxindolyl, imidazo[1,2-a]pyridinyl, imidazo[2,1-b]thiazolyl, and the like. In one embodiment, a heteroaryl group is a 5-membered heteroaryl. In another embodiment, a heteroaryl group is a 6-membered heteroaryl.

The term "heterocyclyl" or "heterocyclic ring"," as used herein, refers to a nonaromatic saturated or partially saturated monocyclic or multicyclic ring system containing 3 to 11 ring atoms, wherein from 1 to 4 of the ring atoms are independently O, S, or N, and the remainder of the ring atoms are carbon atoms. In one embodiment, a heterocyclyl group is monocyclic and has from 3 to 7 ring atoms. In another embodiment, a heterocyclyl group is monocyclic and has from about 4 to 7 ring atoms. In another embodiment, a heterocyclyl group is bicyclic and has from 7 to 11 ring atoms. In still another embodiment, a heterocyclyl group is monocyclic and has 5 or 6 ring atoms. In one embodiment, a heterocyclyl group is monocyclic. In another embodiment, a heterocyclyl group is bicyclic. A heterocyclyl group can be joined to the rest of the molecule via a ring carbon or ring nitrogen atom. The nitrogen or sulfur atom of the heterocyclyl can be optionally oxidized to the corresponding N-oxide, S-oxide or S,S-dioxide. Non-limiting examples of monocyclic heterocyclyl rings include oxetanyl, piperidinyl, pyrrolidinyl, piperazinyl, morpholinyl, thiomorpholinyl, thiazolidinyl, dihydropyranyl, pyran, 1,4-dioxanyl, tetrahydrofuranyl, tetrahydrothiophenyl, delta-lactam, delta-lactone, and the like.

The term "substituted" means that one or more hydrogens on the designated atom/atoms is/are replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound" or "stable structure" is meant a compound that is sufficiently robust to survive isolation from a reaction mixture to a useful degree of purity, and formulation into an efficacious therapeutic agent.

The term "optionally substituted" means that a compound may or may not be substituted with the specified groups, radicals or moieties.

A "subject" is a human or non-human mammal. In one embodiment, a subject is a human. In another embodiment, the subject is a chimpanzee, dog, or cat.

When any substituent or variable occurs more than one time in any constituent or the compound of Formula (I), its definition on each occurrence is independent of its definition at every other occurrence, unless otherwise indicated. For example, description of radicals which include the expression "-N(C₁₋₃alkyl)2" means -N(CH₃)(CH₂CH₃), -N(CH₃)(CH₂CH₂CH₃), and
-N(CH₂CH₃)(CH₂CH₂CH₃), as well as -N(CH₃)₂, -N(CH₂CH₃)₂, and -N(CH₂CH₂CH₃)₂.

The term "in purified form," as used herein, refers to the physical state of a compound after the compound is isolated from a synthetic process (*e.g.,* from a reaction mixture), a natural source, or a combination thereof. The term "in purified form," also refers to the physical state of a compound after the compound is obtained from a purification process or processes described herein or well-known to the skilled artisan (*e.g.,* chromatography, recrystallization and the like), in sufficient purity to be characterizable by standard analytical techniques described herein or well-known to the skilled artisan.
It should also be noted that any carbon as well as heteroatom with unsatisfied valences in the text, schemes, examples and tables herein is assumed to have the sufficient number of hydrogen atom(s) to satisfy the valences.

One or more compounds of the invention may exist in unsolvated as well as solvated forms with pharmaceutically acceptable solvents such as water, ethanol, and the like, and it is intended that the invention embrace both solvated and unsolvated forms. "Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of suitable solvates include ethanolates, methanolates, and the like. "Hydrate" is a solvate wherein the solvent molecule is H₂O.

The compounds of Formula (I) may contain one or more stereogenic centers and can thus occur as racemates, racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within the ambit of this invention. Any formulas, structures or names of compounds described in this specification that do not specify a particular stereochemistry are meant to encompass any and all existing isomers as described above and mixtures thereof in any proportion. When stereochemistry is specified, the invention is meant to encompass that particular isomer in pure form or as part of a mixture with other isomers in any proportion.
Diastereomeric mixtures can be separated into their individual diastereomers on the basis of their physical chemical differences by methods well known to those skilled in the art, such as, for example, by chromatography and/or fractional crystallization. Enantiomers can be separated by converting the enantiomeric mixture into a diastereomeric mixture by reaction with an appropriate optically active compound (*e.g.,* chiral auxiliary such as a chiral alcohol or Mosher's acid chloride), separating the diastereomers and converting (*e.g.,* hydrolyzing) the individual diastereomers to the corresponding pure enantiomers. Also, some of the compounds of Formula (I) may be atropisomers (*e.g.,* substituted biaryls) and are considered as part of this invention. Enantiomers can also be separated by use of chiral HPLC column.

It is also possible that the compounds of Formula (I) may exist in different tautomeric forms, and all such forms are embraced within the scope of the invention.

All stereoisomers (for example, geometric isomers, optical isomers and the like) of the present compounds (including those of the salts and solvates of the compounds as well as the salts, solvates and esters of the prodrugs), such as those which may exist due to asymmetric carbons on various substituents, including enantiomeric forms (which may exist even in the absence of asymmetric carbons), rotameric forms, atropisomers, and diastereomeric forms, are contemplated within the scope of this invention. Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the *IUPAC* 1974 Recommendations.

The compounds of Formula (I) can form salts which are also within the scope of this invention. Reference to a compound of Formula (I) herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a compound of Formula (I) contains both a basic moiety, such as, but not limited to a pyridine or imidazole, and an acidic moiety, such as, but not limited to a carboxylic acid, zwitterions ("inner salts") may be formed and are included within the term "salt(s)" as used herein. Such acidic and basic salts used within the scope of the invention are pharmaceutically acceptable (*i.e.,* non-toxic, physiologically acceptable) salts. Salts of the compounds of Formula (I) may be formed, for example, by reacting a compound of Formula (I) with an amount of acid or base, such as an equivalent amount, in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates,), 1-hydroxy -2-naphthoates (also known as xinafoates) and the like. Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their website). These disclosures are incorporated herein by reference. Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamines, t-butyl amines, and salts with amino acids such as arginine, lysine and the like. Basic nitrogen-containing groups may be quartemized with agents such as lower alkyl halides (*e.g.,* methyl, ethyl, and butyl chlorides, bromides and iodides), dialkyl sulfates (*e.g.,* dimethyl, diethyl, and dibutyl sulfates), long chain halides (*e.g.,* decyl, lauryl, and stearyl chlorides, bromides and iodides), aralkyl halides (*e.g.,* benzyl and phenethyl bromides), and others.
The present invention further includes the compounds of Formula (I) in all their isolated forms. For example, the above-identified compounds are intended to encompass all forms of the compounds such as, any solvates, hydrates, stereoisomers, and tautomers thereof.

As used herein, the term "composition" is intended to encompass a product comprising the specified ingredients in the specified amounts, as well as any product which results, directly or indirectly, from combination of the specified ingredients in the specified amounts.

In the compounds of generic Formula (I), the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds of generic Formula (I). For example, different isotopic forms of hydrogen (H) include protium (¹H) and deuterium (2H). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched compounds within generic Formula (I) can be prepared without undue experimentation by conventional techniques well known to those skilled in the art or by processes analogous to those described in the examples herein using appropriate isotopically-enriched reagents and/or intermediates.

In the above definitions with multifunctional groups, the attachment point is at the last group, unless otherwise specified on the substituent group by a dash. A dash on the substituent group would then represent the point of attachment.

It should be noted that any carbon as well as heteroatom with unsatisfied valences in the text, schemes, examples and tables herein is assumed to have the sufficient number of hydrogen atom(s) to satisfy the valences.

### Compounds of the Invention

In embodiment no. 1, the present invention provides compounds according to Formula (I) 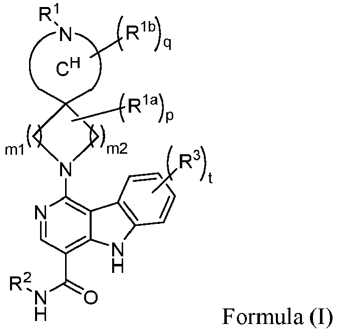 wherein:
ring C^{H} is a 4- to 6-membered saturated heterocyclic ring, wherein said heterocyclic ring optionally contains one additional heteroatom group which is N(H) or O;
the subscripts m1 and m2 are independently 0, 1, 2, or 3, with the proviso that 2 ≤ m1+m2 ≤ 5;
R¹ is:
   (a.) H;
   (b.) C₁₋₃alkyl, wherein said C₁₋₃alkyl of R¹ is unsubstituted or substituted by -C(O)O-R^{1e}, wherein R^{1e} is H or C₁₋₆alkyl;
   (c.) -C(O)O-R^{1c} wherein R^{1c} is C₁₋₆alkyl;
   (d.) -C(O)-R^{1d} wherein R^{1d} is C₁₋₃alkyl or phenyl, wherein said phenyl of R^{1d} is unsubstituted or substituted by 1 to 2 substituents selected from C₁₋₆alkyl, halo, or C₁₋₃alkoxy; or
   (e.) phenyl, wherein said phenyl of R¹ is unsubstituted or substituted by 1 to 2 substituents selected from C₁₋₆alkyl, halo, or C₁₋₃alkoxy;
R^{1a} is C₁₋₃alkyl, C₁₋₃fluoroalkyl, C₁₋₃alkoxy or fluoro;
R^{1b} is hydroxyl, C₁₋₃alkyl, C₁₋₃hydroxyalkyl, C₁₋₃fluoroalkyl, C₁₋₃alkoxy, fluoro, or phenyl; or optionally two R^{1b} moieties when substituted on a common ring carbon atom, together with said carbon atom form a carbonyl;
R² is H or OH;
R³ is
   (a.) halo;
   (b.) C₁₋₃alkyl;
   (c.) -C(H)(OH)-CH₂OH;
   (d.) -CO₂R^{3a}, wherein R^{3a} is H or C₁₋₃alkyl;
   (e.) ring Cⁱ, wherein Cⁱ is
      (i.) phenyl,
      (ii.) 3- to 6-membered cycloalkyl;
      (iii.) 5- to 6-membered heteroaryl containing 1 or 2 heteroatoms selected from N, O, or S; or
      (iv.) 5- to 6-membered saturated heterocyclyl containing 1 to 2 N atoms; w
         herein ring Cⁱ is unsubstituted or substituted by 1 to 4 R^{3b}, wherein R^{3b} is selected from C₁₋₆alkyl, C₁₋₆fluoroalkyl, C₁₋₆hydroxyalkyl, C₁₋₃alkoxy, halo, -C(O)O-C₁₋₆alkyl, -C(O)OH, -C(O)-C₁₋₆alkyl, -C(O)N(H)-C₁₋₃alkyl, -CH₂C(O)OH, -CH₂C(O)O-C₁₋₃alkyl, -S(O)₂-C₁₋₃alkyl, or -C(H)(OH)-C₁₋₃fluoroalkyl;
         or optionally two R^{3b} moieties when substituted on a ring common carbon atom, together with said carbon atom form a carbonyl;
the subscript p is 0, 1 or 2;
the subscript q is 0,1, 2, 3, or 4; and
the subscript t is 0 or 1;
or a pharmaceutically acceptable salt thereof.

In embodiment no. 2, the present invention provides a compound of Formula (I) wherein 2 ≤ m1+m2 ≤ 4, and the remaining variables are as set forth in embodiment no. 1.

In embodiment no. 3, the present invention provides a compound of Formula (I) wherein ring C^{H} is unsubstituted or substituted pyrrolidine or piperidine, and the remaining variables are as set forth in embodiment no. 1.

In embodiment no. 4, the present invention provides a compound of Formula (I) wherein the group 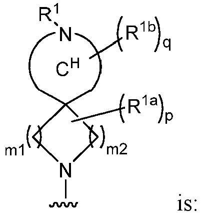 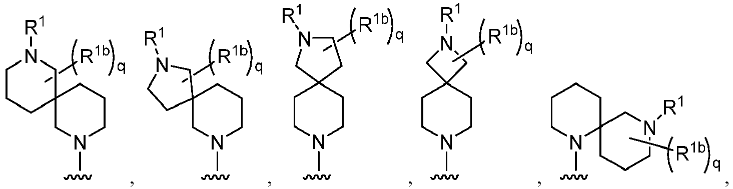 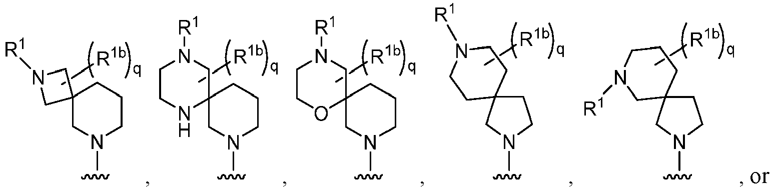 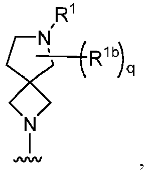 and the remaining variables are as set forth in embodiment no. 1.

In embodiment no. 5, the present invention provides a compound of Formula (I) wherein R¹ is H, methyl, -C(O)CH₃, unsubstituted phenyl, or phenyl substituted by 1 to 2 fluoro or C₁₋₄alkyl, and the remaining variables are as set forth in embodiment no. 1.

In embodiment no. 6, the present invention provides a compound of Formula (I) wherein R^{1b} is hydroxyl, phenyl, -CH₂OH, or optionally two R^{1b} moieties when substituted on a common ring carbon atom, together with said carbon atom form a carbonyl, and the remaining variables are as set forth in embodiment no. 1.

In embodiment no. 7, the present invention provides a compound of Formula (I) wherein the subscript p is 0, and the remaining variables are as set forth in embodiment no. 1.

In embodiment no. 8, the present invention provides a compound of Formula (I) wherein the subscript q is 0, 1, 2, or 3, and the remaining variables are as set forth in embodiment no. 1.

In embodiment no. 9, the present invention provides a compound of Formula (I) wherein
the group 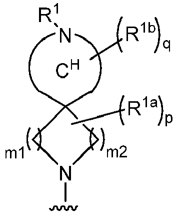 is set forth in embodiment no. 4;
R¹ is as set forth in embodiment no. 5;
R^{1b} is as set forth in embodiment no. 6;
the subscript q is 0, 1, 2, or 3, and the remaining variables are as set forth in embodiment no. 1.

In embodiment no. 10, the present invention provides a compound of Formula (I) wherein R² is H, and the remaining variables are as set forth in embodiment no. 1.

In embodiment no. 11, the present invention provides a compound of Formula (I) wherein the subscript t is 0, and the remaining variables are as set forth in embodiment no. 1.

In embodiment no. 12, the present invention provides a compound of Formula (I) wherein the subscript t is 1, and the remaining variables are as set forth in embodiment no. 1.

In embodiment no. 13, the present invention provides a compound of Formula (I) wherein the subscript t is 1 and R³ is substituted on the 7- or 8-positions of the illustrated gamma-carboline ring of Formula (I),and the remaining variables are as set forth in embodiment no. 1.

In embodiment no. 14, the present invention provides a compound of Formula (I) wherein the subscript t is 1 and R³ is fluoro, -C(O)OH, -C(O)OCH₃, -C(H)(OH)-CH₂OH, or a group selected from: 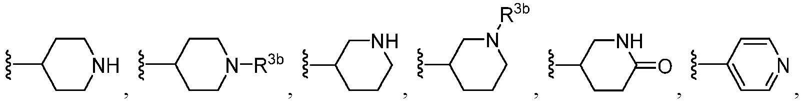 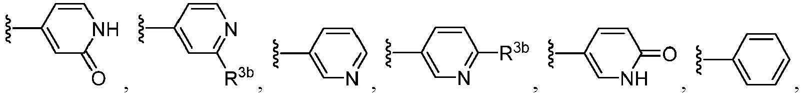 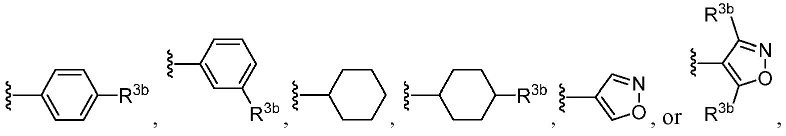 and the remaining variables are as set forth in embodiment no. 1. In embodiment no. 15, the present invention provides a compound of Formula (I) wherein the subscript t is 1 and R^{3b} is -CH₃, -CH₂CH₃, -C(H)(CH₃)₂, -OCH₃, -C(O)OH, -C(O)OCH₃, -C(O)OC(CH₃)₃, -C(O)N(H)CH₃, -CH₂C(O)CH₃, -CH₂C(OH)(CH₃)₂,-C(H)(OH)(CF₃), -S(O)₂CH₃, or -C(O)CH₃; and the remaining variables are as set forth in embodiment no. 1.

In embodiment no. 16, the present invention provides a compound of Formula (I) wherein the subscript t is 1, R³ is as set forth in embodiment no. 14, R^{3b} is as set forth in embodiment no. 15; and the remaining variables are as set forth in embodiment no. 1.

In embodiment no. 17, the present invention provides a compound of Formula (I) wherein:
the group 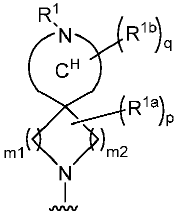 is set forth in embodiment no. 4;
R¹ is as set forth in embodiment no. 5;
R^{1b} is as set forth in embodiment no. 6;
the subscript q is 0, 1, 2, or 3,
the subscript t is 1;
R² is H;
R³ is substituted on the 7- or 8-positions of the illustrated gamma-carboline ring of Formula (I);
R³ is selected from the moieties set forth in embodiment no. 14, and
R^{3b} is as set forth in embodiment no. 15.

Non-limiting examples of the compounds of the present invention include:
1-[(6R)-8-(4-fluorophenyl)-7-oxo-2,8-diazaspiro[5.5]undec-2-yl]-5H-pyrido[4,3-b]indole-4-carboxamide;
1-[(6R)-8-(4-fluorophenyl)-7-oxo-2,8-diazaspiro[5.5]undec-2-yl]-5H-pyrido[4,3-b]indole-4-carboxamide;
1-[4-(4-fluorophenyl)-5-oxo-1-oxa-4,8-diazaspiro[5.5]undec-8-yl]-5H-pyrido[4,3-b]indole-4-carboxamide;
1-[8-(4-fluorophenyl)-7-oxo-2,8-diazaspiro[5.5]undec-2-yl]-5H-pyrido[4,3-b]indole-4-carboxamide;
1-{8-[4-(1-methylethyl)phenyl]-7-oxo-2,8-diazaspiro[5.5]undec-2-yl}-5H-pyrido[4,3-b]indole-4-carboxamide;
8-fluoro-1-[(6R)-8-(4-fluorophenyl)-7-oxo-2,8-diazaspiro[5.5]undec-2-yl]-5H-pyrido[4,3-b]indole-4-carboxamide;
8-fluoro-1-[(6S)-8-(4-fluorophenyl)-7-oxo-2,8-diazaspiro[5.5]undec-2-yl]-5H-pyrido[4,3-b]indole-4-carboxamide;
4-carbamoyl-1-[(6R)-8-(4-fluorophenyl)-7-oxo-2,8-diazaspiro[5.5]undec-2-yl]-5H-pyrido[4,3-b]indole-8-carboxylic acid;
N-hydroxy-1-(1-oxo-2,7-diazaspiro[4.5]dec-7-yl)-5H-pyrido[4,3-b]indole-4-carboxamide;
1-(3-oxo-2,8-diazaspiro[4.5]dec-8-yl)-5H-pyrido[4,3-b]indole-4-carboxamide;
1-(1-oxo-2,8-diazaspiro[4.5]dec-8-yl)-5H-pyrido[4,3-b]indole-4-carboxamide;
1-(2,8-diazaspiro[4.5]dec-8-yl)-5H-pyrido[4,3-b]indole-4-carboxamide;
*tert-butyl* 8-(4-carbamoyl-5H-pyrido[4,3-b]indol-1-yl)-2,8-diazaspiro[4.5]decane-2-carboxylate;
1-(2-acetyl-2,8-diazaspiro[4.5]dec-8-yl)-5H-pyrido[4,3-b]indole-4-carboxamide;
1-(2,8-diazaspiro[4.5]dec-2-yl)-5H-pyrido[4,3-b]indole-4-carboxamide;
1-(7-oxa-2-azaspiro[3.5]non-2-yl)-5H-pyrido[4,3-b]indole-4-carboxamide;
*tert*-butyl 7-(4-carbamoyl-5H-pyrido[4,3-b]indol-1-yl)-2,7-diazaspiro[3.5]nonane-2-carboxylate;
1-[(4S,5R)-4-hydroxy-1-oxo-2,7-diazaspiro[4.5]dec-7-yl]-5H-pyrido[4,3-b]indole-4-carboxamide;
1-[(4R,5S)-4-hydroxy-1-oxo-2,7-diazaspiro[4.5]dec-7-yl]-5H-pyrido[4,3-b]indole-4-carboxamide;
1-(2,7-diazaspiro[3.5]non-7-yl)-5H-pyrido[4,3-b]indole-4-carboxamide;
1-(2-acetyl-2,7-diazaspiro[3.5]non-7-yl)-5H-pyrido[4,3-b]indole-4-carboxamide;
*tert*-butyl 2-(4-carbamoyl-5H-pyrido[4,3-b]indol-1-yl)-2,6-diazaspiro[3.4]octane-6-carboxylate;
1-[(4S,5S)-4-hydroxy-1-oxo-2,7-diazaspiro[4.5]dec-7-yl]-5H-pyrido[4,3-b]indole-4-carboxamide;
1-[(4S,5S)-4-hydroxy-1-oxo-2,7-diazaspiro[4.5]dec-7-yl]-5H-pyrido[4,3-b]indole-4-carboxamide;
1-[(4R,5R)-4-hydroxy-1-oxo-2,7-diazaspiro[4.5]dec-7-yl]-5H-pyrido[4,3-b]indole-4-carboxamide;
1-{8-[(4-*tert*-butylphenyl)carbonyl]-1,8-diazaspiro[5.5]undec-1-yl}-5H-pyrido[4,3-b]indole-4-carboxamide;
1-(2,6-diazaspiro[3.4]oct-2-yl)-5H-pyrido[4,3-b]indole-4-carboxamide;
1-(6-acetyl-2,6-diazaspiro[3.4]oct-2-yl)-5H-pyrido[4,3-b]indole-4-carboxamide;
*tert-*butyl 6-(4-carbamoyl-5H-pyrido[4,3-b]indol-1-yl)-2,6-diazaspiro[3.5]nonane-2-carboxylate;
1-(2-acetyl-2,6-diazaspiro[3.5]non-6-yl)-5H-pyrido[4,3-b]indole-4-carboxamide;
1-(7-oxo-2,8-diazaspiro[5.5]undec-2-yl)-5H-pyrido[4,3-b]indole-4-carboxamide;
methyl 3-[8-(4-carbamoyl-5H-pyrido[4,3-b]indol-1-yl)-1-oxo-2,8-diazaspiro[5.5]undec-2-yl]propanoate;
1-[2-(4-fluorophenyl)-1-oxo-2,7-diazaspiro[4.5]dec-7-yl]-5H-pyrido[4,3-b]indole-4-carboxamide;
1-[7-(4-fluorophenyl)-6-oxo-2,7-diazaspiro[4.5]dec-2-yl]-5H-pyrido[4,3-b]indole-4-carboxamide;
1-(1-oxo-2,7-diazaspiro[4.5]dec-7-yl)-5H-pyrido[4,3-b]indole-4-carboxamide;
1-(8-methyl-7-oxo-2,8-diazaspiro[5.5]undec-2-yl)-5H-pyrido[4,3-b]indole-4-carboxamide;
1-[2-(4-*tert*-butylphenyl)-1-oxo-2,7-diazaspiro[4.5]dec-7-yl]-5H-pyrido[4,3-b]indole-4-carboxamide;
1-(8-oxo-2,9-diazaspiro[5.5]undec-2-yl)-5H-pyrido[4,3-b]indole-4-carboxamide;
1-[9-(4-*tert*-butylphenyl)-8-oxo-2,9-diazaspiro[5.5]undec-2-yl]-5H-pyrido[4,3-b]indole-4-carboxamide;
1-[8-(1-methylethyl)-7-oxo-2,8-diazaspiro[5.5]undec-2-yl]-5H-pyrido[4,3-b]indole-4-carboxamide;
1-[(5R)-1-oxo-2,7-diazaspiro[4.5]dec-7-yl]-5H-pyrido[4,3-b]indole-4-carboxamide;
1-[(5S)-1-oxo-2,7-diazaspiro[4.5]dec-7-yl]-5H-pyrido[4,3-b]indole-4-carboxamide;
1-(1-oxo-2,7-diazaspiro[4.5]dec-7-yl)-7-piperidin-4-yl-5H-pyrido[4,3-b]indole-4-carboxamide;
*tert*-butyl 4-[4-carbamoyl-1-(1-oxo-2,7-diazaspiro[4.5]dec-7-yl)-5H-pyrido[4,3-b]indol-7-yl]piperidine-1-carboxylate;
1-(1-oxo-2,7-diazaspiro[4.5]dec-7-yl)-7-piperidin-3-yl-5H-pyrido[4,3-b]indole-4-carboxamide;
1-(1-oxo-2,7-diazaspiro[4.5]dec-7-yl)-8-piperi din-4-yl-5H-pyrido[4,3-b]indole-4-carboxamide;
*tert*-butyl 4-[4-carbamoyl-1-(1-oxo-2,7-diazaspiro[4.5]dec-7-yl)-5H-pyrido[4,3-b]indol-8-yl]piperidine-1-carboxylate;
8-[1-(methylcarbamoyl)piperidin-4-yl]-1-(1-oxo-2,7-diazaspiro[4.5]dec-7-yl)-5H-pyrido[4,3-b]indole-4-carboxamide;
{4-[4-carbamoyl-1-(1-oxo-2,7-diazaspiro[4.5]dec-7-yl)-5H-pyrido[4,3-b]indol-8-yl]piperidin-1-yl} acetic acid;
8-(1-ethylpiperidin-4-yl)-1-(1-oxo-2,7-diazaspiro[4.5]dec-7-yl)-5H-pyrido[4,3-b]indole-4-carboxamide;
8-[1-(1-methylethyl)piperidin-4-yl]-1-(1-oxo-2,7-diazaspiro[4.5]dec-7-yl)-5H-pyrido[4,3-b]indole-4-carboxamide;
methyl 4-[4-carbamoyl-1-(1-oxo-2,7-diazaspiro[4.5]dec-7-yl)-5H-pyrido[4,3-b]indol-8-yl]piperidine-1-carboxylate;
7-[1-(1-methylethyl)piperidin-4-yl]-1-(1-oxo-2,7-diazaspiro[4.5]dec-7-yl)-5H-pyrido[4,3-b]indole-4-carboxamide;
7-[1-(methylcarbamoyl)piperidin-4-yl]-1-(1-oxo-2,7-diazaspiro[4.5]dec-7-yl)-5H-pyrido[4,3-b]indole-4-carboxamide;
7-[1-(2-hydroxy-2-methylpropyl)piperidin-4-yl]-1-(1-oxo-2,7-diazaspiro[4.5]dec-7-yl)-5H-pyrido[4,3-b]indole-4-carboxamide;
7-(1-methylpiperidin-4-yl)-1-(1-oxo-2,7-diazaspiro[4.5]dec-7-yl)-5H-pyrido[4,3-b]indole-4-carboxamide;
methyl 4-[4-carbamoyl-1-(1-oxo-2,7-diazaspiro[4.5]dec-7-yl)-5H-pyrido[4,3-b]indol-7-yl]piperidine-1-carboxylate;
7-[1-(methylsulfonyl)piperidin-4-yl]-1-(1-oxo-2,7-diazaspiro[4.5]dec-7-yl)-5H-pyrido[4,3-b]indole-4-carboxamide;
7-(1-acetylpiperidin-4-yl)-1-(1-oxo-2,7-diazaspiro[4.5]dec-7-yl)-5H-pyrido[4,3-b]indole-4-carboxamide;
{4-[4-carbamoyl-1-(1-oxo-2,7-diazaspiro[4.5]dec-7-yl)-5H-pyrido[4,3-b]indol-7-yl]piperidin-1-yl} acetic acid;
8-(1-methylpiperidin-4-yl)-1-(1-oxo-2,7-diazaspiro[4.5]dec-7-yl)-5H-pyrido[4,3-b]indole-4-carboxamide;
8-(1-acetylpiperidin-4-yl)-1-(1-oxo-2,7-diazaspiro[4.5]dec-7-yl)-5H-pyrido[4,3-b]indole-4-carboxamide;
8-[1-(2-hydroxy-2-methylpropyl)piperidin-4-yl]-1-(1-oxo-2,7-diazaspiro[4.5]dec-7-yl)-5H-pyrido[4,3-b]indole-4-carboxamide;
1-[(5S)-1-oxo-2,7-diazaspiro[4.5]dec-7-yl]-7-piperidin-4-yl-5H-pyrido[4,3-b]indole-4-carboxamide;
1-[(5R)-1-oxo-2,7-diazaspiro[4.5]dec-7-yl]-7-piperidin-4-yl-5H-pyrido[4,3-b]indole-4-carboxamide;
8-(1,2-dihydroxyethyl)-1-(1-oxo-2,7-diazaspiro[4.5]dec-7-yl)-5H-pyrido[4,3-b]indole-4-carboxamide;
8-[1-(methylsulfonyl)piperidin-4-yl]-1-(1-oxo-2,7-diazaspiro[4.5]dec-7-yl)-5H-pyrido[4,3-b]indole-4-carboxamide;
7-(6-methoxypyridin-3-yl)-1-[(5R)-1-oxo-2,7-diazaspiro[4.5]dec-7-yl]-5H-pyrido[4,3-b]indole-4-carboxamide;
1-[(5R)-1-oxo-2,7-diazaspiro[4.5]dec-7-yl]-7-(6-oxo-1,6-dihydropyridin-3-yl)-5H-pyrido[4,3-b]indole-4-carboxamide;
7-(2-methoxypyridm-4-yl)-1-[(5R)-1-oxo-2,7-diazaspiro[4.5]dec-7-yl]-5H-pyrido[4,3-b]indole-4-carboxamide;
1-[(5R)-1-oxo-2,7-diazaspiro[4.5]dec-7-yl]-7-(2-oxo-1,2-dihydropyridin-4-yl)-5H-pyrido[4,3-b]indole-4-carboxamide;
7-[1-(1-methylethyl)piperidin-4-yl]-1-[(5R)-1-oxo-2,7-diazaspiro[4.5]dec-7-yl]-5H-pyrido[4,3-b]indole-4-carboxamide;
7-[1-(2-hydroxy-2-methylpropyl)piperidin-4-yl]-1-[(5R)-1-oxo-2,7-diazaspiro[4.5]dec-7-yl]-5H-pyrido[4,3-b]indole-4-carboxamide;
1-[(5R)-1-oxo-2,7-diazaspiro[4.5]dec-7-yl]-7-(6-oxopiperidin-3-yl)-5H-pyrido[4,3-b]indole-4-carboxamide;
7-(6-methoxypyridin-3-yl)-1-[(5R)-2-methyl-1-oxo-2,7-diazaspiro[4.5]dec-7-yl]-5H-pyrido[4,3-b]indole-4-carboxamide;
7-(2-methoxypyndin-4-yl)-1-[(5R)-2-methyl-1-oxo-2,7-diazaspiro[4.5]dec-7-yl]-5H-pyrido[4,3-b]indole-4-carboxamide;
7-[1-(1-methylethyl)piperidin-4-yl]-1-[(5R)-2-methyl-1-oxo-2,7-diazaspiro[4.5]dec-7-yl]-5H-pyrido[4,3-b]indole-4-carboxamide;
7-[1-(2-hydroxy-2-methylpropyl)piperidin-4-yl]-1-[(5R)-2-methyl-1-oxo-2,7-diazaspiro[4.5]dec-7-yl]-5H-pyrido[4,3-b]indole-4-carboxamide;
1-[(5R)-2-methyl-1-oxo-2,7-diazaspiro[4.5]dec-7-yl]-7-[4-(2,2,2-trifluoro-1-hydroxyethyl)phenyl]-5H-pyrido[4,3-b]indole-4-carboxamide;
1-[(5R)-2-methyl-1-oxo-2,7-diazaspiro[4.5]dec-7-yl]-7-[3-(2,2,2-trifluoro-1-hydroxyethyl)phenyl]-5H-pyrido[4,3-b]indole-4-carboxamide;
7-(3,5-dimethylisoxazol-4-yl)-1-[(5R)-2-methyl-1-oxo-2,7-diazaspiro[4.5]dec-7-yl]-5H-pyrido[4,3-b]indole-4-carboxamide;
methyl(4-{4-carbamoyl-1-[(5R)-2-methyl-1-oxo-2,7-diazaspiro[4.5]dec-7-yl]-5H-pyrido[4,3-b]indol-7-yl}cyclohexyl)acetate;
7-[4-(2-hydroxy-2-methylpropyl)cyclohexyl]-1-[(5R)-2-methyl-1-oxo-2,7-diazaspiro[4.5]dec-7-yl]-5H-pyrido[4,3-b]indole-4-carboxamide;
methyl 4-carbamoyl-1-(1-oxo-2,7-diazaspiro[4.5]dec-7-yl)-5H-pyrido[4,3-b]indole-7-carboxylate;
4-carbamoyl-1-(1-oxo-2,7-diazaspiro[4.5]dec-7-yl)-5H-pyrido[4,3-b]indole-8-carboxylic acid;
4-carbamoyl-1-(1-oxo-2,7-diazaspiro[4.5]dec-7-yl)-5H-pyrido[4,3-b]indole-7-carboxylic acid;
1-(4-hydroxy-1-oxo-2,7-diazaspiro[4.5]dec-7-yl)-5H-pyrido[4,3-b]indole-4-carboxamide;
1-(5-oxo-1,4,8-triazaspiro[5.5]undec-8-yl)-5H-pyrido[4,3-b]indole-4-carboxamide;
1-[(4R,5R)-1-oxo-4-phenyl-2,7-diazaspiro[4.5]dec-7-yl]-5H-pyrido[4,3-b]indole-4-carboxamide;
1-[(4R,5S)-1-oxo-4-phenyl-2,7-diazaspiro[4.5]dec-7-yl]-5H-pyrido[4,3-b]indole-4-carboxamide;
1-[(4S,5 S)-4-(hydroxymethyl)-2-methyl-1-oxo-2,7-diazaspiro[4.5]dec-7-yl]-5H-pyrido[4,3-b]indole-4-carboxamide;
1-[(4R,5S)-4-(hydroxymethyl)-2-methyl-1-oxo-2,7-diazaspiro[4.5]dec-7-yl]-5H-pyrido[4,3-b]indole-4-carboxamide; or
a pharmaceutically acceptable salt thereof.

### Utilities

The compounds having Formula (I) and pharmaceutical compositions thereof can be used to treat or prevent a variety of conditions, diseases or disorders mediated by Bruton's Tyrosine kinase (Btk). Such Btk-mediated conditions, diseases or disorders include, but are not limited to: (1) arthritis, including rheumatoid arthritis, juvenile arthritis, psoriatic arthritis and osteoarthritis; (2) asthma and other obstructive airways diseases, including chronic asthma, late asthma, airway hyper-responsiveness, bronchitis, bronchial asthma, allergic asthma, intrinsic asthma, extrinsic asthma, dust asthma, adult respiratory distress syndrome, recurrent airway obstruction, and chronic obstruction pulmonary disease including emphysema; (3) autoimmune diseases or disorders, including those designated as single organ or single cell-type autoimmune disorders, for example Hashimoto's thyroiditis, autoimmune hemolytic anemia, autoimmune atrophic gastritis of pernicious anemia, autoimmune encephalomyelitis, autoimmune orchitis, Goodpasture's disease, autoimmune thrombocytopenia including idiopathic thrombopenic purpura, sympathetic ophthalmia, myasthenia gravis, Graves' disease, primary biliary cirrhosis, chronic aggressive hepatitis, ulcerative colitis and membranous glomerulopathy, those designated as involving systemic autoimmune disorder, for example systemic lupus erythematosis, immune thrombocytopenic purpura, rheumatoid arthritis, Sjogren's syndrome, Reiter's syndrome, polymyositis-dermatomyositis, systemic sclerosis, polyarteritis nodosa, multiple sclerosis and bullous pemphigoid, and additional autoimmune diseases, which can be B-cell (humoral) based or T-cell based, including Cogan's syndrome, ankylosing spondylitis, Wegener's granulomatosis, autoimmune alopecia, Type I or juvenile onset diabetes, and thyroiditis; (4) cancers or tumors, including alimentary/gastrointestinal tract cancer, colon cancer, liver cancer, skin cancer including mast cell tumor and squamous cell carcinoma, breast and mammary cancer, ovarian cancer, prostate cancer, lymphoma and leukemia (including but not limited to acute myelogenous leukemia, chronic myelogenous leukemia, mantle cell lymphoma, NHL B cell lymphomas (e.g., precursor B-ALL, marginal zone B cell lymphoma, chronic lymphocytic leukemia, diffuse large B cell lymphoma, Burkitt lymphoma, mediastinal large B-cell lymphoma), Hodgkin lymphoma, NK and T cell lymphomas; TEL-Syk and ITK-Syk fusion driven tumors, myelomas including multiple myeloma, myeloproliferative disorders kidney cancer, lung cancer, muscle cancer, bone cancer, bladder cancer, brain cancer, melanoma including oral and metastatic melanoma, Kaposi's sarcoma, proliferative diabetic retinopathy, and angiogenic-associated disorders including solid tumors, and pancreatic cancer; (5) diabetes, including Type I diabetes and complications from diabetes; (6) eye diseases, disorders or conditions including autoimmune diseases of the eye, keratoconjunctivitis, vernal conjunctivitis, uveitis including uveitis associated with Behcet's disease and lens-induced uveitis, keratitis, herpetic keratitis, conical keratitis, corneal epithelial dystrophy, keratoleukoma, ocular premphigus, Mooren's ulcer, scleritis, Grave's ophthalmopathy, Vogt-Koyanagi-Harada syndrome, keratoconjunctivitis sicca (dry eye), phlyctenule, iridocyclitis, sarcoidosis, endocrine ophthalmopathy, sympathetic ophthalmitis, allergic conjunctivitis, and ocular neovascularization; (7) intestinal inflammations, allergies or conditions including Crohn's disease and/or ulcerative colitis, inflammatory bowel disease, coeliac diseases, proctitis, eosinophilic gastroenteritis, and mastocytosis; (8) neurodegenerative diseases including motor neuron disease, Alzheimer's disease, Parkinson's disease, amyotrophic lateral sclerosis, Huntington's disease, cerebral ischemia, or neurodegenerative disease caused by traumatic injury, strike, glutamate neurotoxicity or hypoxia; ischemic/ reperfusion injury in stroke, myocardial ischemica, renal ischemia, heart attacks, cardiac hypertrophy, atherosclerosis and arteriosclerosis, organ hypoxia; (9) platelet aggregation and diseases associated with or caused by platelet activation, such as arteriosclerosis, thrombosis, intimal hyperplasia and restenosis following vascular injury; (10) conditions associated with cardiovascular diseases, including restenosis, acute coronary syndrome, myocardial infarction, unstable angina, refractory angina, occlusive coronary thrombus occurring post-thrombolytic therapy or post-coronary angioplasty, a thrombotically mediated cerebrovascular syndrome, embolic stroke, thrombotic stroke, transient ischemic attacks, venous thrombosis, deep venous thrombosis, pulmonary embolus, coagulopathy, disseminated intravascular coagulation, thrombotic thrombocytopenic purpura, thromboangiitis obliterans, thrombotic disease associated with heparin-induced thrombocytopenia, thrombotic complications associated with extracorporeal circulation, thrombotic complications associated with instrumentation such as cardiac or other intravascular catheterization, intra-aortic balloon pump, coronary stent or cardiac valve, conditions requiring the fitting of prosthetic devices, and the like; (11) skin diseases, conditions or disorders including atopic dermatitis, eczema, psoriasis, scleroderma, pruritus and other pruritic conditions; (12) allergic reactions including anaphylaxis, allergic rhinitis, allergic dermatitis, allergic urticaria, angioedema, allergic asthma, or allergic reaction to insect bites, food, drugs, or pollen; (13) transplant rejection, including pancreas islet transplant rejection, bone marrow transplant rejection, graft- versus-host disease, organ and cell transplant rejection such as bone marrow, cartilage, cornea, heart, intervertebral disc, islet, kidney, limb, liver, lung, muscle, myoblast, nerve, pancreas, skin, small intestine, or trachea, and xeno transplantation; and (14) low grade scarring including scleroderma, increased fibrosis, keloids, post-surgical scars, pulmonary fibrosis, vascular spasms, migraine, reperfusion injury, and post-myocardial infarction.

The invention thus provides compounds of Formula (I) and salts thereof for use in therapy, and particularly in the treatment of disorders, diseases and conditions mediated by inappropriate Btk activity.

The inappropriate Btk activity referred to herein is any Btk activity that deviates from the normal Btk activity expected in a particular mammalian subject. Inappropriate Btk activity may take the form of, for instance, an abnormal increase in activity, or an aberration in the timing and or control of Btk activity. Such inappropriate activity may result then, for example, from overexpression or mutation of the protein kinase leading to inappropriate or uncontrolled activation.

In one embodiment, the present invention provides for the use of a compound of Formula (I), or a pharmaceutically acceptable salt thereof for the manufacture of a medicament for the treatment of a Btk-mediated disorder.

A further aspect of the invention resides in the use of a compound of Formula (I) or a pharmaceutically acceptable salt thereof for the manufacture of a medicament to be used for the treatment of chronic B cell disorders in which T cells play a prominent role.

Thus, the compounds according to the invention may be used in therapies to treat or prevent Bruton's Tyrosine Kinase (Btk) mediated diseases, conditions and disorders. Btk mediated diseases, conditions and disorders as used herein, mean any disease, condition or disorder in which B cells, mast cells, myeloid cells or osteoclasts play a central role. These diseases include but are not limited to, immune, autoimmune and inflammatory diseases, allergies, infectious diseases, bone resorption disorders and proliferative diseases.

Immune, autoimmune and inflammatory diseases that may be treated or prevented with the compounds of the present invention include rheumatic diseases (*e.g.,* rheumatoid arthritis, psoriatic arthritis, infectious arthritis, progressive chronic arthritis, deforming arthritis, osteoarthritis, traumatic arthritis, gouty arthritis, Reiter's syndrome, polychondritis, acute synovitis and spondylitis), glomerulonephritis (with or without nephrotic syndrome), Goodpasture's syndrome, (and associated glomerulonephritis and pulmonary hemorrhage), atherosclerosis, autoimmune hematologic disorders (*e.g.,* hemolytic anemia, aplasic anemia, idiopathic thrombocytopenia, chronic idiopathic thrombocytopenic purpura (ITP), and neutropenia), autoimmune gastritis, and autoimmune inflammatory bowel diseases (*e.g.,* ulcerative colitis and Crohn's disease), irritable bowel syndrome, host versus graft disease, allograft rejection, chronic thyroiditis, Graves' disease, Sjogren's disease, scleroderma, diabetes (type I and type II), active hepatitis (acute and chronic), pancreatitis, primary billiary cirrhosis, myasthenia gravis, multiple sclerosis, systemic lupus erythematosis, psoriasis, atopic dermatitis, dermatomyositis, contact dermatitis, eczema, skin sunburns, vasculitis (*e.g.,* Behcet's disease), ANCA-associated and other vasculitudes, chronic renal insufficiency, Stevens-Johnson syndrome, inflammatory pain, idiopathic sprue, cachexia, sarcoidosis, Guillain-Barré syndrome, uveitis, conjunctivitis, kerato conjunctivitis, otitis media, periodontal disease, Addison's disease, Parkinson's disease, Alzheimer's disease, diabetes, septic shock, myasthenia gravis, pulmonary interstitial fibrosis, asthma, bronchitis, rhinitis, sinusitis, pneumoconiosis, pulmonary insufficiency syndrome, pulmonary emphysema, pulmonary fibrosis, silicosis, chronic inflammatory pulmonary disease (*e.g.,* chronic obstructive pulmonary disease) and other inflammatory or obstructive disease on airways.

Allergies that may be treated or prevented include, among others, allergies to foods, food additives, insect poisons, dust mites, pollen, animal materials and contact allergans, type I hypersensitivity allergic asthma, allergic rhinitis, allergic conjunctivitis.

Infectious diseases that may be treated or prevented include, among others, sepsis, septic shock, endotoxic shock, sepsis by Gram-negative bacteria, shigellosis, meningitis, cerebral malaria, pneumonia, tuberculosis, viral myocarditis, viral hepatitis (hepatitis A, hepatitis B and hepatitis C), HIV infection, retinitis caused by cytomegalovirus, influenza, herpes, treatment of infections associated with severe burns, myalgias caused by infections, cachexia secondary to infections, and veterinary viral infections such as lentivirus, caprine arthritic virus, visna-maedi virus, feline immunodeficiency virus, bovine immunodeficiency virus or canine immunodeficiency virus.

Bone resorption disorders that may be treated or prevented include, among others, osteoporosis, osteoarthritis, traumatic arthritis, gouty arthritis and bone disorders related with multiple myeloma.

Proliferative diseases that may be treated or prevented include, among others, non-Hodgkin lymphoma (in particular the subtypes diffuse large B-cell lymphoma (DLBCL) and mantle cell lymphoma (MCL)), B cell chronic lymphocytic leukemia and acute lymphoblastic leukemia (ALL) with mature B cell, ALL in particular.

In particular the compounds of Formula (I) or pharmaceutically acceptable salts may be used for the treatment of B cell lymphomas resulting from chronic active B cell receptor signaling.

Yet another aspect of the present invention provides compounds for use in a method for treating diseases caused by or associated with Fc receptor signaling cascades, including FceRI and/or FcgRI-mediated degranulation as a therapeutic approach towards the treatment or prevention of diseases characterized by, caused by and/or associated with the release or synthesis of chemical mediators of such Fc receptor signaling cascades or degranulation. In addition, Btk is known to play a critical role inimmunotyrosine-based activation motif (ITAM) singaling, B cell receptor signaling, T cell receptor signaling and is an essential component of integrin beta (1), beta (2), and beta (3) signaling in neutrophils. Thus, compounds of the present invention can be used to regulate Fc receptor, ITAM, B cell receptor and integrin signaling cascades, as well as the cellular responses elicited through these signaling cascades. Non-limiting examples of cellular responses that may be regulated or inhibited include respiratory burst, cellular adhesion, cellular degranulation, cell spreading, cell migration, phagocytosis, calcium ion flux, platelet aggregation and cell maturation.

### Combination Therapy

Included herein are pharmaceutical compositions in which at least one compound of Formula (I) or a pharmaceutically acceptable salt thereof is administered in combination with at least one other active agent. The other active agent is an anti-inflammatory agent, an immunosuppressant agent, or a chemotherapeutic agent. Anti-inflammatory agents include but are not limited to NSAIDs, non-specific and COX-2 specific cyclooxgenase enzyme inhibitors, gold compounds, corticosteroids, methotrexate, tumor necrosis factor receptor (TNF) receptors antagonists, immunosuppressants and methotrexate.

Examples of NSAIDs include, but are not limited to, ibuprofen, flurbiprofen, naproxen and naproxen sodium, diclofenac, combinations of diclofenac sodium and misoprostol, sulindac, oxaprozin, diflunisal, piroxicam, indomethacin, etodolac, fenoprofen calcium, ketoprofen, sodium nabumetone, sulfasalazine, tolmetin sodium, and hydroxychloroquine. Examples of NSAIDs also include COX-2 specific inhibitors such as celecoxib, valdecoxib, lumiracoxib and/or etoricoxib.

In some embodiments, the anti-inflammatory agent is a salicylate. Salicylates include by are not limited to acetylsalicylic acid or aspirin, sodium salicylate, and choline and magnesium salicylates.

The anti-inflammatory agent may also be a corticosteroid. For example, the corticosteroid may be cortisone, dexamethasone, methylprednisolone, prednisolone, prednisolone sodium phosphate, or prednisone.

In additional embodiments the anti-inflammatory agent is a gold compound such as gold sodium thiomalate or auranofin.

The invention also includes embodiments in which the anti-inflammatory agent is a metabolic inhibitor such as a dihydrofolate reductase inhibitor, such as methotrexate or a dihydroorotate dehydrogenase inhibitor, such as leflunomide.

Other embodiments of the invention pertain to combinations in which at least one anti-inflammatory agent is an anti-C5 monoclonal antibody (such as eculizumab or pexelizumab), a TNF antagonist, such as entanercept, or infliximab, which is an anti-TNF alpha monoclonal antibody.

Still other embodiments of the invention pertain to combinations in which at least one active agent is an immunosuppressant agent, such as an immunosuppressant compound chosen from methotrexate, leflunomide, cyclosporine, tacrolimus, azathioprine, and mycophenolate mofetil.

B-cells and B-cell precursors expressing BTK have been implicated in the pathology of B-cell malignancies, including, but not limited to, B-cell lymphoma, lymphoma (including Hodgkin's and non-Hodgkin's lymphoma), hairy cell lymphoma, multiple myeloma, chronic and acute myelogenous leukemia and chronic and acute lymphocytic leukemia.

BTK has been shown to be an inhibitor of the Fas/APO-1 (CD-95) death inducing signaling complex (DISC) in B-lineage lymphoid cells. The fate of leukemia/lymphoma cells may reside in the balance between the opposing proapoptotic effects of caspases activated by DISC and an upstream anti-apoptotic regulatory mechanism involving BTK and/or its substrates (Vassilev et al., J. Biol. Chem. 1998, 274, 1646-1656).

It has also been discovered that BTK inhibitors are useful as chemosensitizing agents, and, thus, are useful in combination with other chemotherapeutic agents, in particular, drugs that induce apoptosis. Examples of other chemotherapeutic agents that can be used in combination with chemosensitizing BTK inhibitors include topoisomerase I inhibitors (camptothecin or topotecan), topoisomerase II inhibitors (*e.g.,* daunomycin and etoposide), alkylating agents (*e.g.,* cyclophosphamide, melphalan and BCNU), tubulin directed agents (*e.g.,* taxol and vinblastine), and biological agents (*e.g.,* antibodies such as anti CD20 antibody, IDEC 8, immunotoxins, and cytokines).

Btk activity has also been associated with some leukemias expressing the bcr-abl fusion gene resulting from translocation of parts of chromosome 9 and 22. This abnormality is commonly observed in chronic myelogenous leukemia. Btk is constitutively phosphorylated by the bcr-abl kinase which initiates downstream survival signals which circumvents apoptosis in bcr-abl cells. (N. Feldhahn et al. J. Exp. Med. 2005 201(11):1837-1852).

The compound(s) of Formula (I) and the other pharmaceutically active agent(s) may be administered together or separately and, when administered separately this may occur simultaneously or sequentially in any order. The amounts of the compound(s) of Formula (I) and the other pharmaceutically active agent(s) and the relative timings of administration will be selected in order to achieve the desired combined therapeutic effect.

For the treatment of the inflammatory diseases, rheumatoid arthritis, psoriasis, inflammatory bowel disease, COPD, asthma and allergic rhinitis a compound of Formula (I) may be combined with one or more other active agents such as: (1) TNF-α inhibitors such as infliximab (Remicade®), etanercept (Enbrel®), adalimumab (Humira®), certolizumab pegol (Cimzia®), and golimumab (Simponi®); (2) non-selective COX-I/COX-2 inhibitors (such as piroxicam, diclofenac, propionic acids such as naproxen, flubiprofen, fenoprofen, ketoprofen and ibuprofen, fenamates such as mefenamic acid, indomethacin, sulindac, etodolac, azapropazone, pyrazolones such as phenylbutazone, salicylates such as aspirin); (3) COX-2 inhibitors (such as meloxicam, celecoxib, valdecoxib and etoricoxib); (4) other agents for treatment of rheumatoid arthritis including methotrexate, leflunomide, sulfasalazine, azathioprine, cyclosporin, tacrolimus, penicillamine, bucillamine, actarit, mizoribine, lobenzarit, ciclesonide, hydroxychloroquine, d-penicillamine, aurothiomalate, auranofin or parenteral or oral gold, cyclophosphamide, Lymphostat-B, BAFF/APRIL inhibitors and CTLA-4-Ig or mimetics thereof; (5) leukotriene biosynthesis inhibitor, 5-lipoxygenase (5-LO) inhibitor or 5-lipoxygenase activating protein (FLAP) antagonist such as zileuton; (6) LTD4 receptor antagonist such as zafirlukast, montelukast and pranlukast; (7) PDE4 inhibitor such as roflumilast, cilomilast, AWD-12-281 (Elbion), and PD-168787 (Pfizer); (8) antihistaminic HI receptor antagonists such as cetirizine, levocetirizine, loratadine, desloratadine, fexofenadine, astemizole, azelastine, levocabastine, olopatidine, methapyrilene and chlorpheniramine; (9) α1- and α2-adrenoceptor agonist vasoconstrictor sympathomimetic agent, such as propylhexedrine, phenylephrine, phenylpropanolamine, pseudoephedrine, naphazoline hydrochloride, oxymetazoline hydrochloride, tetrahydrozoline hydrochloride, xylometazoline hydrochloride, and ethylnorepinephrine hydrochloride; (10) anticholinergic agents such as ipratropium bromide, tiotropium bromide, oxitropium bromide, aclindinium bromide, glycopyrrolate, (R,R)-glycopyrrolate, pirenzepine, and telenzepine; (11) β-adrenoceptor agonists such as metaproterenol, isoproterenol, isoprenaline, albuterol, formoterol (particularly the fumarate salt), salmeterol (particularly the xinafoate salt), terbutaline, orciprenaline, bitolterol mesylate, fenoterol, and pirbuterol, or methylxanthanines including theophylline and aminophylline, sodium cromoglycate; (12) insulin-like growth factor type I (IGF-1) mimetic; (13) glucocorticosteroids, especially inhaled glucocorticoid with reduced systemic side effects, such as prednisone, prednisolone, flunisolide, triamcinolone acetonide, beclomethasone dipropionate, budesonide, fluticasone propionate, ciclesonide and mometasone furoate; (14) kinase inhibitors such as inhibitors of the Janus Kinases (JAK 1 and/or JAK2 and/or JAK 3 and/or TYK2), p38 MAPK and IKK2; (15) B-cell targeting biologics such as rituximab (Rituxan®); (16) selective costimulation modulators such as abatacept (Orencia); (17) interleukin inhibitors, such as IL-1 inhibitor anakinra (Kineret) and IL-6 inhibitor tocilizumab (Actemra).

The present invention also provides for "triple combination" therapy, comprising a compound of Formula (I) or a pharmaceutically acceptable salt thereof together with beta₂-adrenoreceptor agonist and an anti-inflammatory corticosteroid. Preferably this combination is for treatment and/or prophylaxis of asthma, COPD or allergic rhinitis. The beta₂-adrenoreceptor agonist and/or the anti-inflammatory corticosteroid can be as described above and/or as described in WO 03/030939 A1. Representative examples of such a "triple" combination are a compound of Formula (I) or a pharmaceutically acceptable salt thereof in combination with the components of Advair® (salmeterol xinafoate and fluticasone propionate), Symbicort® (budesonide and formoterol fumarate), or Dulera® (mometasone furoate and formoterol).

For the treatment of cancer, a compound of Formula (I) may be combined with one or more of an anticancer agents. Examples of such agents can be found in Cancer Principles and Practice of Oncology by V.T. Devita and S. Hellman (editors), 6th edition (February 15, 2001), Lippincott Williams & Wilkins Publishers. A person of ordinary skill in the art would be able to discern which combinations of agents would be useful based on the particular characteristics of the drugs and the cancer involved. Such anti-cancer agents include, but are not limited to, the following: (1) estrogen receptor modulator such as diethylstibestral, tamoxifen, raloxifene, idoxifene, LY353381, LY117081, toremifene, fluoxymestero, and SH646; (2) other hormonal agents including aromatase inhibitors (*e.g*.,, aminoglutethimide, tetrazole anastrozole, letrozole and exemestane), luteinizing hormone release hormone (LHRH) analogues, ketoconazole, goserelin acetate, leuprolide, megestrol acetate and mifepristone; (3) androgen receptor modulator such as finasteride and other 5α-reductase inhibitors, nilutamide, flutamide, bicalutamide, liarozole, and abiraterone acetate; (4) retinoid receptor modulator such as bexarotene, tretinoin, 13-cis-retinoic acid, 9-cis-retinoic acid, α-difluoromethylornithine, ILX23-7553, trans-N-(4'-hydroxyphenyl) retinamide, and N-4-carboxyphenyl retinamide; (5) antiproliferative agent such asantisense RNA and DNA oligonucleotides such as G3139, ODN698, RVASKRAS, GEM231, and INX3001, and antimetabolites such as enocitabine, carmofur, tegafur, pentostatin, doxifluridine, trimetrexate, fludarabine, capecitabine, galocitabine, cytarabine ocfosfate, fosteabine sodium hydrate, raltitrexed, paltitrexid, emitefur, tiazofurin, decitabine, nolatrexed, pemetrexed, nelzarabine, 2'-deoxy-2'-methylidenecytidine, 2'-fluoromethylene-2'-deoxycytidine, N6-[4-deoxy-4-[N2-[2(E),4(E)-tetradeca-dienoyl]glycylamino]-L-glycero-B-L-manno-heptopyranosyl]adenine, aplidine, ecteinascidin, troxacitabine, aminopterin, 5-fluorouracil, floxuridine, methotrexate, leucovarin, hydroxyurea, thioguanine (6-TG), mercaptopurine (6-MP), cytarabine, pentostatin, fludarabine phosphate, cladribine (2-CDA), asparaginase, gemcitabine, alanosine, swainsonine, lometrexol, dexrazoxane, methioninase, and 3-aminopyridine-2-carboxaldehyde thiosemicarbazone; (6) prenyl-protein transferase inhibitor including farnesyl-protein transferase (FPTase), geranylgeranyl-protein transferase type I (GGPTase-I), and geranylgeranyl-protein transferase type-II (GGPTase-II, also called Rab GGPTase); (7) HMG-CoA reductase inhibitor such as lovastatin, simvastatin, pravastatin, atorvastatin, fluvastatin and rosuvastatin; (8) angiogenesis inhibitor such as inhibitors of the tyrosine kinase receptors Flt-1 (VEGFR1) and Flk-1/KDR (VEGFR2), inhibitors of epidermal-derived, fibroblast-derived, or platelet derived growth factors, MMP (matrix metalloprotease) inhibitors, integrin blockers, interferon-a, interleukin-12, erythropoietin (epoietin-α), granulocyte-CSF (filgrastin), granulocyte, macrophage-CSF (sargramostim), pentosan polysulfate, cyclooxygenase inhibitors, steroidal anti-inflammatories, carboxyamidotriazole, combretastatin A-4, squalamine, 6-O-chloroacetyl-carbonyl)-fumagillol, thalidomide, angiostatin, troponin-1, angiotensin II antagonists, heparin, carboxypeptidase U inhibitors, and antibodies to VEGF, endostatin, ukrain, ranpirnase, IM862, acetyldinanaline, 5-amino-1-[[3,5-dichloro-4-(4-chlorobenzoyl)phenyl]methyl]-1H-1,2,3-triazole-4-carboxamide,CM101, squalamine, combretastatin, RPI4610, NX31838, sulfated mannopentaose phosphate, and 3-[(2,4-dimethylpyrrol-5-yl)methylene]-2-indolinone (SU5416); (9) PPAR-γ agonists, PPAR-δ agonists, thiazolidinediones (such as DRF2725, CS-011, troglitazone, rosiglitazone, and pioglitazone), fenofibrate, gemfibrozil, clofibrate, GW2570, SB219994, AR-H039242, JTT-501, MCC-555, GW2331, GW409544, NN2344, KRP297, NP0110, DRF4158, NN622, GI262570, PNU182716, DRF552926, 2-[(5,7-dipropyl-3-trifluoromethyl-1,2-benzisoxazol-6-yl)oxy]-2-methylpropionic acid (disclosed in USSN 09/782,856), and (2R)-7-(3-(2-chloro-4-(4-fluorophenoxy)phenoxy)propoxy)-2-ethylchromane-2-carboxylic acid (disclosed in USSN 60/235,708 and 60/244,697); (9) inhibitor of inherent multidrug resistance including inhibitors of p-glycoprotein (P-gp), such as LY335979, XR9576, OC144-093, R101922, VX853 and PSC833 (valspodar); (10) inhibitor of cell proliferation and survival signaling such as inhibitors of EGFR (for example gefitinib and erlotinib), inhibitors of ERB-2 (for example trastuzumab), inhibitors of IGF1R such as MK-0646 (dalotuzumab), inhibitors of CD20 (rituximab), inhibitors of cytokine receptors, inhibitors of MET, inhibitors of PI3K family kinase (for example LY294002), serine/threonine kinases (including but not limited to inhibitors of Akt such as described in (WO 03/086404, WO 03/086403, WO 03/086394, WO 03/086279, WO 02/083675, WO 02/083139, WO 02/083140 and WO 02/083138), inhibitors of Raf kinase (for example BAY-43-9006), inhibitors of MEK (for example CI-1040 and PD-098059) and inhibitors of mTOR (for example Wyeth CCI-779 and Ariad AP23573); (11) a bisphosphonate such as etidronate, pamidronate, alendronate, risedronate, zoledronate, ibandronate, incadronate or cimadronate, clodronate, EB-1053, minodronate, neridronate, piridronate and tiludronate; (12) γ-secretase inhibitors, (13) agents that interfere with receptor tyrosine kinases (RTKs) including inhibitors of c-Kit, Eph, PDGF, Flt3 and c-Met; (14) agent that interferes with a cell cycle checkpoint including inhibitors of ATR, ATM, the Chk1 and Chk2 kinasesand cdk and cdc kinase inhibitors and are specifically exemplified by 7-hydroxystaurosporin, flavopiridol, CYC202 (Cyclacel) and BMS-387032; (15) BTK inhibitors such as PCI32765, AVL-292 and AVL-101; (16) PARP inhibitors including iniparib, olaparib, AGO 14699, ABT888 and MK4827; (16) ERK inhibitors; (17) mTOR inhibitors such as sirolimus, ridaforolimus, temsirolimus, everolimus; (18) cytotoxic/cytostatic agents.

"Cytotoxic/cytostatic agents" refer to compounds which cause cell death or inhibit cell proliferation primarily by interfering directly with the cell's functioning or inhibit or interfere with cell mytosis, including alkylating agents, tumor necrosis factors, intercalators, hypoxia activatable compounds, microtubule inhibitors/microtubule-stabilizing agents, inhibitors of mitotic kinesins, inhibitors of histone deacetylase, inhibitors of kinases involved in mitotic progression, antimetabolites; biological response modifiers; hormonal/anti-hormonal therapeutic agents, haematopoietic growth factors, monoclonal antibody targeted therapeutic agents, topoisomerase inhibitors, proteasome inhibitors and ubiquitin ligase inhibitors.

Examples of cytotoxic agents include, but are not limited to, sertenef, cachectin, chlorambucil, cyclophosphamide, ifosfamide, mechlorethamine, melphalan, uracil mustard, thiotepa, busulfan, carmustine, lomustine, streptozocin, tasonermin, lonidamine, carboplatin, altretamine, dacarbazine, procarbazine, prednimustine, dibromodulcitol, ranimustine, fotemustine, nedaplatin, oxaliplatin, temozolomide, heptaplatin, estramustine, improsulfan tosilate, trofosfamide, nimustine, dibrospidium chloride, pumitepa, lobaplatin, satraplatin, profiromycin, cisplatin, irofulven, dexifosfamide, cis-aminedichloro(2-methylpyridine)platinum, benzylguanine, glufosfamide, GPX100, (trans, trans, trans)-bis-mu-(hexane-1,6-diamine)-mu-[diamine-platinum(II)]bis[diamine(chloro)platinum(II)]tetrachloride, diarizidinylspermine, arsenic trioxide, 1-(11-dodecylamino-10-hydroxyundecyl)-3,7-dimethylxanthine, zorubicin, doxorubicin, daunorubicin, idarubicin, anthracenedione, bleomycin, mitomycin C, dactinomycin, plicatomycin, bisantrene, mitoxantrone, pirarubicin, pinafide, valrubicin, amrubicin, antineoplaston, 3'-deamino-3'-morpholino-13-deoxo-10-hydroxycarminomycin, annamycin, galarubicin, elinafide, MEN10755, and 4-demethoxy-3-deamino-3-aziridinyl-4-methylsulphonyl-daunorubicin.

An example of a hypoxia activatable compound is tirapazamine.

Examples of proteasome inhibitors include but are not limited to lactacystin and bortezomib.

Examples of microtubule inhibitors/microtubule-stabilising agents include vincristine, vinblastine, vindesine, vinzolidine, vinorelbine, vindesine sulfate, 3',4'-didehydro-4'-deoxy-8'-norvincaleukoblastine, podophyllotoxins (*e.g.,* etoposide (VP-16) and teniposide (VM-26)), paclitaxel, docetaxol, rhizoxin, dolastatin, mivobulin isethionate, auristatin, cemadotin, RPR109881, BMS184476, vinflunine, cryptophycin, anhydrovinblastine, N,N-dimethyl-L-valyl-L-valyl-N-methyl-L-valyl-L-prolyl-L-proline-t-butylamide, TDX258, the epothilones (see for example U.S. Pat. Nos. 6,284,781 and 6,288,237) and BMS188797.

Some examples of topoisomerase inhibitors are topotecan, hycaptamine, irinotecan, rubitecan, 6-ethoxypropionyl-3',4'-O-exo-benzylidene-chartreusin, lurtotecan, 7-[2-(N-isopropylamino)ethyl]-(20S)camptothecin, BNP1350, BNPI1100, BN80915, BN80942, etoposide phosphate, teniposide, sobuzoxane, 2'-dimethylamino-2'-deoxy-etoposide, GL331, N-[2-(dimethylamino)ethyl]-9-hydroxy-5,6-dimethyl-6H-pyrido[4,3-b]carbazole-1-carboxamide, asulacrine, 2,3-(methylenedioxy)-5-methyl-7-hydroxy-8-methoxybenzo[c]-phenanthridinium, 5-(3-aminopropylamino)-7,10-dihydroxy-2-(2-hydroxyethylaminomethyl)-6H-pyrazolo[4,5,1-de]acridin-6-one, N-[1-[2-(diethylamino)ethylamino]-7-methoxy-9-oxo-9H-thioxanthen-4-ylmethyl]formamide, N-(2-(dimethylamino)ethyl)acridine-4-carboxamide, 6-[[2-(dimethylamino)ethyl]amino]-3-hydroxy-7H-indeno[2,1-c]quinolin-7-one, and dimesna.

Examples of inhibitors of mitotic kinesins include, but are not limited to inhibitors of KSP, inhibitors of MKLP1, inhibitors of CENP-E, inhibitors of MCAK, inhibitors of Kif14, inhibitors of Mphosph1 and inhibitors of Rab6-KIFL.

Examples of "histone deacetylase inhibitors" include, but are not limited to, vorinostat, trichostatin A, oxamflatin, PXD101, MG98, valproic acid and scriptaid.

"Inhibitors of kinases involved in mitotic progression" include, but are not limited to, inhibitors of aurora kinase, inhibitors of Polo-like kinases (PLK; in particular inhibitors of PLK-1), inhibitors of bub-1 and inhibitors of bub-R1. An example of an "aurora kinase inhibitor" is VX-680.

"Antiproliferative agents" includes antisense RNA and DNA oligonucleotides such as G3139, ODN698, RVASKRAS, GEM231, and INX3001, and antimetabolites such as enocitabine, carmofur, tegafur, pentostatin, doxifluridine, trimetrexate, fludarabine, capecitabine, galocitabine, cytarabine ocfosfate, fosteabine sodium hydrate, raltitrexed, paltitrexid, emitefur, tiazofurin, decitabine, nolatrexed, pemetrexed, nelzarabine, 2'-deoxy-2'-methylidenecytidine, 2'-fluoromethylene-2'-deoxycytidine, N6-[4-deoxy-4-[N2-[2,4-tetradecadienoyl]glycylamino]-L-glycero-B-L-manno-heptopyranosyl]adenine, aplidine, ecteinascidin, troxacitabine, aminopterin, 5-flurouracil, floxuridine, methotrexate, leucovarin, hydroxyurea, thioguanine (6-TG), mercaptopurine (6-MP), cytarabine, pentostatin, fludarabine phosphate, cladribine (2-CDA), asparaginase, gemcitabine, alanosine, swainsonine, lometrexol, dexrazoxane, methioninase, and 3-aminopyridine-2-carboxaldehyde thiosemicarbazone.

Non-limiting examples of suitable agents used in cancer therapy that may be combined with compounds of Formula (I) include, but are not limited to, abarelix; aldesleukin; alemtuzumab; alitretinoin; allopurinol; altretamine; amifostine; anastrozole; arsenic trioxide; asparaginase; azacitidine; bendamustine; bevacuzimab; bexarotene; bleomycin; bortezomib; busulfan; calusterone; capecitabine; carboplatin; carmustine; cetuximab; chlorambucil; cisplatin; cladribine; clofarabine; cyclophosphamide; cytarabine; dacarbazine; dactinomycin, actinomycin D; dalteparin; darbepoetin alfa; dasatinib; daunorubicin; degarelix; denileukin diftitox; dexrazoxane; docetaxel; doxorubicin; dromostanolone propionate; eculizumab; Elliott's B Solution; eltrombopag; epirubicin; epoetin alfa; erlotinib; estramustine; etoposide phosphate; etoposide; everolimus; exemestane; filgrastim; floxuridine; fludarabine; fluorouracil; fulvestrant; gefitinib; gemcitabine; gemtuzumab ozogamicin; goserelin acetate; histrelin acetate; hydroxyurea; ibritumomab tiuxetan; idarubicin; ifosfamide; imatinib mesylate; interferon alfa 2a; interferon alfa-2b; irinotecan; ixabepilone; lapatinib; lenalidomide; letrozole; leucovorin; leuprolide acetate; levamisole; lomustine; meclorethamine, nitrogen mustard; megestrol acetate; melphalan, L-PAM; mercaptopurine; mesna; methotrexate; methoxsalen; mitomycin C; mitotane; mitoxantrone; nandrolone phenpropionate; nelarabine; nilotinib; Nofetumomab; ofatumumab; oprelvekin; oxaliplatin; paclitaxel; palifermin; pamidronat; panitumumab; pazopanib; pegademase; pegaspargase; Pegfilgrastim; pemetrexed disodium; pentostatin; pipobroman; plerixafor; plicamycin, mithramycin); porfimer sodium; pralatrexate; procarbazine; quinacrine; Rasburicase; raloxifene hydrochloride; Rituximab; romidepsin; romiplostim; sargramostim; sargramostim; satraplatin; sorafenib; streptozocin; sunitinib maleate; tamoxifen; temozolomide; temsirolimus; teniposide; testolactone; thioguanine; thiotepa; topotecan; toremifene; tositumomab; trastuzumab; tretinoin; uracil mustard; valrubicin; vinblastine; vincristine; vinorelbine; vorinostat; and zoledronate.

It will be clear to a person skilled in the art that, where appropriate, the other therapeutic ingredient(s) may be used in the form of salts, for example as alkali metal or amine salts or as acid addition salts, or prodrugs, or as esters, for example lower alkyl esters, or as solvates, for example hydrates, to optimise the activity and/or stability and/or physical characteristics, such as solubility, of the therapeutic ingredient. It will be clear also that, where appropriate, the therapeutic ingredients may be used in optically pure form.

The combinations referred to above may conveniently be presented for use in the form of a pharmaceutical composition and thus pharmaceutical compositions comprising a combination as defined above together with a pharmaceutically acceptable diluent, carrier or excipient represent a further aspect of the invention. These combinations are of particular interest in respiratory diseases and are conveniently adapted for inhaled or intranasal delivery.

The individual compounds of such combinations may be administered either sequentially or simultaneously in separate or combined pharmaceutical compositions. Preferably, the individual compounds will be administered simultaneously in a combined pharmaceutical composition. Appropriate doses of known therapeutic agents will be readily appreciated by those skilled in the art.

### Pharmaceutical Compositions

While it is possible that, for use in therapy, a compound of Formula (I), as well as salts, solvates and physiological functional derivatives thereof, may be administered as the raw chemical, it is possible to present the active ingredient as a pharmaceutical composition. Accordingly, the invention further provides a pharmaceutical composition which comprises a compound of Formula (I) and salts, solvates and physiological functional derivatives thereof, and one or more pharmaceutically acceptable carriers, diluents, or excipients. The compounds of the Formula (I) and salts, solvates and physiological functional derivatives thereof, are as described above. The carrier(s), diluent(s) or excipient(s) must be acceptable in the sense of being compatible with the other ingredients of the formulation and not deleterious to the recipient thereof. In accordance with another aspect of the invention there is also provided a process for the preparation of a pharmaceutical composition including admixing a compound of the Formula (I), or salts, solvates and physiological functional derivatives thereof, with one or more pharmaceutically acceptable carriers, diluents or excipients.

### Routes of Administration

Pharmaceutical compositions of the present invention may be presented in unit dose forms containing a predetermined amount of active ingredient per unit dose. Such a unit may contain, for example, 5µg to 1 g, preferably 1 mg to 700 mg, more preferably 5 mg to 100 mg of a compound of the Formula (I), depending on the condition being treated, the route of administration and the age, weight and condition of the patient. Such unit doses may therefore be administered more than once a day. Preferred unit dosage compositions are those containing a daily dose or sub-dose (for administration more than once a day), as herein above recited, or an appropriate fraction thereof, of an active ingredient. Furthermore, such pharmaceutical compositions may be prepared by any of the methods well known in the pharmacy art.

Pharmaceutical compositions of the present invention may be adapted for administration by any appropriate route, for example by the oral (including buccal or sublingual), rectal, topical, inhaled, nasal, ocular, sublingual, subcutaneous, local or parenteral (including intravenous and intramuscular) route, and the like, all in unit dosage forms for administration. Such compositions may be prepared by any method known in the art of pharmacy, for example by bringing into association the active ingredient with the carrier(s) or excipient(s). Dosage forms include tablets, troches, dispersions, suspensions, solutions, capsules, creams, ointments, aerosols, and the like.

In a further embodiment, the present invention provides a pharmaceutical composition adapted for administration by the oral route, for treating, for example, rheumatoid arthritis.

In a further embodiment, the present invention provides a pharmaceutical composition adapted for administration by the nasal route, for treating, for example, allergic rhinitis.

In a further embodiment, the present invention provides a pharmaceutical composition adapted for administration by the inhaled route, for treating, for example, asthma, Chronic Obstructive Pulmonary disease (COPD) or Acute Respiratory Distress Syndrome (ARDS).

In a further embodiment, the present invention provides a pharmaceutical composition adapted for administration by the ocular route, for treating, diseases of the eye, for example, conjunctivitis.

In a further embodiment, the present invention provides a pharmaceutical composition adapted for administration by the parenteral (including intravenous) route, for treating, for example, cancer.

For parenteral administration, the pharmaceutical composition of the invention may be presented in unit-dose or multi-dose containers, *e.g.,* injection liquids in predetermined amounts, for example in sealed vials and ampoules, and may also be stored in a freeze dried (lyophilized) condition requiring only the addition of sterile liquid carrier, *e.g.,* water, prior to use.

Mixed with such pharmaceutically acceptable auxiliaries, *e.g.,* as described in the standard reference, Gennaro, A.R. et al., Remington: The Science and Practice of Pharmacy (20th Edition., Lippincott Williams & Wilkins, 2000, see especially Part 5: Pharmaceutical Manufacturing), the active agent may be compressed into solid dosage units, such as pills, tablets, or be processed into capsules or suppositories. By means of pharmaceutically acceptable liquids the active agent can be applied as a fluid composition, *e.g.,* as an injection preparation, in the form of a solution, suspension, emulsion, or as a spray, *e.g.,* a nasal spray.

For making solid dosage units, the use of conventional additives such as fillers, colorants, polymeric binders and the like is contemplated. In general any pharmaceutically acceptable additive which does not interfere with the function of the active compounds can be used. Suitable carriers with which the active agent of the invention can be administered as solid compositions include lactose, starch, cellulose derivatives and the like, or mixtures thereof, used in suitable amounts. For parenteral administration, aqueous suspensions, isotonic saline solutions and sterile injectable solutions may be used, containing pharmaceutically acceptable dispersing agents and/or wetting agents, such as propylene glycol or butylene glycol.

Pharmaceutical compositions of the present invention which are adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

For instance, for oral administration in the form of a tablet or capsule, the active drug component can be combined with an oral, non-toxic pharmaceutically acceptable inert carrier such as ethanol, glycerol, water and the like. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules are made by preparing a powder mixture, as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like. Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by wetting with a binder such as syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with a free flowing inert carrier and compressed into tablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of the compound. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners, and the like can also be added.

Where appropriate, dosage unit compositions for oral administration can be microencapsulated. The formulation can also be prepared to prolong or sustain the release, for example, by coating or embedding particulate material in polymers, wax or the like.

The compounds of Formula (I), and salts, solvates and physiological functional derivatives thereof, can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The compounds of Formula (I) and salts, solvates and physiological functional derivatives thereof may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxypropylmethacrylamide-phenol, polyhydroxyethylaspartamidephenol, or polyethyleneoxidepolylysine substituted with palmitoyl residues. Furthermore, the compounds may be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels.

Dosage forms for inhaled administration may conveniently be formulated as aerosols or dry powders.

For compositions suitable and/or adapted for inhaled administration, it is preferred that the compound or salt of Formula (I) is in a particle-size-reduced form, and more preferably the size-reduced form is obtained or obtainable by micronisation. The preferable particle size of the size-reduced (*e.g.,* micronised) compound or salt or solvate is defined by a D50 value of about 0.5 to about 10 microns (for example as measured using laser diffraction).

Aerosol formulations, *e.g.,* for inhaled administration, can comprise a solution or fine suspension of the active substance in a pharmaceutically acceptable aqueous or non-aqueous solvent. Aerosol formulations can be presented in single or multidose quantities in sterile form in a sealed container, which can take the form of a cartridge or refill for use with an atomising device or inhaler. Alternatively the sealed container may be a unitary dispensing device such as a single dose nasal inhaler or an aerosol dispenser fitted with a metering valve (metered dose inhaler) which is intended for disposal once the contents of the container have been exhausted.

Where the dosage form comprises an aerosol dispenser, it preferably contains a suitable propellant under pressure such as compressed air, carbon dioxide or an organic propellant such as a hydrofluorocarbon (HFC). Suitable HFC propellants include 1,1,1,2,3,3,3-heptafluoropropane and 1,1,1,2-tetrafluoroethane. The aerosol dosage forms can also take the form of a pump-atomiser. The pressurised aerosol may contain a solution or a suspension of the active compound. This may require the incorporation of additional excipients *e.g.,* co-solvents and/or surfactants to improve the dispersion characteristics and homogeneity of suspension formulations. Solution formulations may also require the addition of co-solvents such as ethanol. Other excipient modifiers may also be incorporated to improve, for example, the stability and/or taste and/or fine particle mass characteristics (amount and/or profile) of the formulation.

For pharmaceutical compositions suitable and/or adapted for inhaled administration, it is preferred that the pharmaceutical composition is a dry powder inhalable composition. Such a composition can comprise a powder base such as lactose, glucose, trehalose, mannitol or starch, the compound of Formula (I) or salt or solvate thereof (preferably in particle-size-reduced form, *e.g.,* in micronised form), and optionally a performance modifier such as L-leucine or another amino acid, and/or metals salts of stearic acid such as magnesium or calcium stearate. Preferably, the dry powder inhalable composition comprises a dry powder blend of lactose and the compound of Formula (I) or salt thereof. The lactose is preferably lactose hydrate *e.g.,* lactose monohydrate and/or is preferably inhalation-grade and/or fine-grade lactose. Preferably, the particle size of the lactose is defined by 90% or more (by weight or by volume) of the lactose particles being less than 1000 microns (micrometres) (*e.g.,* 10-1000 microns *e.g.,* 30-1000 microns) in diameter, and/or 50% or more of the lactose particles being less than 500 microns (*e.g.,* 10-500 microns) in diameter. More preferably, the particle size of the lactose is defined by 90% or more of the lactose particles being less than 300 microns (*e.g.,* 10-300 microns *e.g.,* 50-300 microns) in diameter, and/or 50% or more of the lactose particles being less than 100 microns in diameter. Optionally, the particle size of the lactose is defined by 90% or more of the lactose particles being less than 100-200 microns in diameter, and/or 50% or more of the lactose particles being less than 40-70 microns in diameter. It is preferable that about 3 to about 30% (*e.g.,* about 10%) (by weight or by volume) of the particles are less than 50 microns or less than 20 microns in diameter. For example, without limitation, a suitable inhalation-grade lactose is E9334 lactose (10% fines) (Borculo Domo Ingredients, Hanzeplein 25, 8017 J D Zwolle, Netherlands).

Optionally, in particular for dry powder inhalable compositions, a pharmaceutical composition for inhaled administration can be incorporated into a plurality of sealed dose containers (*e.g.,* containing the dry powder composition) mounted longitudinally in a strip or ribbon inside a suitable inhalation device. The container is rupturable or peel-openable on demand and the dose of *e.g.,* the dry powder composition can be administered by inhalation via the device such as the DISKUS® device(GlaxoSmithKline). Other dry powder inhalers are well known to those of ordinary skill in the art, and many such devices are commercially available, with representative devices including Aerolizer® (Novartis), Airmax™ (IVAX), ClickHaler® (Innovata Biomed), Diskhaler® (GlaxoSmithKline), Accuhaler (GlaxoSmithKline), Easyhaler® (Orion Pharma), Eclipse™ (Aventis), FlowCaps® (Hovione), Handihaler® (Boehringer Ingelheim), Pulvinal® (Chiesi), Rotahaler® (GlaxoSmithKline), SkyeHaler™ or Certihaler™ (SkyePharma), Twisthaler (Schering-Plough), Turbuhaler® (AstraZeneca), Ultrahaler® (Aventis), and the like.

Dosage forms for ocular administration may be formulated as solutions or suspensions with excipients suitable for ophthalmic use.

Dosage forms for nasal administration may conveniently be formulated as aerosols, solutions, drops, gels or dry powders.

Pharmaceutical compositions adapted for administration by inhalation include fine particle dusts or mists, which may be generated by means of various types of metered, dose pressurized aerosols, nebulizers or insufflators.

For pharmaceutical compositions suitable and/or adapted for intranasal administration, the compound of Formula (I) or a pharmaceutically acceptable salt or solvate thereof may be formulated as a fluid formulation for delivery from a fluid dispenser. Such fluid dispensers may have, for example, a dispensing nozzle or dispensing orifice through which a metered dose of the fluid formulation is dispensed upon the application of a user-applied force to a pump mechanism of the fluid dispenser. Such fluid dispensers are generally provided with a reservoir of multiple metered doses of the fluid formulation, the doses being dispensable upon sequential pump actuations. The dispensing nozzle or orifice may be configured for insertion into the nostrils of the user for spray dispensing of the fluid formulation into the nasal cavity. A fluid dispenser of the aforementioned type is described and illustrated in WO-A-2005/044354, the entire content of which is hereby incorporated herein by reference. The dispenser has a housing which houses a fluid discharge device having a compression pump mounted on a container for containing a fluid formulation. The housing has at least one finger-operable side lever which is movable inwardly with respect to the housing to cam the container upwardly in the housing to cause the pump to compress and pump a metered dose of the formulation out of a pump stem through a nasal nozzle of the housing. A particularly preferred fluid dispenser is of the general type illustrated in FIGS. 30-40 of WO-A-2005/044354.

The invention further includes a pharmaceutical composition of a compound of Formula (I) or pharmaceutically acceptable salts thereof, as hereinbefore described, in combination with packaging material suitable for said composition, said packaging material including instructions for the use of the composition for the use as hereinbefore described.

The following are examples of representative pharmaceutical dosage forms for the compounds of this invention:

| Injectable Suspension (I.M.) | mg/mL |
|---|---|
| Compound of Formula (I) | 10 |
| Methylcellulose | 5.0 |
| Tween 80 | 0.5 |
| Benzyl alcohol | 9.0 |
| Benzalkonium chloride | 1.0 |
| Water for injection to a total volume of 1 mL | |

| Tablet | mg/tablet |
|---|---|
| Compound of Formula (I) | 25 |
| Microcrystalline Cellulose | 415 |
| Providone | 14.0 |
| Pregelatinized Starch | 43.5 |
| Magnesium Stearate | 2.5 |
| | 500 |

| Capsule | mg/capsule |
|---|---|
| Compound of Formula (I) | 25 |
| Lactose Powder | 573.5 |
| Magnesium Stearate | 1.5 |
| | 600 |

| Aerosol | Per canister |
|---|---|
| Compound of Formula (I) | 24 mg |
| Lecithin, NF Liquid Concentrate | 1.2 mg |
| Trichlorofluoromethane, NF | 4.025 gm |
| Dichlorodifluoromethane, NF | 12.15 gm |

It will be appreciated that when the compound of the present invention is administered in combination with other therapeutic agents normally administered by the inhaled, intravenous, oral or intranasal route, that the resultant pharmaceutical composition may be administered by the same routes.

It should be understood that in addition to the ingredients particularly mentioned above, the compositions may include other agents conventional in the art having regard to the type of formulation in question, for example those suitable for oral administration may include flavoring agents.

A therapeutically effective amount of a compound of the present invention will depend upon a number of factors including, for example, the age and weight of the animal, the precise condition requiring treatment and its severity, the particular compound having Formula (I), the nature of the formulation, and the route of administration, and will ultimately be at the discretion of the attendant physician or veterinarian. However, an effective amount of a compound of Formula (I) for the treatment of diseases or conditions associated with inappropriate Btk activity, will generally be in the range of 5 µg to 100 mg/kg body weight of recipient (mammal) per day and more usually in the range of 5 µg to 10 mg/kg body weight per day. This amount may be given in a single dose per day or more usually in a number (such as two, three, four, five or six) of sub-doses per day such that the total daily dose is the same. An effective amount of a salt or solvate, thereof, may be determined as a proportion of the effective amount of the compound of Formula (I) per se.

In general parenteral administration requires lower dosages than other methods of administration which are more dependent upon absorption. However, a dosage for humans preferably contains 0.0001-25 mg of a compound of Formula (I) or pharmaceutically acceptable salts thereof per kg body weight. The desired dose may be presented as one dose or as multiple subdoses administered at appropriate intervals throughout the day. The dosage as well as the regimen of administration may differ between a female and a male recipient.

### General Synthesis

The compounds of the present invention can be prepared by methods well known in the art of organic chemistry. See, for example, J. March, 'Advanced Organic Chemistry' 4thEdition, John Wiley and Sons. During synthetic sequences it may be necessary and/or desirable to protect sensitive or reactive groups on any of the molecules concerned. This is achieved by means of conventional protecting groups, such as those described in T.W. Greene and P.G.M. Wutts 'Protective Groups in Organic Synthesis' 3rd Edition, John Wiley and Sons, 1999. The protective groups are optionally removed at a convenient subsequent stage using methods well known in the art.

The products of the reactions are optionally isolated and purified, if desired, using conventional techniques, but not limited to, filtration, distillation, crystallization, chromatography and the like. Such materials are optionally characterized using conventional means, including physical constants and spectral data.

The compounds of Formula (I) can be prepared by the general synthetic routes shown in the schemes below.

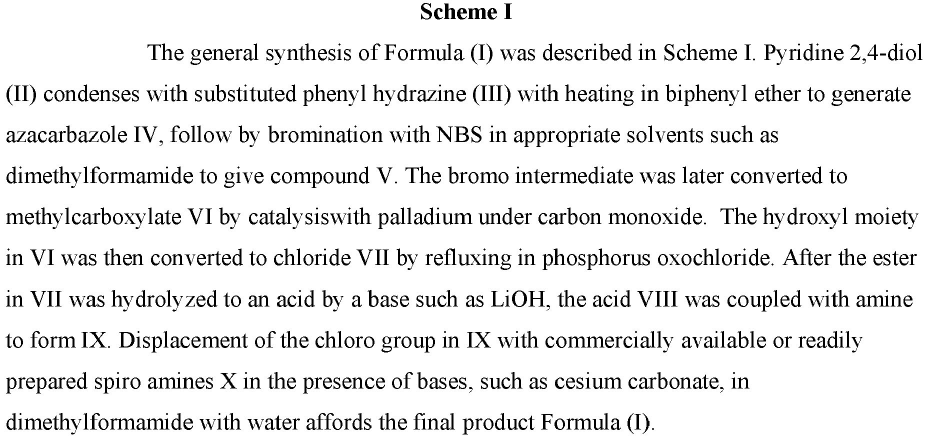

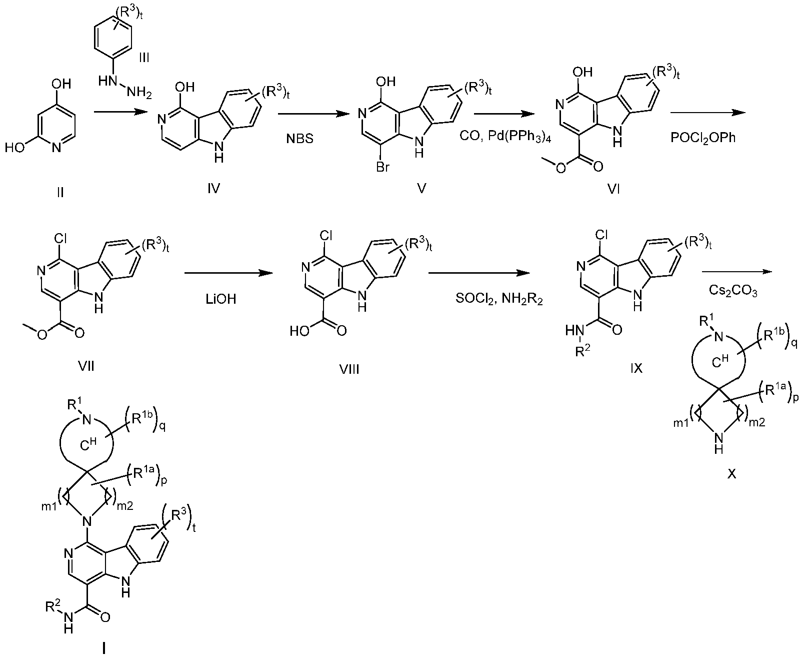

The following abbreviations are used throughout the application with respect to chemical terminology:
- 2-BuOH: 2-Butanol
- DMAP: 4-Dimethylaminopyridine
- aq: Aqueous
- ATP: Adenosine triphosphate
- Cbz: Benzyloxycarbonyl
- Cbz-Cl: Benzylchloroformate
- CDCl₃: Deuterochloroform
- CD₃OD: Tetradeuteromethanol
- CDI: 1,1'-Carbonyl diimidazole
- DCM: Dichloromethane
- DEA: Diethylamine
- DIPEA: N,N-Diisopropylethylamine
- DME: Dimethoxyethane
- DMF: N,N-Dimethylformamide
- DMSO: Dimethyl sulfoxide
- DTT: Dithiothreitol
- EGTA: Ethylene glycol tetraacetic acid
- Et₂O: Diethylether
- Et₃N: Triethylamine
- EtOAc: Ethyl acetate
- EtOH: Ethanol
- HOAc: Acetic acid
- HPLC: High Pressure Liquid Chromatography
- *i*-PrOH: 2-Propanol
- LCMS: Liquid Chromatography / Mass Spectrometry
- LiHMDS: Lithium hexamethyldisilazide
- MeCN: Acetonitrile
- MeI: Iodomethane
- MeOH: Methanol
- MsCl: Methanesulfonyl chloride
- NBS: N-Bromosuccinimide
- NIS: N-Iodosuccinimide
- NMP: N-Methyl-2-pyrrolidone
- n-BuLi: n-Butyllithium
- Pd/C: Palladium-on-carbon
- Pd(dppf)Cl₂: 1,1'-Bis(diphenylphosphino)ferrocene palladium (II) chloride,
- Pd(OAc)₂: Palladium(II) acetate
- PE: Petroleum ether
- Ph: Phenyl
- Prep-HPLC: Preparative High Performance Liquid Chromatography
- PyBOP: O-Benzotriazole-1-yl-oxy-trispyrrolidinophosphonium
- rt: Room Temperature
- satd.: Saturated
- SFC: Supercritical fluid chromatography
- TBAF: tetrabutylammonium fluoride
- TBSCl: *tert*-Butyldimethylsilyl chloride
- TEA: Triethylamine
- TFA: Trifluoroacetic acid
- THF: Tetrahydrofuran
- TMSCH₂N₂: Trimethylsilyldiazomethane
- Tris-HCl: Tris(hydroxymethyl)aminomethane hydrochloride
- V:V: volume/volume
- Xantphos: 4,5-Bis(diphenylphosphino)-9,9-dimethylxanthene
- X-phos: 2-Dicyclohexylphosphino-2',4',6'-triisopropylbiphenyl The invention is illustrated by the following examples.

### EXAMPLES

The following examples are illustrative embodiments of the invention, not limiting the scope of the invention in any way. Reagents are commercially available or are prepared according to procedures in the literature.

Mass Spectrometry: Electron Spray spectra were recorded on the Applied Biosystems API-165 single quad mass spectrometer in alternating positive and negative ion mode using Flow Injection. The mass range was 120-2000 Da. and scanned with a step rate of 0.2 Da. and the capillary voltage was set to 5000 V. N₂gas was used for nebulisation.

LC-MS spectrometer (Waters) Detector: PDA (200-320 nm), Mass detector: ZQ and Eluent: A: acetonitrile with 0.05% trifluoroacetic acid, B: acetronitrile/water = 1/9 (v/v) with 0.05% trifluoroacetic acid.

### Method A:

Sample Info : Easy-Access Method: '1-Short_TFA_Pos'
Method Info : B222 Column Agilent SBC (3.0x50 mm, 1.8 µm); Flow 1.0 mL/min; solvent A: H2O-0.1% TFA;
solvent B: MeCN-0.1% TFA;
GRADIENT TABLE: 0 min:10% B, 0.3 min:10%B, 1.5min: 95% B, 2.70min: 95% B, 2.76 min:10% B
stop time 3.60 min, PostTime 0.70 min.

### Method B:

Sample Info : Easy-Access Method: '1_Fast'
Method Info : A330 Column Agilent Zorbax SB-C18 (2.1x30 mm, 3.5 µm); Flow 2.0 mL/min;
solvent A: H2O-0.1% TFA;
solvent B: MeCN-0.1% TFA;
GRADIENT TABLE: 0.01 min:10% B, 1.01 min:95% B, 1.37 min:95% B, 1.38 min:10% B, stop time 1.7min, PostTime=OFF

Proton nuclear magnetic resonance (¹H NMR) spectra and carbon-13 nuclear magnetic resonance (13C NMR) spectra were recorded on spectrometersat the frequencies in the solvents indicated and referenced totetramethylsilane (TMS). Chemical shifts (δ) are reported in parts per million (ppm). Coupling constants (*J*) are reported in Hertz (Hz). The followingabbreviations are used: s (singlet), br s (broad singlet), d (doublet), t(triplet), q (quartet), and m (multiplet).

### Intermediate 1:

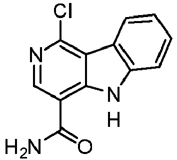

### 1-chloro-5H-pyrido[4,3-b]indole-4-carboxamide

### Step 1: 5H-pyrido[4,3-b]indol-1-ol

Into a 10000-mL 4-necked round-bottom flask was placed pyridine-2,4-diol (560 g, 5.04 mol, 1.00 equiv), phenylhydrazine (1800 g, 16.65 mol, 3.33 equiv), and diphenyl ether (4000 mL). The resulting solution was stirred for 24 h at 230°C. The resulting solution was cooled to room temperature and diluted with 2000 mL of toluene. The solid was collected by filtration and washed with MeOH. This resulted in 5H-pyrido[4,3-b]indol-1-ol as a solid.

### Step 2: 4-bromo-5H-pyrido[4,3-b]indol-1-ol

Into a 1000-mL 4-necked round-bottom flask was placed a solution of 5H-pyrido[4,3-b]indol-1-ol (500 g, 2.71 mol, 1.00 equiv) in N,N-dimethylformamide (3000 mL). This was followed by the addition of N-bromosuccinimide (570 g, 3.20 mol, 1.19 equiv), in portions at below 20°C. The resulting solution was stirred for 30 minutes at room temperature. The reaction was then quenched by pouring into 5000 mL of water. The solid was collected by filtration to provide 4-bromo-5H-pyrido[4,3-b]indol-1-ol as a solid.

### Step 3: methyl 1-hydroxy-5H-pyrido[4,3-b]indole-4-carboxylate

Into a 20000-mL pressure tank reactor (2.5 MPa) was placed a solution of 4-bromo-5H-pyrido[4,3-b]indol-1-ol (300 g, 1.14 mol, 1.00 equiv) in methanol (12000 mL), Pd(PPh₃)₂Cl₂ (192 g, 273.54 mmol, 0.01 equiv), and TEA (192 g, 1.90 mol, 1.66 equiv). The reactor was charged with CO gas and stirred for 2 days at 150°C. The solid was collected by filtration to afford methyl 1-hydroxy-5H-pyrido[4,3-b]indole-4-carboxylate as a solid.

### Step 4: methyl 1-chloro-5H-pyrido[4,3-b]indole-4-carboxylate

Into a 2000-mL 4-necked round-bottom flask was placed methyl 1-hydroxy-5H-pyrido[4,3-b]indole-4-carboxylate (200 g, 825.66 mmol, 1.00 equiv), and phenoxyphosphonoyl dichloride (1000 mL). After stirring for 1 hour at 140°C, the resulting solution was cooled to room temperature. The solid was collected by filtration to provide methyl 1-chloro-5H-pyrido[4,3-b]indole-4-carboxylate as a solid.

### Step 5: 1-chloro-5H-pyrido[4,3-b]indole-4-carboxylic acid

Into a 10000-mL 4-necked round-bottom flask was placed a solution of methyl 1-chloro-5H-pyrido[4,3-b]indole-4-carboxylate (248 g, 951.37 mmol, 1.00 equiv) in 1,4-dioxane (3000 mL). This was followed by the addition of aq. LiOH (1M, 5400 mL, 5.00 equiv) dropwise with stirring at 0°C. The resulting solution was stirred for 1 hour at room temperature. The pH of the solution was adjusted to 2 with HCl (1M). The solid was collected by filtration to provide 1-chloro-5H-pyrido[4,3-b]indole-4-carboxylic acid as a solid.

### Step 6: 1-chloro-5H-pyrido[4,3-b]indole-4-carbonyl chloride

Into a 5000-mL round-bottom flask was placed 1-chloro-5H-pyrido[4,3-b]indole-4-carboxylic acid (230 g, 932.50 mmol, 1.00 equiv), and thionyl chloride (3000 mL). The resulting solution was heated to reflux for 2 h in an oil bath. The resulting mixture was cooled and concentrated under vacuum to provide 1-chloro-5H-pyrido[4,3-b]indole-4-carbonyl chloride as a solid.

### Step 7: 1-chloro-5H-pyrido[4,3-b]indole-4-carboxamide

Into a 5000-mL 4-necked round-bottom flask was placed a solution of 1-chloro-5H-pyrido[4,3-b]indole-4-carbonyl chloride (204 g, 769.54 mmol, 1.00 equiv) in 1,4-dioxane (2500 mL). The solution was bubbled slowly with NH₃ (gas) at 0°C. The resulting solution was stirred for 2 h at room temperature. The solid was collected by filtration to provide 1-chloro-5H-pyrido[4,3-b]indole-4-carboxamide as a solid. (ES, *m*/*z*)*:* 245 [M+H]⁺. ¹H-NMR (300MHz, DMSO-*d*₆): δ 7.35 (1H, m), 7.549 (1H, m), 7.847 (2H, m), 8.381 (2H, m), 8.787 (1H, s), 12.164 (1H, s) ppm.

### Intermediate 2:

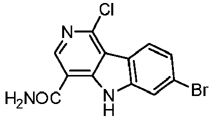

### 7-bromo-1-chloro-5H-pyrido[4,3-b]indole-4-carboxamide

### Step 1: 7-bromo-5H-pyrido[4,3-b]indol-1-ol

Into a 10000-mL 4-necked round-bottom flask was placed a solution of pyridine-2,4-diol (500 g, 4.50 mol, 1.00 equiv) in phenyl ether (4000 mL), and (3-bromophenyl)hydrazine (1675 g, 8.96 mol, 2.00 equiv). The resulting solution was stirred for 6 h at 235°C. The resulting solution was cooled and diluted with 2000 mL of methylbenzene. The solids were collected by filtration to provide 7-bromo-5H-pyrido[4,3-b]indol-1-ol as a solid.

### Step 2: 7-bromo-4-iodo-5H-pyrido[4,3-b]indol-1-ol

Into a 10000-mL 4-necked round-bottom flask was placed a solution of 7-bromo-5H-pyrido[4,3-b]indol-1-ol (729 g, 2.77 mol, 1.00 equiv) in N,N-dimethylformamide (5000 mL), and acetic acid (390 mL). This was followed by the addition of N-iodo-succinimide (960 g, 4.27 mol, 1.50 equiv), in portions at<20°C. The resulting solution was stirred for 1 hour at room temperature. The reaction was then quenched by pouring into 15 L of water. The solids were collected by filtration to provide 7-bromo-4-iodo-5H-pyrido[4,3-b]indol-1-ol as a solid.

### Step 3: methyl 7-bromo-1-hydroxy-5H-pyrido[4,3-b]indole-4-carboxylate

Into a 20-L pressure tank reactor (15 MPa) was placed a solution of 7-bromo-4-iodo-5H-pyrido[4,3-b]indol-1-ol (300 g, 771.23 mmol, 1.00 equiv) in methanol (12 mL), Pd(ppH₃)₂Cl₂ (43.2 g, 61.55 mmol, 0.08 equiv), and TEA (128.4 mL, 1.20 equiv). The reactor was charged with CO and stirred for 24 h at 70°C. The solids were collected by filtrationto provide methyl 7-bromo-1-hydroxy-5H-pyrido[4,3-b]indole-4-carboxylate as a solid.

### Step 4: methyl 7-bromo-1-chloro-5H-pyridor[4,3-b]indole-4-carboxylate

Into a 10000-mL 4-necked round-bottom flask was placed a solution of methyl 7-bromo-1-hydroxy-5H-pyrido[4,3-b]indole-4-carboxylate (380 g, 1.18 mol, 1.00 equiv) in 1,4-dioxane (4000 mL) and POCl₃ (320 mL). The resulting solution was stirred for 1 hour at 108°C in an oil bath. The solids were collected by filtration and purified by silica gel column chromatography elutingwith THF: PE (1:10) to provide methyl 7-bromo-1-chloro-5H-pyrido[4,3-b]indole-4-carboxylate as a solid.

### Step 5: 7-bromo-1-chloro-5H-pyrido[4,3-b]indole-4-carboxylic acid

Into a 1000-mL 4-necked round-bottom flask was placed a solution of methyl 7-bromo-1-chloro-5H-pyrido[4,3-b]indole-4-carboxylate (53 g, 156.08 mmol, 1.00 equiv) in 1,4-dioxane (427 mL). This was followed by the addition of a solution of LiOH (25 g, 595.24 mmol, 3.80 equiv) in water (427 mL) dropwise with stirring at <20°C. The resulting solution was stirred for 1 hour at room temperature. The pH of the solution was adjusted to 1 with HCl (1M). The solids were collected by filtration to provide 7-bromo-1-chloro-5H-pyrido[4,3-b]indole-4-carboxylic acid as a solid.

### Step 6: 7-bromo-1-chloro-5H-pyrido[4,3-b]indole-4-carboxamide

Into a 5000-mL 4-necked round-bottom flask was placed a solution of 7-bromo-1-chloro-5H-pyrido[4,3-b]indole-4-carboxylic acid (50.8 g, 156.05 mmol, 1.00 equiv) in thionyl chloride (500 mL). This solution was heated at reflux for 2 h. Then it was concentrated under vacuum. The residue was dissolved in 1,4-dioxane (1200 mL). The solution was bubbled with NH₃ (g) at 0°C until the reaction mixture was made alkaline. The resulting solution was stirred for 1 hour at room temperature. The reaction was then quenched by pouring into 5000 mL of water. The solids were collected by filtration to provide 7-bromo-1-chloro-5H-pyrido[4,3-b]indole-4-carboxamide as a solid. LC-MS (ES, *m*/*z*): 324 [M+H]⁺, ¹H-NMR (CDCl₃): δ 7.546 (1H, d), 7.806 (1H, s), 7.948 (1H, s), 8.034 (2H, m), 8.820 (1H, s), 12.357(1H, s) ppm.

### Intermediate 3

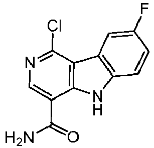

### 1-chloro-8-fluoro-5H-pyrido[4,3-b]indole-4-carboxamide

### Step 1: 8-fluoro-5H-pyrido[4,3-b]indol-1-ol

A flask equipped with a Dean-Stark trap was charged with biphenyl ether(1000 mL)and pyridine- 2,4-diol (67 g,0.6 mol), followed with (4-fluoro-phenyl)-hydrazine (242 g, 1.92 mol). The mixture was heated to 230 °C for 5h. Upon cooling, toluene was added and the precipitate was collected, washed with petroleum ether and methanol to afford 8-fluoro-5H-pyrido[4,3-b]indol-1-ol. ¹H NMR(400 MHz, DMSO-*d*₆) δ 11.79 (s, 1H), 11.14 (s, 1H), 7.73 (dd,*J*₁= 2.8 Hz, *J*₂= 9.6 Hz,1H), 7.47 (dd,*J*₁= 4.4 Hz, *J*₂= 8.8 Hz,1H), 7.31 (t,*J*= 6.0 Hz,1H), 7.09 ∼ 7.15 (m, 1H), 6.49 (d,*J*= 7.2 Hz,1H) ppm.

### Step 2: 4-bromo-8-fluoro-5H-pyrido[4,3-b]indol-1-ol

To a solution of 8-fluoro-5H-pyrido[4,3-b]indol-1-ol (57 g, 281.9 mmol) in DMF (600 mL) was added NBS (50.7 g, 284.7 mmol) at 0 °C, and stirred for 5 min. After stirring at 25 °C for 3 h, water (800 mL) was added slowly at 0 °C. And then precipitate was collected with filtration to afford 4-bromo-8-fluoro-5H-pyrido[4,3-b]indol-1-ol. ¹H (400 MHz, DMSO-*d*₆) δ 12.07 (s, 1H), 7.95 (s, 1H), 7.74 (dd,*J*₁= 2.4 Hz, *J*₂= 9.2 Hz,1H), 7.62 (s, 1H), 7.55 (dd,*J*₁= 4.8 Hz, *J*₂= 8.8 Hz,1H), 7.17 ∼ 7.23 (m, 1H) ppm.

### Step 3: methyl 8-fluoro-1-hydroxy-5H-pyrido[4,3-b]indole-4-carboxylate

A solution of 4-bromo-8-fluoro-5H-pyrido[4,3-b]indol-1-ol (60 g, 275.1 mmol), Et₃N (55.7 g, 550.2 m mol), Pd(PPh₃)₄ (10 g, 8.2 mmol) in methanol (4 L) was stirred under CO atmosphere (2.5 Mpa) at 150 °C for 48h. The reaction mixture was filtered to afford methyl 8-fluoro-1-hydroxy-5H-pyrido[4,3-b]indole-4-carboxylate. ¹H NMR (400 MHz, DMSO-*d*₆) δ11.78 (s, 1H), 8.08 (s, 1H), 7.75 (dd,*J*₁= 2.4 Hz, *J*₂= 9.2 Hz,1H), 7.70 (dd,*J*₁= 4.8 Hz, *J*₂= 8.8 Hz,1H), 7.17 ∼ 7.22 (m, 1H), 3.91 (s, 3H) ppm.

### Step 4: methyl 1-chloro-8-fluoro-5H-pyrido[4,3-b]indole-4-carboxylate

A solution of methyl 8-fluoro-1-hydroxy-5H-pyrido[4,3-b]indole-4-carboxylate (26 g, 0.1 mol) in POCl₂OPh (300 mL) was heated at 140 °C for 1h. Isopropyl ether (300 mL) was added into the reaction after cooling down to rt. The precipitate was collected by filtration and washed with petroleum ether to givemethyl 1-chloro-8-fluoro-5H-pyrido[4,3-b]indole-4-carboxylate. ¹H NMR (400 MHz, DMSO-*d*₆) δ 12.28 (s, 1H), 8.81 (s, 1H), 8.10 ∼ 8.13 (m, 1H), 7.85 (dd,*J*₁= 4.4 Hz, *J*₂= 8.8 Hz,1H), 7.49 ∼ 7.54 (m, 1H), 4.02 (s, 3H) ppm.

### Step 5: 1-chloro-8-fluoro-5H-pyrido[4,3-b]indole-4-carboxylic acid

To a solution of methyl 1-chloro-8-fluoro-5H-pyrido[4,3-b]indole-4-carboxylate (22.5 g, 80.7 mmol) in dioxane (400 mL) was added LiOH (21 g, 0.5 mol) slowly at 0 °C, and stirred for 3 h. The reaction mixture was acidified to pH4-5 with hydrochloric acid (1 M), and filtered to afford the 1-chloro-8-fluoro-5H-pyrido[4,3-b]indole-4-carboxylic acid. ¹H NMR (300MHz, DMSO-*d*₆) δ 12.23 (s, 1H), 8.77 (s, 1H), 8.08 ∼ 8.12 (m, 1H), 7.86 (dd,*J*₁= 1.5 Hz, *J*₂= 9.0 Hz,1H), 7.45 ∼ 7.53 (m, 1H) ppm.

### Step 6:1-chloro-8-fluoro-5H-pyrido[4,3-b]indole-4-carboxamide

A mixture of 1-chloro-8-fluoro-5H-pyrido[4,3-b]indole-4-carboxylic acid (17.5 g, 66.1 mmol) and SOCl₂ (200 mL) was heated at 120 °C for 2h. The reaction mixture was evaporated to afford the residue, which was dissolved in mixture of dry dioxane (200 mL) and liquid NH₃ (6 mL) at 0 °C. After stirred for 2h, water (1 L) was added and stirred for 1h. 1-chloro-8-fluoro-5H-pyrido[4,3-b]indole-4-carboxamide was obtained after filtration. 1HNMR (300MHz, DMSO-*d*₆) δ = 12.23 (s, 1H), 8.79 (s, 1H), 8.36 (s, 1H), 8.08 (dd,*J*₁= 2.7 Hz, *J*₂= 9.3 Hz,1H), 7.85 (dd,J1 = 1.8 Hz, J2 = 9.3 Hz,1H), 7.78 (s, 1H), 7.42 ∼ 7.50 (m, 1H) ppm.

### Intermediate 4

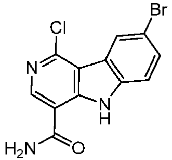

### 8-bromo-1-chloro-5H-pyrido[4,3-b]indole-4-carboxamide

To a solution of Intermediate 1 (500 mg, 2.04 mmol) in CH₃COOH (20 mL) was added NBS (381 mg, 2.14 mmol) and then stirring at rt overnight. The mixture was poured into H₂O (100 mL), filtered to afford 8-bromo-1-chloro-5H-pyrido[4,3-b]indole-4-carboxamide.¹HNMR (300MHz, DMSO-*d*₆) δ12.30 (s, 1H), 8.80 (s,1 H),8.46 (s, 1 H), 8.35-8.38 (m, 1 H), 7.69-7.82 (m, 3 H) ppm.

### Intermediate 5

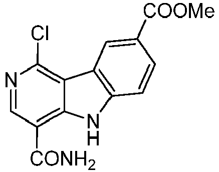

### Methyl 4-carbamoyl-1-chloro-5H-pyrido[4,3-b]indole-8-carboxylate

### Step 1: 1-chloro-8-iodo-5H-pyrido[4,3-b]indole-4-carboxamide

To the mixture of Intermediate 1(1 g, 4.07 mmol) in 30 mL of CF₃COOH was added NIS (0.916 g, 4.07 mmol), then the mixture was stirred at room temperature overnight. Water (200 mL) was added into the mixture after it was cooled to room temperature, which was filtered and washed with water to yield 1-chloro-8-iodo-5H-pyrido[4,3-b]indole-4-carboxamide, which was used in the next step without further purification. ¹HNMR(300MHz, DMSO-*d*₆)δ 12.33 (s, 1 H), 8.67 (s, 1 H), 8.61 (s,1 H), 8.37 (s, 1 H), 7.83 (d, *J*=8.4 Hz, 1 H), 7.61 (d, *J*=8.4 Hz, 1 H) ppm.

### Step 2: 4-carbamoyl-1-chloro-5H-pyrido[4,3-b]indole-8-carboulate

To the solution of 1-chloro-8-iodo-5H-pyrido[4,3-b]indole-4-carboxamide (25.5 g, 68.6 mmol) in the mix solution of MeOH (0.5 L) and DMF (0.5 L) was added Pd(dppf)Cl₂ (2.5 g, 3.43 mmol) and Et₃N (35 mL, 480.2 mmol). Then the mixture was stirred in 30 ∼ 40 °Cunder 30 PSI CO for 48 h. After removing MeOH under vacuum and filtration, 4-carbamoyl-1-chloro-5H-pyrido[4,3-b]indole-8-carboxylate was obtained. ¹HNMR(400MHz, DMSO-*d*₆)δ 12.52 (s, 1 H), 8.99 (s, 1 H), 8.85 (s, 1 H), 8.42 (br, 1 H), 8.17 (d, *J* = 8.4 Hz, 1 H), 7.92 (d, *J* = 8.4 Hz, 1 H), 7.85 (br, 1 H), 3.92 (s, 3 H) ppm.

### Intermediate 6

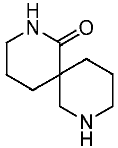

### 2,8-diazaspiro[5.5]undecan-1-one

2,8-Diazaspiro[5.5]undecan-1-one was prepared using the procedure reported in PCT International Publication No. WO2009/100872.

### Intermediate 7

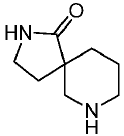

### 2,7 -diazaspiro[4.5]decan-1-one

2,7-Diazaspiro[4.5]decan-1-one was prepared using the procedure reported in U.S. Patent Application Publication No. US20090291946.

### Intermediate 8

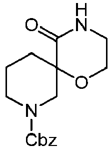

### Benzyl 5-oxo-1-oxa-4,8-diazaspiro[5.5]undecane-8-carboxylate

### Step 1: benzyl 1,4-dioxa-7-azaspiro[4.5]decane-7-carboxylate

To a solution of benzyl 3-oxopiperidine-1-carboxylate (28 g, 0.12 mol) and ethane-1,2-diol (16.8 mL, 0.3 mol) in toluene (300 mL) was added 4-methylbenzenesulfonic acid (2.3 g, 0.012 mol) in a flask equipped with a Dean-Stark trap. Then the reaction was heated to reflux overnight. After cooling to room temperature the mixture was washed with water (200 mL),satd.NaHCO₃ (aq) (100 mL), water (100 mL) and brine (100 mL), and dried over Na₂SO₄. The solution was concentrated *in vacuo* to obtain a residue, which was purified by silica gel chromatography (PE:EtOAc= 3 :1) to provide benzyl 1,4-dioxa-7-azaspiro[4.5]decane-7-carboxylate. ¹H NMR (400 MHz, DMSO-*d*₆) = 7.44 - 7.27 (m, 5H), 5.08 (s, 2H), 3.95 - 3.79 (m, 4H), 3.43 - 3.29 (m, 4H), 1.73 - 1.65 (m, 2H), 1.64 - 1.55 (m, 2H) ppm.

### Step 2 : benzyl 3-cyano-3-(2-hydroxyethoxy)piperidine-1-carboxylate

To a solution of benzyl 1,4-dioxa-7-azaspiro[4.5]decane-7-carboxylate (18.9 g, 0.068 mol) in DCM (400 mL) at 0 °C was added TMSCN (16.4 mL, 0.130 mol) following by BF₃.OEt₂ (11.2 mL, 0.089 mol). The mixture was stirred at -7 ∼ 0 °C for 1 hour. The reaction was quenched with adding water (200 mL). The mixture was parted and the organic layer was dried over Na₂SO₄, and evaporated under vacuum to obtain a residue. The residue was purified by silica gel chromatography (PE:EtOAc= 3 : 1∼ 0 : 1) to yield benzyl 3-cyano-3-(2-hydroxyethoxy)piperidine-1-carboxylate.¹H NMR (400 MHz, DMSO-*d*₆) = 7.35 (m, 5H), 5.09 (br. s., 2H), 4.80 - 4.69 (m, 1H), 4.04 -3.98 (m, 1H), 3.69 - 3.54 (m, 3H), 3.53 - 3.44 (m, 2H), 3.44 - 3.35 (m, 1H), 3.27 - 3.05 (m, 1H), 2.26 - 2.15 (m, 1H), 1.88 - 1.78 (m, 1H), 1.76 - 1.65 (m, 1H), 1.57 - 1.41 (m, 1H) ppm.

### Step 3: benzyl 3-(2-azidoethoxy)-3-cyanopiperidine-1-carboxylate

To a solution of benzyl 3-cyano-3-(2-hydroxyethoxy)piperidine-1-carboxylate (15.2g, 0.05 mol) and Et₃N (14 mL, 0.1 mol) in dry DCM (150 mL) was added MsCl (5.84 mL, 0.075 mol) dropwise at 0 °C. The reaction was stirred at 0 °C for 125 min. The reaction was quenched with adding water (100 mL). The organic layer was washed with water, brine, dried over Na₂SO₄, concentrated *in vacuo* to give a residue. To a solution of the residue in 150 mL of DMF, NaN₃ (4.88 g, 0.075 mol) was added. Then the mixture was heated to 70 ~90 °C for 2.5 h. The reaction mixture was cooled to room temperature, and partitioned between EtOAc (250 mL) and water (500 mL). The organic layer was washed with water (250 mLx3) and brine(250 mL), dried over Na₂SO₄, filtered, concentrated to give crude benzyl 3-(2-azidoethoxy)-3-cyanopiperidine-1-carboxylate. ¹H NMR (400 MHz, DMSO-*d*₆) δ 7.37 (m, 5H), 5.11 (s, 2H), 4.00 - 3.71 (m, 3H), 3.69 - 3.57 (m, 1H), 3.56 - 3.46 (m, 1H), 3.45 - 3.35 (m, 3H), 2.29 - 2.17 (m, 1H), 1.97 - 1.88 (m, 1H), 1.81 - 1.66 (m, 1H), 1.61 - 1.45 (m, 1H) ppm.

### Step 4:benzyl 3-(2-aminoethoxy)-3-cyanopiperidine-1-carboxylate

To a solution of benzyl 3-(2-azidoethoxy)-3-cyanopiperidine-1-carboxylate (11.1g, 0.033 mol) in THF/H₂O (V:V= 1 : 1)(300 mL) was added PPh₃ (9.7 g, 0.037 mmol). The reaction was stirred at 19 ∼ 25 °C for 75 min. The solution was concentrated *in vacuo* and extracted with DCM 150 mL.The organic layers were dried over Na₂SO₄, filtered, evaporated to provide crude product benzyl 3-(2-aminoethoxy)-3-cyanopiperidine-1-carboxylate. MS: 303.9(M+H).

### Step 5: 3-(2-aminoethoxy)-1-((benzyloxy)carbonyl)piperidine-3-carboxylic acid

Benzyl 3-(2-aminoethoxy)-3-cyanopiperidine-1-carboxylate was dissolvedin EtOH/H₂O (V:V= 4 : 1) (150 mL). To the solution was added KOH (1.88 g, 0.165 mol), then it was heated to 50 ∼ 60 °C overnight. The reaction was quenched by addition of water (1000 mL), washed with CH₂Cl₂(500 mL × 2), neutralized with 2N HCl(aq.) to pH=7∼8, and concentrated *in vacuo* to provide crude product 3-(2-aminoethoxy)-1-((benzyloxy)carbonyl)piperidine-3-carboxylic acid, which was used in the next step directly. ¹H NMR (400 MHz, DMSO-*d*₆) δ 7.50 - 7.19 (m, 5H), 5.06 (s, 2H), 4.15 - 3.79 (m, 3H), 3.29 - 2.69 (m, 7H), 1.76 (s, 5H) ppm. MS: 322.9[M+H]⁺.

### Step 6: benzyl 5-oxo-1-oxa-4,8-diazaspiror[5.5]undecane-8-carboxylate

To a solution of crude 3-(2-aminoethoxy)-1-((benzyloxy)carbonyl)piperidine-3-carboxylic acid (16.5 g, 0.05 mol) in DMF (160 mL) was added CDI (12.5 g, 0.075 mol).And then the reaction was stirred at 16 ~ 25 °C for 1.5 h.Then the mixture was purified by Prep HPLC to yield benzyl 5-oxo-1-oxa-4,8-diazaspiro[5.5]undecane-8-carboxylate.¹H NMR (400 MHz, CDC1₃) δ 7.35 (m, 5H), 6.42 (d, J=15.8 Hz, 1H), 5.24 - 5.05 (m, 2H), 4.58 - 4.10 (m, 2H), 4.06 - 3.57 (m, 2H), 3.57 - 3.39 (m, 1H), 3.37 - 3.04 (m, 2H), 2.95 - 2.72 (m, 1H), 2.16 - 2.02 (m, 1H), 2.00 - 1.73 (m, 2H), 1.51 (br. s., 1H) ppm.

### Intermediate 9

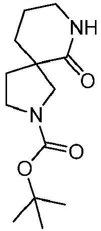

### 7-(4-fluorophenyl)-2,7-diazaspiror[4.5]decan-6-one

### Step 1: 1-tert-bgtyl 3-methyl 3-(2-cyanoethyl)pyrrolidine-1,3-dicarboxylate

To a solution of 1-*tert*-butyl 3-methyl pyrrolidine-1,3-dicarboxylate (500 mg, 2.19 mmol) in THF (6 mL) was added dropwise LDA (2.19 mL, 4.38 mmol, 2M) in THF under N₂ protection at -70°C for 1h. Then 3-bromopropionitrile (323.17 mg, 2.41 mmol) was added dropwise at -70 °C and the temperature was raised to 20°C for 10h. The reaction was quenched with NH₄Cl (aq) at 0 °C and then extracted with EtOAc. The organic phase was washed with brine, dried over Na₂SO₄ and concentrated *in vacuo.* The residue was purified by silica gel column chromatography to yield the product 1-*tert*-butyl 3-methyl 3-(2-cyanoethyl)pyrrolidine-1,3-dicarboxylate.¹H NMR (400 MHz, CDC1₃) = 3.82 - 3.73 (m, 1H), 3.70 (s, 3H), 3.46 - 3.29 (m, 2H), 3.17 (t, J=12.3 Hz, 1H), 2.31 (t, J=7.4 Hz, 2H), 2.11 - 1.94 (m, 3H), 1.78 (dd, J=5.0, 12.5 Hz, 1H), 1.40 (s, 9H) ppm.

### Step 2: tert-butyl 6-oxo-2,7-diazaspiro[4.5]decane-2-carboxylate

To the solution of 1-*tert*-butyl 3-methyl 3-(2-cyanoethyl)pyrrolidine-1,3-dicarboxylate (120 mg, 0.425 mmol) in MeOH (3 mL) was added satd. NH₃(aq.) (3 mL),followed by Raney Ni (120 mg). Then the mixture was stirred under H₂ (50psi) at 25 °C for 24h. The reaction mixture was filtered and the filtrate was partitioned between EtOAc and H₂O. The organic phase was washed with brine, dried over Na₂SO₄ and concentrated *in vacuo.* The residue was purified by silica gel column chromatography to provide the product *tert*-butyl 6-oxo-2,7-diazaspiro[4.5]decane-2-carboxylate. ¹H NMR (300MHz, CD₃OD) δ 4.90 (s, 2H), 3.80 - 3.68 (m, 3H), 2.42 - 2.25 (m, 1H), 1.91 - 1.82 (m, 6H), 1.52 - 1.32 (m, 9H) ppm.

### Intermediate 10

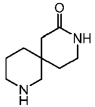

### 2,9-diazaspiro[5.5]undecan-8-one

### Step 1: 1-((benzyloxy)carbonyl)-3-(cyanomethyl)piperidine-3-carboxylic acid

To a solution of 1-benzyl 3-methyl 3-(cyanomethyl)piperidine-1,3-dicarboxylate (10 g, 30 mmol) in MeOH (100 mL) was added LiOH (100 mL, 0.1 mol, 1M) slowly at 0 °C, and the mixture was stirred at 25 °C for 13h. ThepH of the reaction mixture was adjusted to 1 with hydrochloric acid (1 M). Then the mixture was diluted with water (500 mL) and DCM (1 L). The organic phase was separated and washed with brine (100 mL), dried over Na₂SO₄ and concentrated *in vacuo* to afford crude 1-((benzyloxy)carbonyl)-3-(cyanomethyl)piperidine-3-carboxylic acid. ¹H NMR (400 MHz, DMSO-*d*₆) δ 13.15 (br. s., 1H), 7.46 - 7.23 (m, 5H), 5.19 - 4.95 (m, 2H), 3.97 - 3.61 (m, 1H), 3.46 - 3.33 (m, 3H), 2.97 - 2.64 (m, 2H), 2.05 - 1.87 (m, 1H), 1.72 - 1.42 (m, 3H) ppm.

### Step 2: benzyl 3-(cyanomethyl)-3-(2-diazoacetyl)piperidine-1-carboxylate

The solution of 1-((benzyloxy)carbonyl)-3-(cyanomethyl)piperidine-3-carboxylic acid (3.02 g, 10 mmol) inCH₂Cl₂ (50 mL), (COCl)₂ (2.54 g, 20 mmol) was added at 0 °C. DMF (2drops) was added to the mixture stirring at 25 °C for 4h. Then the reaction mixture was concentrated *in vacuo* to give crude acyl chloride . To the solution of this crude acyl chloride in THF / CH₃CN (20 mL / 20 mL), Et₃N (2.8 mL, 20 mmol) and TMSCH₂N₂ (8 mL, 16 mmol) was added dropwise at -10 °C ∼ 0 °C sequentially under N₂ protection. After stirring at room temperature ovemight,the mixture was poured into water (200 mL), and extracted with EtOAc (100 mL × 3). The combined organic phases were washed with brine (100 mL), dried over Na₂SO₄ and concentrated *in vacuo.* The residue was purified by silica gel column chromatography (EtOAc / PE = 0 ∼ 50%) to give benzyl 3-(cyanomethyl)-3-(2-diazoacetyl)piperidine-1-carboxy late.

### Step 3: benzyl 3-(cyanomethyl)-3-(2-ethoxy-2-oxoethyl)pipendine-1-carboxylate

To a solution of benzyl 3-(cyanomethyl)-3-(2-diazoacetyl)piperidine-1-carboxylate (1.6 g, 4.9 mmol) in EtOH (20 mL) was added AgOAc (818 mg, 4.9 mmol) slowly under N₂ protection, then the mixture was heated to reflux for 1h. The mixture was cooled to room temperature and filtered. The resulting solid was purified by silica gel column chromatography (THF / PE = 0 ∼ 50%) on silica-gel to give to benzyl 3-(cyanomethyl)-3-(2-ethoxy-2-oxoethyl)piperidine-1-carboxylate. ¹H NMR (400 MHz, CDCl₃) δ 7.41 - 7.29 (m, 5H), 5.13 (d, J=2.0 Hz, 2H), 4.28 - 4.00 (m, 2H), 3.71 - 3.23 (m, 4H), 2.82 - 2.57 (m, 2H), 2.55 - 2.35 (m, 2H), 1.73 - 1.55 (m, 4H), 1.30 - 1.21 (m, 3H) ppm.

### Step 4: benzyl 8-oxo-2,9-diazaspiro[5.5]undecane-2-carboxylate

A mixture of benzyl 3-(cyanomethyl)-3-(2-ethoxy-2-oxoethyl)piperidine-1-carboxylate (700 mg, 2 mmol), Ni (200 mg) in MeOH / ammonium hydroxide (20 mL / 5 mL) was stirred at 25 °C overnight under H₂ (50 psi). The mixture was filtered and concentrated *in vacuo.* The crude product was purified by silica gel chromatography (MeOH / DCM = 0 ∼ 5%) to afford benzyl 8-oxo-2,9-diazaspiro[5.5]undecane-2-carboxylate. ¹H NMR (400 MHz, CDCl₃) δ= 7.35 (br. s., 5H), 5.85 (br. s., 1H), 5.12 (br. s., 2H), 3.76 - 3.15 (m, 5H), 3.11 - 2.91 (m, 1H), 2.18 (br. s., 2H), 1.68 - 1.53 (m, 6H) ppm.

### Step 5: 2.9-diazaspiro[5.5]undecan-8-one

A mixture of benzyl 8-oxo-2,9-diazaspiro[5.5]undecane-2-carboxylate (100 mg, 0.33 mmol), Pd/C (100 mg) in MeOH (10 mL) was stirred at 25 °C for 2h under H₂ (20 psi). The mixture was filtered and concentrated *in vacuo* to afford 2,9-diazaspiro[5.5]undecan-8-one. MS: 169 [M+H]⁺.

### Intermediate 11

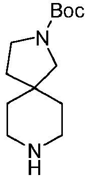

### tert-butyl 2,8-diazaspiro[4.5]decane-2-carboxylate

The title compound, *tert*-butyl 2,8-diazaspiro[4.5]decane-2-carboxylate was prepared according to the procedure disclosedin International Application Publication No. WO20080247964.

### Intermediate 12

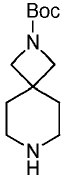

### tert-butyl 2,7-diazaspiro[3.5]nonane-2-carboxylate

The title compound *tert*-butyl 2,7-diazaspiro[3.5]nonane-2-carboxylate was prepared according to the procedure reported by International Application Publication No. WO 2007030061.

### Intermediate 13

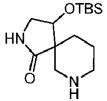

### 4-((tert-butyldimethylsilyl)on)-2,7-diazaspiro[4.5]decan-1-one

### Step 1: 2-(dibenzylamino)ethanol

To a solution of 2-aminoethanol (10 g, 163.71 mmol) in MeCN (200 mL) was added K₂CO₃ (45.2 g, 327.42 mmol) and tetrabutylammonium iodide (6.05 g, 16.37 mmol) sequentially. (Bromomethyl)benzene (56.08 g, 327.42 mmol) was added dropwise at 10°C. The suspension was stirred for 20 h. After the addition of CHCl₃ (25 mL), the solid was filtered and washed with CHCl₃ (2 x 20 mL). The resulting filtrate was carefully evaporated , and the crude product was purified by silica gel column chromatographyto give 2-(dibenzylamino)ethanol.¹H NMR (400 MHz, CDCl₃) = 7.38 - 7.21 (m, 10H), 3.63 (s, 4H), 3.59 (t, J=5.1 Hz, 2H), 2.67 (t, J=5.4 Hz, 2H), 2.58 (br. s., 1H) ppm.

### Step 2: 2-(dibenzylamino)acetaldehyde

To a solution of oxalyl chloride (6.95 g, 54.7 mmol) in dichloromethane (150 mL), a solution of dimethyl sulfoxide (9.26 g, 118.5 mmol) in dichloromethane (100 mL) was added dropwise at -70°C. After stirring for 30 min at -70°C, a solution of 2-(dibenzylamino)ethanol (11 g, 45.58 mmol) in dichloromethane (150 mL) was added to the reaction mixture dropwise. After stirring for another 45 min, triethylamine (19.2 mL, 136.7 mmol) was added dropwise and stirred at -70°C for 1.5h.Saturated sodium bicarbonate (200 mL) was added into the reaction mixture, which was extracted with dichloromethane (200 mL). The organic extracts were washed with brine (50 mL), dried over MgSO₄ and concentrated *in vacuoto* afford the 2-(dibenzylamino)acetaldehyde, which was immediately used in the next step.¹H NMR (400 MHz, CDC1₃) δ 9.54 (t, J=1.5 Hz, 1H), 7.42 - 7.24 (m, 10H), 3.71 (s, 4H), 3.20 (d, J=1.5 Hz, 2H) ppm.

### Step 3: 1-tert-butyl 3-methyl 3-(2-(dibenzylamino)-1-hydroxyethyl)piperidine-1,3-dicarboxylate

To a solution of 1-*tert*-butyl 3-methyl piperidine-1,3-dicarboxylate (13.4 g, 55.16 mmol) in anhydrous THF (200 mL) was added LiHMDS (55.2 mL, 55.2 mmol) at -78°C. The mixture was stirred at -78°C for 40 mins. Then a solution of 2-(dibenzylamino)acetaldehyde (11 g, 45.97 mmol) in anhydrous THF (100 mL) was added at -78°C dropwise and the mixture was stirred at this temperature for 2 h. The mixture was quenched by satd.NH₄Cl (30 mL) slowly at -78°C and extracted with ethyl acetate (200 mL x 3).The combined organic layers were washed with brine (20 mL), dried over sodium sulfate, filtered and concentrated to afford the crude product, which was purified on silica gel column chromatography to give 1-*tert*-butyl 3-methyl 3-(2-(dibenzylamino)-1-hydroxyethyl)piperidine-1,3-dicarboxylate.¹H NMR (400 MHz, CDCl₃) δ 7.37 - 7.23 (m, 11H), 4.31 - 3.99 (m, 1H), 3.94 - 3.65 (m, 4H), 3.60 - 3.31 (m, 5H), 3.17 - 2.94 (m, 1H), 2.85 (br. s., 1H), 2.77 - 2.54 (m, 1H), 2.49 - 2.22 (m, 1H), 2.01 - 1.76 (m, 1H), 1.67 - 1.48 (m, 2H), 1.46 - 1.39 (m, 9H) ppm.

### Step 4:tert-butyl 4-hydroxy-1-oxo-2,7-diazaspiro[4.5]decane-7-carboxylate

To a solution of 1-*tert*-butyl 3-methyl 3-(2-(dibenzylamino)-1-hydroxyethyl)piperidine-1,3-dicarboxylate (2.4 g, 4.95 mmol) in methanol (100 mL) was added Pd(OH)₂ (250 mg). The resulting solution was stirred at 50°C under 55 psi hydrogen overnight. The mixture was filtered, the filtrate was concentrated under reduced pressure to afford *tert*-butyl 4-hydroxy-1-oxo-2,7-diazaspiro[4.5]decane-7-carboxylate. ¹H NMR (400 MHz, CD₃OD) δ 4.17 (d, J=3.3 Hz, 1H), 4.06 - 3.95 (m, 1H), 3.72 (br. s., 1.5H), 3.61 (dd, J=5.0, 11.0 Hz, 0.5H), 3.28 - 3.02 (m, 2H), 3.00 - 2.73 (m, 1H), 1.95 - 1.53 (m, 4H), 1.51 - 1.45 (m, 9H) ppm.

### Step 5:tert-butyl 4-((tert-butyldimethylsilyl)oxy)-1-oxo-2,7-diazaspiro[4.5]decane-7-carboxylate

To a solution of *tert*-butyl 4-hydroxy-1-oxo-2,7-diazaspiro[4.5]decane-7-carboxylate (300 mg, 1.11 mmol) in anhydrous DMF (5 mL) was added TBSCl (200 mg, 1.33 mmol) and 4H-imidazole (227 mg, 3.33 mmol). The mixture was stirred at 25°C for 16 h. The mixture was quenched by the addition of water (30 mL), then extracted with EtOAc (30 mL x 3).The combined organic layers were washed with brine (20mL x 5), dried over sodium sulfate, filtered and concentrated to afford the crude product, which was purified on silica gel column chromatography to give *tert*-butyl 4-((*tert*-butyldimethylsilyl)oxy)-1-oxo-2,7-diazaspiro[4.5]decane-7-carboxylate.¹H NMR (400 MHz, CDC1₃) δ 4.37 (t, J=5.5 Hz, 1H), 4.01 - 3.93 (m, 1H), 3.91 - 3.67 (m, 3H), 3.52 - 3.39 (m, 1.5H), 3.21 - 2.99 (m, 0.5H), 2.47 - 2.27 (m, 1H), 2.06 - 1.96 (m, 1H), 1.88 - 1.76 (m, 2H), 1.71 (d, J=2.8 Hz, 9H), 1.20 - 1.14 (m, 9H), 0.38 - 0.32 (m, 6H) ppm.

### Step 6: 4-((tert-butyldimethylsilyl)oxy)-2.7-diazaspiro[4.5]decan-1-one

To a solution of *tert*-butyl 4-((*tert*-butyldimethylsilyl)oxy)-1-oxo-2,7-diazaspiro[4.5]decane-7-carboxylate (570 mg, 1.48 mmol) in CH₂Cl₂ (20 mL) was added TFA (4 mL) dropwise at 0°C. The mixture was stirred at 20°C for 1.5 h. The mixture was diluted with CH₂Cl₂ (50 mL) and concentrated to give 4-((*tert*-butyldimethylsilyl)oxy)-2,7-diazaspiro[4.5]decan-1-one. MS (ESI)MS:285.2[M+H]⁺.

### Intermediate 14

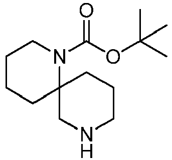

### tert-butyl 1,8-diazaspiro[5.5]undecane-1-carboxylate

The title compound*tert*-butyl 1,8-diazaspiro[5.5]undecane-1-carboxylate was prepared in the procedure reported by Synthetic Communication (2007), 37(21), p 3793-3799.

### Example 1

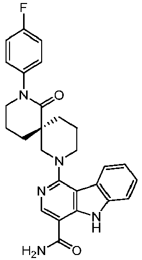

### (R)-1-(8-(4-fluorophepyl)-7-oxo-2.8-diazaspiro[5.5]undecan-2-yl)-5H-pyrido[4.3]indole-4-carboxamide

### Step 1: (R)-benzyl 7-oxo-2,8-diazaspiro[5.5]undecane-2-carboxylate and (S)-benzyl 7-oxo-2,8-diazaspiro[5.5]undecane-2-carboxylate

A mixture of 1-benzyl 3-ethyl 3-(2-cyanoethyl)piperidine-1,3-dicarboxylate (4g, 11.6 mmol), Raney Nickel (400 mg) in MeOH (40 mL) was stirred at room temperature for 10 h under H₂ (55 psi). The mixture was filtered and concentrated *in vacuo.* The crude product was purified by chromatography to afford compound benzyl 7-oxo-2,8-diazaspiro[5.5]undecane-2-carboxylate. The mixture of the two stereoisomers was purified by chiral SFC (Column : AD 250mm^{∗}50mm,10um; Mobile phase: A: Supercritical CO₂, B: MeOH (0.05%NH₃H₂O), A:B =60:40 at 240 mL/min; Column Temp: 38°C; Nozzle Pressure: 100Bar ; Nozzle Temp: 60°C; Evaporator Temp: 20°C; Trimmer Temp: 25°C; Wavelength: 220nm) to afford (R)-benzyl 7-oxo-2,8-diazaspiro[5.5]undecane-2-carboxylate (fastereluting): ¹H NMR (400 MHz, CD₃OD) δ 7.36 (d, J=4.3 Hz, 5H), 5.24 - 4.97 (m, 2H), 4.14 - 3.88 (m, 2H), 3.27 - 3.10 (m, 3H), 2.99 - 2.79 (m, 1H), 2.14 - 2.03 (m, 1H), 1.80 (br. s., 2H), 1.69 - 1.49 (m, 5H) ppm and (S)-benzyl 7-oxo-2,8-diazaspiro[5.5]undecane-2-carboxylate (slower eluting): ¹H NMR (400 MHz, CD₃OD) δ 7.43 - 7.27 (m, 1H), 5.25 - 4.98 (m, 1H), 4.18 - 3.87 (m, 1H), 3.29 - 3.11 (m, 1H), 2.99 - 2.82 (m, 1H), 2.16 - 2.05 (m, 1H), 1.90 - 1.70 (m, 2H), 1.70 - 1.52 (m, 5H) ppm.

### Step 2: (R)-benzyl 8-(4- fluorophenyl)-7-oxo-2,8-diazaspiro[5.5]undecane-2-carboxylate

To a mixture of (R)-benzyl 7-oxo-2,8-diazaspiro[5.5]undecane-2-carboxylate (300 mg, 1 mmol), 1-fluoro-4-iodobenzene (444 mg, 2 mmol) and K₃PO₄ (637 g, 3 mmol) in DMF (7 mL) was added CuI (286 mg, 1.5mmol) and N,N-dimethylethane-1,2-diamine (176 mg, 2 mmol) under N₂. The mixture was heated at 145°C for 8 h. The mixture was cooled to room temperature, then partitioned between water (10 mL) and EtOAc (30 mL). The organic phase was concentrated *in vacuo* and purified by chromatography to afford (R)-benzyl 8-(4-fluorophenyl)-7-oxo-2,8-diazaspiro[5.5]undecane-2-carboxylate. ¹H NMR (400 MHz, CD₃OD) δ 7.37 (d, J=4.8 Hz, 5H), 7.21 (d, J=9.3 Hz, 2H), 7.16 - 7.06 (m, 2H), 5.25 - 5.01 (m, 2H), 4.11 - 4.01 (m, 2H), 3.69 - 3.47 (m, 2H), 3.30 - 3.24 (m, 1H), 3.09 - 2.85 (m, 1H), 2.20 - 1.83 (m, 4H), 1.75 (d, J=13.3 Hz, 2H), 1.68 - 1.59 (m, 2H) ppm.

### Step 3: (R)-2-(4-fluorophepyl)-2,8-diazaspiro[5.5]undecan-1-one

A mixture of (R)-benzyl 8-(4-fluorophenyl)-7-oxo-2,8-diazaspiro[5.5]undecane-2-carboxylate (200 mg, 0.5 mmol), Pd/C (100 mg) in MeOH (4 mL) was stirred at room temperature overnight under H₂ (40 psi). The mixture was filtered and the filtrate was concentrated under vacuum to afford (S)-2-(4-fluorophenyl)-2,8-diazaspiro[5.5]undecan-1-one. ¹H NMR (400 MHz, DMSO-*d*₆) δ= 7.30 - 7.10 (m, 4H), 3.61 - 3.39 (m, 3H), 2.85 - 2.62 (m, 3H), 2.14 - 1.92 (m, 3H), 1.88 - 1.78 (m, 2H), 1.73 - 1.63 (m, 1H), 1.53 (d, J=13.3 Hz, 1H), 1.47 - 1.30 (m, 2H) ppm.

### Step 4:(R)-1-(8-(4-fluorophenyl)-7-oxo-2,8-diazaspiro[5.5]undecan-2-yl)-5H-pyrido[4,3-b]lindole-4-carboxamide

To a solution of (S)-2-(4-fluorophenyl)-2,8-diazaspiro[5.5]undecan-1-one (120 mg, 0.45 mmol) in NMP (3 mL) was added 1-chloro-5H-pyrido[4,3-b]indole-4-carboxamide (Intermediate 1, 123 mg, 0.5mmol) and DIPEA (174 mg, 1.35 mmol), and the mixture was heated under microwave irradiation for 1.5 h at 150 °C.The mixture was purified with prep-HPLC to give (R)-1-(8-(4-fluorophenyl)-7-oxo-2,8-diazaspiro[5.5]undecan-2-yl)-5H-pyrido[4,3-b]indole-4-carboxamide. MS: 472.3 [M+H]⁺. ¹H NMR (400 MHz, CD₃OD) δ 8.45 (s, 1H), 8.03 (d, J=8.0 Hz, 1H), 7.81 (d, J=8.0 Hz, 1H), 7.62 (t, J=7.3 Hz, 1H), 7.55 - 7.49 (m, 1H), 7.11 - 7.06 (m, 4H), 4.28 (d, J=12.5 Hz, 1H), 4.03 - 3.93 (m, 1H), 3.87 (d, J=12.5 Hz, 1H), 3.80 (d, J=4.8 Hz, 1H), 3.63 - 3.53 (m, 2H), 2.43 (ddd, J=4.8, 7.7, 12.9 Hz, 1H), 2.25 - 2.07 (m, 3H), 2.07 - 1.98 (m, 3H), 1.94 - 1.84 (m, 1H) ppm.

### Example 2

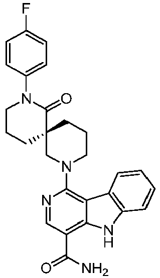

### (S)-1-(8-(4-fluorophepyl)-7-oxo-2.8-diazaspiro[5.5]undecan-2-yl)-5H-pyrido[4,3-b]indole-4-carboxamide

Starting with (S)-benzyl 7-oxo-2,8-diazaspiro[5.5]undecane-2-carboxylate obtained from the step 1 of Example 1, (S)-1-(8-(4-fluorophenyl)-7-oxo-2,8-diazaspiro[5.5]undecan-2-yl)-5H-pyrido[4,3-b]indole-4-carboxamide was obtained followinga similar procedure as describedinExample 1.MS: 472.3 [M+H]⁺. ¹H NMR (400 MHz, CD₃OD) δ 8.45 (s, 1H), 8.03 (d, J=8.0 Hz, 1H), 7.81 (d, J=8.0 Hz, 1H), 7.62 (t, J=7.3 Hz, 1H), 7.55 - 7.49 (m, 1H), 7.11 - 7.06 (m, 4H), 4.28 (d, J=12.5 Hz, 1H), 4.03 - 3.93 (m, 1H), 3.87 (d, J=12.5 Hz, 1H), 3.80 (d, J=4.8 Hz, 1H), 3.63 - 3.53 (m, 2H), 2.43 (ddd, J=4.8, 7.7, 12.9 Hz, 1H), 2.25 - 2.07 (m, 3H), 2.07 - 1.98 (m, 3H), 1.94 - 1.84 (m, 1H) ppm.

### Example 3

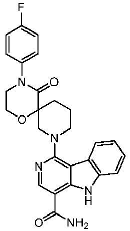

### 1-(4-(4-fluorophnyl)-5-oxo-1-oxa-4.8-diazaspiro[5.5]undecan-8-yl)-5H-arido[4,3-b]indole-4-carboxamide

### Step 1: 4-(4-fluorophenyl)-1-oxa-4,8-diazaspiro[5.5]undecan-5-one

A mixture of benzyl 4-(4-fluorophenyl)-5-oxo-1-oxa-4,8-diazaspiro[5.5]undecane-8-carboxylate (220 mg, 0.55 mmol) and Pd/C (50 mg) in MeOH (5 mL) was stirred at room temperature for 3 h under H₂. The mixture was filtered and the filtrate was concentrated *in vacuo* to afford 4-(4-fluorophenyl)-1-oxa-4,8-diazaspiro[5.5]undecan-5-one. MS: 264.9[M+H]⁺.

### Step 2: 1-(4-(4-fluorophenyl)-5-oxo-1-oxa-4,8-diazaspiro[5.5]undecan-8-yl)-5H-pyrido[4,3-b]indole-4-carboxamide

To a solution of 4-(4-fluorophenyl)-1-oxa-4,8-diazaspiro[5.5]undecan-5-one (120 mg, 0.45 mmol) in NMP (3 mL) was added 1-chloro-5H-pyrido[4,3-b]indole-4-carboxamide (123 mg, 0.5mmol) and DIEA (174 mg, 1.35 mmol), and the mixture was heated under microwave irradiation for 1.5 h at 150 °C.The mixture was purified with prep-HPLC to give 1-(4-(4-fluorophenyl)-5-oxo-1-oxa-4,8-diazaspiro[5.5]undecan-8-yl)-5H-pyrido[4,3-b]indole-4-carboxamide. ¹H NMR (400 MHz, CD₃OD) δ 8.51 (s, 1H), 8.03 (d, J=8.0 Hz, 1H), 7.82 (d, J=8.0 Hz, 1H), 7.62 (t, J=7.7 Hz, 1H), 7.54 - 7.44 (m, 1H), 7.31 - 7.23 (m, 2H), 7.14 - 7.06 (m, 2H), 4.40 (d, J=13.3 Hz, 1H), 4.27 - 4.14 (m, 2H), 4.00 - 3.90 (m, 1H), 3.80 - 3.66 (m, 3H), 3.64 - 3.52 (m, 1H), 2.54 - 2.39 (m, 1H), 2.38 - 2.22 (m, 2H), 1.94 - 1.79 (m, 1H) ppm.

The following examples in Table 1 were prepared using similar procedures to those described in Examples 1-3, starting with different spiro piperidine or pyrrolidine compounds and the azacarbazole chloride intermediates for C-N bond formation.

**Table 1:**

| **Ex. No.** | **Structure** | **Name** | **Exact Mass [M+H]+** | **Retention time (min)** |
|---|---|---|---|---|
| 4 | 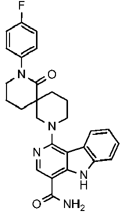 | 1-[8-(4-fluorophenyl)-7-oxo-2,8-diazaspiro[5.5]undec-2-yl]-5H-pyrido[4,3-b]indole-4-carboxamide | Calc'd 472.2, found 472.3 | 1.828 |
| 5 | 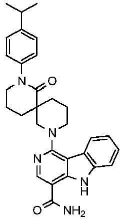 | 1-{8-[4-(1-methylethyl)phenyl]-7-oxo-2,8-diazaspiro[5.5]undec-2-yl}-5H-pyrido[4,3-b]indole-4-carboxamide | Calc'd 496.3, found 496.3 | 2.099 |
| 6 | 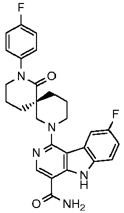 | 8-fluoro-1-[(6R)-8-(4-fluorophenyl)-7-oxo-2,8-diazaspiro[5.5]undec-2-yl]-5H-pyrido[4,3-b]indole-4-carboxamide | Calc'd 490.2, found 490.3 | 1.596 |
| 7 | 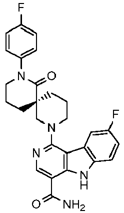 | 8-fluoro-1-[(6S)-8-(4-fluorophenyl)-7-oxo-2,8-diazaspiro[5.5]undec-2-yl]-5H-pyrido[4,3-b]indole-4-carboxamide | Calc'd 490.2, found 490.3 | 1.576 |
| 8 | 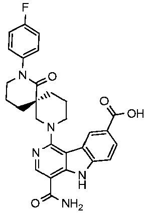 | 4-carbamoyl-1-[(6R)-8-(4-fluorophenyl)-7-oxo-2,8-diazaspiro[5.5]undec-2-yl]-5H-pyrido[4,3-b]indole-8-carboxylic acid | Calc'd 516.2, found 516.3 | 2.077 |
| 9 | 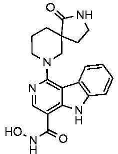 | N-hydroxy-1-(1-oxo-2,7-diazaspiro[4.5]dec-7-yl)-5H-pyrido[4,3-b]indole-4-carboxamide | Calc'd 380.2, found 380.3 | 2.123 |
| 10 | 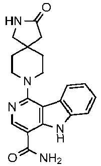 | 1-(3-oxo-2,8-diazaspiro[4.5]dec-8-yl)-5H-pyrido[4,3-b]indole-4-carboxamide | Calc'd 364.2, found 364.3 | 1.781 |
| 11 | 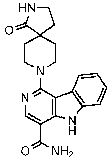 | 1-(1-oxo-2,8-diazaspiro[4.5]dec-8-yl)-5H-pyrido[4,3-b]indole-4-carboxamide | Calc'd 364.2, found 364.3 | 1.812 |
| 12 | 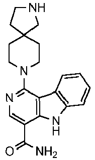 | 1-(2,8-diazaspiro[4.5]dec-8-yl)-5H-pyrido[4,3-b]indole-4-carboxamide | Calc'd 350.2, found 350.3 | 2.058 |
| 13 | 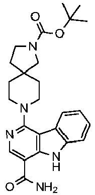 | *tert*-butyl 8-(4-carbamoyl-5H-pyrido[4,3-b]indol-1-yl)-2,8-diazaspiro[4.5]decane-2-carboxylate | Calc'd 450.3, found 450.4 | 2.373 |
| 14 | 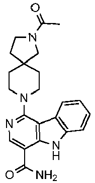 | 1-(2-acetyl-2,8-diazaspiro[4.5]dec-8-yl)-5H-pyrido[4,3-b]indole-4-carboxamide | Calc'd 392.2, found 392.3 | 2.244 |
| 15 | 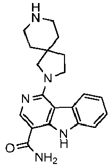 | 1-(2,8-diazaspiro[4.5]dec-2-yl)-5H-pyrido[4,3-b]indole-4-carboxamide | Calc'd 350.2, found 350.3 | 1.978 |
| 16 | 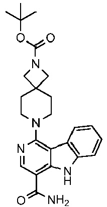 | *tert*-butyl 7-(4-carbamoyl-5H-pyrido[4,3-b]indol-1-yl)-2,7-diazaspiro[3.5]nonane-2-carboxylate | Calc'd 436.2, found 436.4 | 2.346 |
| 17 | 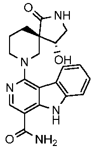 | 1-[(4S,5R)-4-hydroxy-1-oxo-2,7-diazaspiro[4.5]dec-7-yl]-5H-pyrido[4,3-b]indole-4-carboxamide | Calc'd 380.2, found 380.3 | 2.113 |
| 18 | 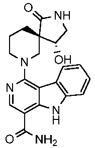 | 1-[(4R,SS)-4-hydroxy-1-oxo-2,7-diazaspiro[4.5]dec-7-yl]-5H-pyrido[4,3-b]indole-4-carboxamide | Calc'd 380.2, found 380.3 | 2.122 |
| 19 | 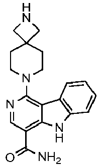 | 1-(2,7-diazaspiro[3.5]non-7-yl)-5H-pyrido[4,3-b]indole-4-carboxamide | Calc'd 336.2, found 336.3 | 2.941 |
| 20 | 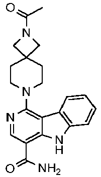 | 1-(2-acetyl-2,7-diazaspiro[3.5]non-7-yl)-5H-pyrido[4,3-b]indole-4-carboxamide | Calc'd 378.2, found 378.3 | 2.355 |
| 21 | 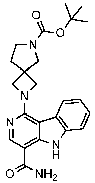 | *tert*-butyl 2-(4-carbamoyl-5H-pyrido[4,3-b]indol-1-yl)-2,6-diazaspiro[3.4]octane-6-carboxylate | Calc'd 422.2, found 422.4 | 2.535 |
| 22 | 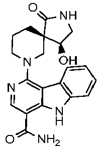 | 1-[(4S,5S)-4-hydroxy-1-oxo-2,7-diazaspiro[4.5]dec-7-yl]-5H-pyrido[4,3-b]indole-4-carboxamide | Calc'd 380.2, found 380.3 | 2.045 |
| 23 | 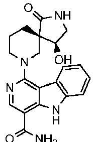 | 1-[(4S,5S)-4-hydroxy-1-oxo-2,7-diazaspiro[4.5]dec-7-yl]-5H-pyrido[4,3-b]indole-4-carboxamide | Calc'd 380.2, found 380.3 | 2.065 |
| 24 | 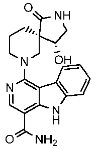 | 1-[(4R,5R)-4-hydroxy-1-oxo-2,7-diazaspiro[4.5]dec-7-yl]-5H-pyrido[4,3-b]indole-4-carboxamide | Calc'd 380.2, found 380.3 | 2.063 |
| 25 | 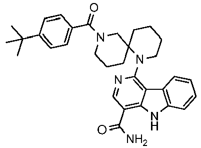 | 1-{8-[(4-*tert-*butylphenyl)carbonyl]-1,8-diazaspiro[5.5]undec-1-yl}-5H-pyrido[4,3-b]indole-4-carboxamide | Calc'd 524.3, found 524.5 | 2.305 |
| 26 | 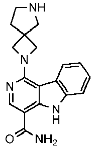 | 1-(2,6-diazaspiro[3.4]oct-2-yl)-5H-pyrido[4,3-b]indole-4-carboxamide | Calc'd 322.2, found 322.3 | 1.893 |
| 27 | 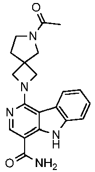 | 1-(6-acetyl-2,6-diazaspiro [3.4]oct-2-yl)-5H-pyrido[4,3-b]indole-4-carboxamide | Calc'd 364.2, found 364.3 | 2.141 |
| 28 | 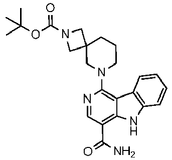 | *tert*-butyl 6-(4-carbamoyl-5H-pyrido[4,3-b]indol-1-yl)-2,6-diazaspiro[3.5]nonane-2-carboxylate | Calc'd 436.2, found 436.4 | 2.290 |
| 29 | 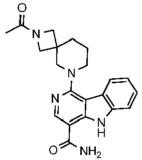 | 1-(2-acetyl-2,6-diazaspiro[3.5]non-6-yl)-5H-pyrido[4,3-b]indole-4-carboxamide | Calc'd 378.2, found 378.3 | 2.174 |
| 30 | 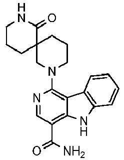 | 1-(7-oxo-2,8-diazaspiro[5.5]undec-2-yl)-5H-pyndo[4,3-b]indole-4-carboxamide | Calc'd 378.2, found 378.3 | 2.229 |
| 31 | 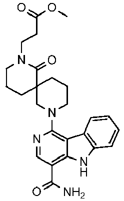 | methyl 3-[8-(4-carbamoyl-5H-pyrido[4,3-b]indol-1-yl)-1-oxo-2,8-diazaspiro[5.5]undec-2-yl]propanoate | Calc'd 464.2, found 464.4 | 2.373 |
| 32 | 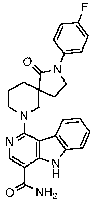 | 1-[2-(4-fluorophenyl)-1-oxo-2,7-diazaspiro[4.5]dec-7-yl]-5H-pyrido[4,3-b]indole-4-carboxamide | Calc'd 458.2, found 458.4 | 2.280 |
| 33 | 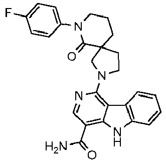 | 1-[7-(4-fluorophenyl)-6-oxo-2,7-diazaspiro[4.5]dec-2-yl]-5H-pyrido[4,3-b]indole-4-carboxamide | Calc'd 458.2, found 458.2 | 2.292 |
| 34 | 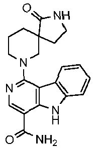 | 1-(1-oxo-2,7-diazaspiro[4.5]dec-7-yl)-5H-pyrido[4,3-b]indole-4-carboxamide | Calc'd 364.2, found 364.3 | 2.196 |
| 35 | 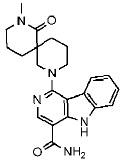 | 1-(8-methyl-7-oxo-2,8-diazaspiro[5.5]undec-2-yl)-5H-pyndo[4,3-blindole-4-carboxamide | Calc'd 392.2, found 392.4 | 2.300 |
| 36 | 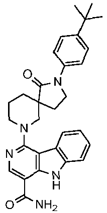 | 1-[2-(4-*tert*-butylphenyl)-1-oxo-2,7-diazaspiro[4.5]dec-7-yl]-5H-pyrido[4,3-b]indole-4-carboxamide | Calc'd 496.3, found 496.4 | 2.259 |
| 37 | 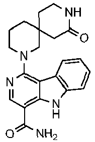 | 1-(8-oxo-2,9-diazaspiro[5.5]undec-2-yl)-5H-pyrido[4,3-blindole-4-carboxamide | Calc'd 378.2, found 378.3 | 2.131 |
| 38 | 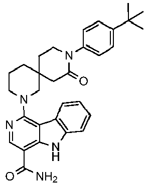 | 1-[9-(4-*tert*-butylphenyl)-8-oxo-2,9-diazaspiro [5.5] undec-2-yl]-5H-pyrido[4,3-b]indole-4-carboxamide | Calc'd 510.3, found 510.5 | 2.062 |
| 39 | 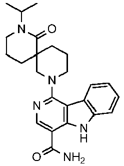 | 1-[8-(1-methylethyl)-7-oxo-2,8-diazaspiro[5.5]undec-2-yl]-5H-pyrido[4,3-b]indole-4-carboxamide | Calc'd 420.2, found 420.4 | 2.196 |
| 40 | 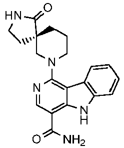 | 1-[(5R)-1-oxo-2,7-diazaspiro[4.5]dec-7-yl]-5H-pyrido[4,3-b]indole-4-carboxamide | Calc'd 364.2, found 364.3 | 2.142 |
| 41 | 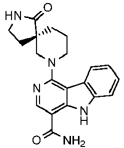 | 1-[(5S)-1-oxo-2,7-diazaspiro[4.5]dec-7-yl]-5H-pyrido[4,3-b]indole-4-carboxamide | Calc'd 364.2, found 364.3 | 2.143 |

### Example 42

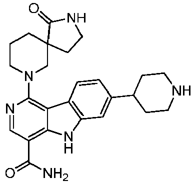

### 1-(1-oxo-2.7-diazaspiro[4.5]decan-7-yl)-7-(piperidin-4-yl)-5H-pyrido[4,3]indole-4-carboxamide

### Step 1: 7-bromo-1-(1-oxo-2,7-diazaspiror[4.5]decan-7-yl)-5H-pyrido[4,3-b]indole-4-carboxamide

To a solution of 7-bromo-1-chloro-5H-pyrido[4,3-b]indole-4-carboxamide (500 mg, 1.54 mmol) in NMP (5 mL) was added a solution of 2,7-diazaspiro[4.5]decan-1-one (360 mg, 2.31 mmol) and DIEA (900 mg, 6.93 mmol) in NMP (5 mL). The mixture was heated at 150°Cin a microwave oven for 1 h. Then the mixture was cooled to room temperature and poured into H₂O (100 mL). The mixture was filtered and the solid was purified by chromatography(MeOH / DCM = 0 ∼ 10%) to give 7-bromo-1-(1-oxo-2,7-diazaspiro[4.5]decan-7-yl)-5H-pyrido[4,3-b]indole-4-carboxamide. ¹H NMR (400 MHz, DMSO-*d₆*) δ 11.87 (s, 1H), 8.69 (s, 1H), 8.12 (br. s., 1H), 7.98 (s, 1H), 7.75 (d, *J*=8.5 Hz, 1H), 7.65 (s, 1H), 7.46 (d, *J*=8.5 Hz, 2H), 3.93 (d, *J*=12.5 Hz, 1H), 3.63 (d, *J*=12.0 Hz, 1H), 3.22 (d, *J*=12.5 Hz, 1H), 3.14 - 2.97 (m, 2H), 2.80 (t, *J*=12.0 Hz, 1H), 2.11 - 1.88 (m, 3H), 1.86 - 1.70 (m, 2H), 1.67 - 1.54 (m, 1H) ppm.

### Step 2: tert-butyl 4-(4-carbamoyl-1-(1-oxo-2,7-diazaspiro[4.5]decan-7-yl)-5H-pyrido[4,3-b]indol-7-yl)-5,6-dihydropyridine-1(2H)-carboxylate

To a solution of 7-bromo-1-(1-oxo-2,7-diazaspiro[4.5]decan-7-yl)-5H-pyrido[4,3-b]indole-4-carboxamide (100 mg, 0.23 mmol), *tert*-butyl 4-(4,4,5,5-tetramethyl-1,3,2-dioxaborolan-2-yl)-5,6-dihydropyridine-1(2H)-carboxylate (105 mg, 0.34 mmol) and Cs₂CO₃ (295 mg, 0.90 mmol) in DMF / H₂O (2 mL / 0.6 mL) was added Pd(dppf)Cl₂ (17 mg, 0.02 mmol) under a N₂ atmosphere. The mixture was stirred at 100 °Cfor 12h. After cooling, DCM (20 mL) and H₂O (10 mL) was added and the mixture was filtered. The organic layer was washed with brine (10 mL), dried over Na₂SO₄, concentrated *in vacuo.* The residue was purified by chromatography (MeOH / DCM = 0 ∼ 10%) to give *tert*-butyl 4-(4-carbamoyl-1-(1-oxo-2,7-diazaspiro[4.5]decan-7-yl)-5H-pyrido[4,3-b]indol-7-yl)-5,6-dihydropyridine-(2H)-carboxylate. MS: 545.4(M-18).

### Step 3:tert-butyl4-(4-carbamoyl-1-(1-oxo-2,7-diazaspiro[4.5]decan-7-yl)-5H-pyrido[4,3-b]indol-7-yl)piperidine-1-carboxylate

To a solution of *tert*-butyl 4-(4-carbamoyl-1-(1-oxo-2,7-diazaspiro[4.5]decan-7-yl)-5H-pyrido[4,3-b]indol-7-yl)-5,6-dihydropyridine-1(2H)-carboxylate (100 mg, 0.18 mmol) in MeOH (10 mL) was added Pd/C (60 mg). The mixture was stirred at room temperature under a H₂ atmosphere for 12 h. Then the mixture was filtered and the filtrate concentrated under reduced pressure. The residue was purified by prep-HPLC to afford *tert*-butyl 4-(4-carbamoyl-1-(1-oxo-2,7-diazaspiro[4.5]decan-7-yl)-5H-pyrido[4,3-b]indol-7-yl)piperidine-1-carboxylate.¹H NMR (400 MHz, CD₃OD) δ 8.50 (s, 1H), 7.91 (d, *J*=8.5 Hz, 1H), 7.71 (s, 1H), 7.42 (d, *J*=8.5 Hz, 1H), 4.25 (d, *J*=13.1 Hz, 2H), 4.00 (d, *J*=13.6 Hz, 1H), 3.90 - 3.81 (m, 1H), 3.80 - 3.69 (m, 1H), 3.64 - 3.49 (m, 1H), 3.39 - 3.33 (m, 1H), 3.28 - 3.20 (m, 1H), 3.03 - 2.79 (m, 3H), 2.22 - 2.05 (m, 5H), 1.97 - 1.83 (m, 3H), 1.78 - 1.61 (m, 2H), 1.49 (s, 9H) ppm. MS: 547.5[M+H]⁺.

### Step 4: 1-(1-oxo-2,7-diazaspiro[4.5]decan-7-yl)-7-(piperidin-4-yl)-5H-pyrido[4,3-blindole-4-carboxamide

To a solution of *tert*-butyl 4-(4-carbamoyl-1-(1-oxo-2,7-diazaspiro[4.5]decan-7-yl)-5H-pyrido[4,3-b]indol-7-yl)piperidine-1-carboxylate (50 mg, 0.09 mmol) was added HCl-dioxane (2 mL) and the resulting mixture was stirred at room temperature for 2 h. The mixture was concentrated under reduced pressure and the residue purified by prep-HPLC to afford 1-(1-oxo-2,7-diazaspiro[4.5]decan-7-yl)-7-(piperidin-4-yl)-5H-pyrido[4,3-b]indole-4-carboxamide.¹H NMR (400 MHz, CD₃OD) δ 8.56 (s, 1H), 7.98 (d, *J*=8.5 Hz, 1H), 7.78 (s, 1H), 7.47 (d, *J*=8.5 Hz, 1H), 4.03 (d, *J*=13.6 Hz, 1H), 3.90 - 3.75 (m, 2H), 3.58 (d, *J*=13.1 Hz, 3H), 3.41 - 3.35 (m, 1H), 3.30 - 3.14 (m, 4H), 2.25 - 2.03 (m, 9H), 1.95 - 1.85 (m, 1H) ppm.MS: 447.4[M+H]⁺.

The examples in Table 2 were prepared using similarprocedures as described in Example 42. The substitutions on the azacarbazole 7- and 8- positions were introduced by Suzuki coupling, with some further derivatization by acylation or alkylation of the piperidine nitrogen.

**Table 2**

| **Ex. No.** | **Structure** | **Name** | **Exact Mass [M+H]+** | **Retention time (min)** |
|---|---|---|---|---|
| 43 | 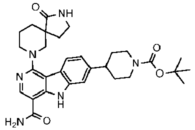 | *tert*-butyl 4-[4-carbamoyl-1-(1-oxo-2,7-diazaspiro[4.5]dec-7-yl)-5H-pyrido[4,3-b]indol-7-yl]piperidine-1-carboxylate | Calc'd 547.3, found 547.5 | 2.33 |
| 44 | 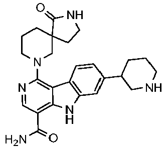 | 1-(1-oxo-2,7-diazaspiro[4.5]dec-7-yl)-7-piperidin-3-yl-5H-pyrido[4,3-b]indole-4-carboxamide | Calc'd 447.3, found 447.4 | 1.95 |
| 45 | 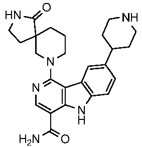 | 1-(1-oxo-2,7-diazaspiro[4.5]dec-7-yl)-8-piperidin-4-yl-5H-pyrido[4,3-b]indole-4-carboxamide | Calc'd 447.3, found 447.4 | 1.92 |
| 46 | 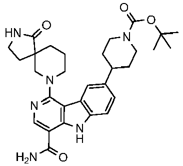 | *tert*-butyl 4-[4-carbamoyl-1-(1-oxo-2,7-diazaspiro[4.5]dec-7-yl)-5H-pyrido[4,3-b]indol-8-yl]piperidine-1-carboxylate | Calc'd 547.3, found 547.5 | 2.33 |
| 47 | 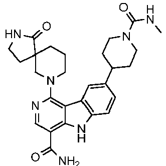 | 8-[1-(methylcarbamoyl)piperidin -4-yl]-1-(1-oxo-2,7-diazaspiro[4.5]dec-7-yl)-5H-pyrido[4,3-b]indole-4-carboxamide | Calc'd 504.3, found 504.4 | 2.16 |
| 48 | 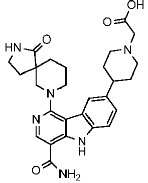 | {4-[4-carbamoyl-1-(1-oxo-2,7-diazaspiro[4.5]dec-7-yl)-5H-pyrido[4,3-b]indol-8-yl]piperidin-1-yl}acetic acid | Calc'd 505.3, found 505.4 | 1.93 |
| 49 | 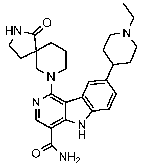 | 8-(1-ethylpiperidin-4-yl)-1-(1-oxo-2,7-diazaspiro[4.5]dec-7-yl)-5H-pyrido[4,3-b]indole-4-carboxamide | Calc'd 475.3, found 475.4 | 1.96 |
| 50 | 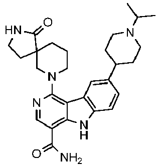 | 8-[1-(1-methylethyl)piperidin-4-yl]-1-(1-oxo- 2,7-diazaspiro[4.5]dec-7-yl)-5H-pyrido[4,3-b]indole-4-carboxamide | Calc'd 489.3, found 489.4 | 2.01 |
| 51 | 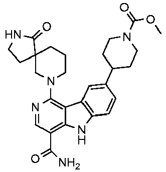 | methyl 4-[4-carbamoyl-1-(1-oxo-2,7-diazaspiro[4.5]dec-7-yl)-5H-pyrido[4,3-b]indol-8-yl]piperidine-1-carboxylate | Calc'd 505.3, found 505.4 | 2.35 |
| 52 | 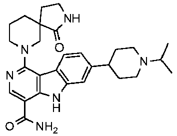 | 7-[1-(1-methylethyl)piperidin-4-yl]-1-(1-oxo-2,7-diazaspiro[4.5]dec-7-yl)-5H-pyrido[4,3-b]indole-4-carboxamide | Calc'd 489.3, found 489.4 | 2.02 |
| 53 | 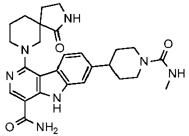 | 7-[1-(methylcarbamoyl)piperidin-4-yl]-1-(1-oxo-2,7-diazaspiro[4.5]dec-7-yl)-5H-pyrido[4,3-b]indole-4-carboxamide | Calc'd 504.3, found 504.4 | 2.20 |
| 54 | 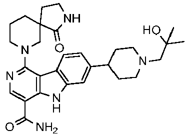 | 7-[1-(2-hydroxy-2-methylpropyl)piperidin-4-yl]-1-(1-oxo-2,7-diazaspiro[4.5]dec-7-yl)-5H-pyrido[4,3-b]indole-4-carboxamide | Calc'd 519.3, found 519.5 | 1.98 |
| 55 | 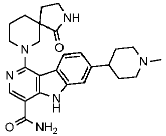 | 7-(1-methylpiperidin-4-yl)-1-(1-oxo- 2,7-diazaspiro[4.5]dec-7-yl)-5H-pyrido[4,3-b]indole-4-carboxamide | Calc'd 461.3, found 461.4 | 1.94 |
| 56 | 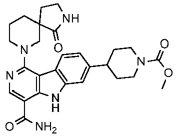 | methyl 4-[4-carbamoyl-1-(1-oxo-2,7-diazaspiro[4.5]dec-7-yl)-5H-pyrido[4,3-b]indol-7-yl]piperidine-1-carboxylate | Calc'd 505.3, found 505.4 | 2.38 |
| 57 | 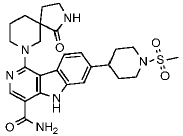 | 7-[1-(methylsulfonyl)piperidin-4-yl]-1-(1-oxo-2,7-diazaspiro[4.5]dec-7-yl)-5H-pyrido[4,3-b]indole-4-carboxamide | Calc'd 525.2, found 525.4 | 2.28 |
| 58 | 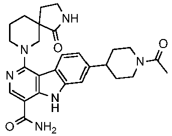 | 7-(1-acetylpiperidin-4-yl)-1-(1-oxo-2,7-diazaspiro[4.5]dec-7-yl)-5H-pyrido[4,3-b]indole-4-carboxamide | Calc'd 489.3, found 489.4 | 2.22 |
| 59 | 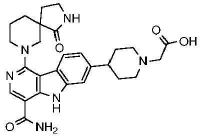 | {4-[4-carbamoyl-1-(1-oxo-2,7-diazaspiro[4.5]dec-7-yl)-5H-pyrido[4,3-b]indol-7-yl]piperidin-1-yl}acetic acid | Calc'd 505.3, found 505.4 | 1.94 |
| 60 | 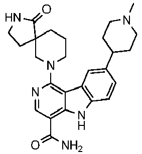 | 8-(1-methylpiperidin-4-yl)-1-(1-oxo-2,7-diazaspiro[4.5]dec-7-yl)-5H-pyrido[4,3-b]indole-4-carboxamide | Calc'd 461.3, found 461.4 | 1.925 |
| 61 | 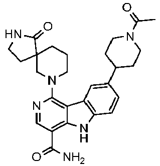 | 8-(1-acetylpiperidin-4-yl)-1-(1-oxo-2,7-diazaspiro[4.5]dec-7-yl)-5H-pyrido[4,3-b]indole-4-carboxamide | Calc'd 489.3, found 489.4 | 1.918 |
| 62 | 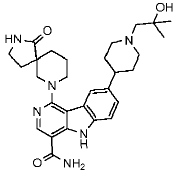 | 8-[1-(2-hydroxy-2-methylpropyl)piperidin-4-yl]-1-(1-oxo-2,7 - diazaspiro[4.5]dec-7-yl)-5H-pyrido[4,3-b]indole-4-carboxamide | Calc'd 519.3, found 519.5 | 1.966 |
| 63 | 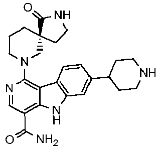 | 1-[(5S)-1-oxo-2,7-diazaspiro[4.5]dec-7-yl]-7-piperidin-4-yl-5H-pyrido[4,3-b]indole-4-carboxamide | Calc'd 447.3, found 447.4 | 1.927 |
| 64 | 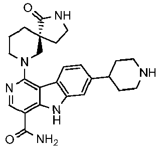 | 1-[(5R)-1-oxo-2,7-diazaspiro[4.5]dec-7-yl]-7-piperidin-4-yl-5H-pyrido[4,3-b]indole-4-carboxamide | Calc'd 447.3, found 447.4 | 1.931 |
| 65 | 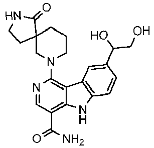 | 8-(1,2-dihydroxyethyl)-1-(1-oxo-2,7-diazaspiro[4.5]dec-7-yl)-5H-pyrido[4,3-b]indole-4-carboxamide | Calc'd 424.2, found 424.3 | 1.915 |
| 66 | 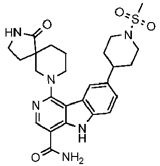 | 8-[1-(methylsulfonyl)piperidin-4-yl]-1-(1-oxo-2,7-diazaspiro[4.5]dec-7-yl)-5H-pyrido[4,3-b]indole-4-carboxamide | Calc'd 525.2, found 525.4 | 2.270 |
| 67 | 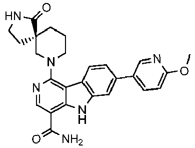 | 7-(6-methoxypyridin-3-yl)-1-[(5R)-1-oxo-2,7-diazaspiro[4.5]dec-7-yl]-5H-pyrido[4,3-b]indole-4-carboxamide | Calc'd 471.2, found 471.4 | 2.407 |
| 68 | 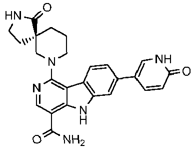 | 1-[(5R)-1-oxo-2,7-diazaspiro[4.5]dec-7-yl]-7-(6-oxo-1,6-dihydropyridin-3-yl)-5H-pyrido[4,3-blindole-4-carboxamide | Calc'd 457.2, found 457.3 | 2.071 |
| 69 | 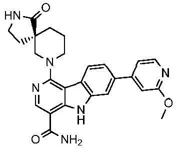 | 7-(2-methoxypyridin-4-yl)-1-[(5R)-1-oxo-2,7-diazaspiro[4.5]dec-7-yl]-5H-pyrido[4,3-b]indole-4-carboxamide | Calc'd 471.2, found 471.4 | 2.340 |
| 70 | 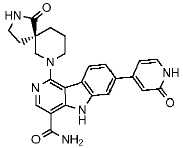 | 1-[(5R)-1-oxo-2,7-diazaspiro[4.5]dec-7-yl]-7-(2-oxo-1,2-dihydropyridin-4-yl)-5H-pyrido[4,3-b]indole-4-carboxamide | Calc'd 457.2, found 457.4 | 2.071 |
| 71 | 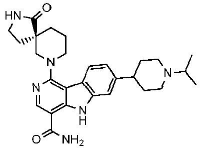 | 7-[1-(1-methylethyl)piperidin-4-yl]-1-[(5R)-1-oxo-2,7-diazaspiro[4.5]dec-7-yl]-5H-pyrido[4,3-b]indole-4-carboxamide | Calc'd 489.3, found 489.4 | 1.681 |
| 72 | 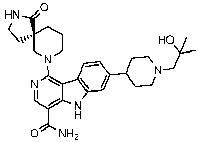 | 7-[1-(2-hydroxy-2-methylpropyl)piperidin-4-yl]-1-[(5R)-1-oxo-2, 7-diazaspiro[4.5]dec-7-yl]-5H-pyrido[4,3-b]indole-4-carboxamide | Calc'd 519.3, found 519.5 | 2.005 |
| 73 | 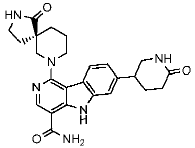 | 1-[(5R)-1-oxo-2,7-diazaspiro[4.5]dec-7-yl]-7-(6-oxopipendin-3-yl)-5H-pyrido[4,3-b]indole-4-carboxamide | Calc'd 461.2, found 461.3 | 2.091 |
| 74 | 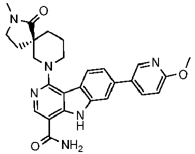 | 7-(6-methoxypyridin-3-yl)-1-[(5R)-2-methyl-1-oxo-2,7-diazaspiro[4.5] dec-7-yl]-5H-pyrido[4,3-b]indole-4-carboxamide | Calc'd 485.2, found 485.4 | 2.428 |
| 75 | 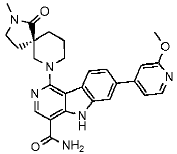 | 7-(2-methoxypyridin-4-yl)-1-[(5R)-2-methyl-1-oxo-2,7-diazaspiro[4.5]dec-7-yl]-5H-pyrido[4,3-b]indole-4-carboxamide | Calc'd 485.2, found 485.3 | 2.367 |
| 76 | 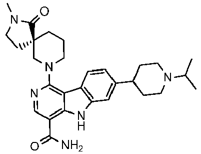 | 7-[1-(1-methylethyl)piperidin-4-yl]-1-[(5R)-2-methyl-1-oxo-2,7-diazaspiro[4.5]dec-7-yl]-5H-pyrido[4,3-b]indole-4-carboxamide | Calc'd 503.3, found 503.4 | 1.748 |
| 77 | 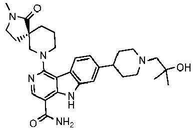 | 7-[1-(2-hydroxy-2-methylpropyl)piperidin-4-yl]-1-[(5R)-2-methyl-1-oxo-2,7-diazaspiro[4.5]dec-7-yl]-5H-pyrido[4,3-b]indole-4-carboxamide | Calc'd 533.3, found 533.4 | 2.016 |
| 78 | 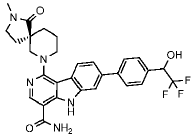 | 1-[(5R)-2-methyl-1-oxo-2,7-diazaspiro[4.5]dec-7-yl]-7-[4-(2,2,2-trifluoro-1-hydroxyethyl)phenyl]-5H-pyrido[4,3-b]indole-4-carboxamide | Calc'd 552.2, found 552.4 | 2.244 |
| 79 | 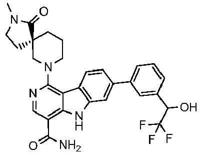 | 1-[(5R)-2-methyl-1-oxo-2,7-diazaspiro[4.5]dec-7-yl]-7-[3-(2,2,2-trifluoro-1-hydroxyethyl)phenyl]-5H-pyrido[4,3-b]indole-4-carboxamide | Calc'd 552.2, found 552.4 | 2.266 |
| 80 | 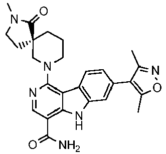 | 7-(3,5-dimethylisoxazol-4-yl)-1-[(5R)-2-methyl-1-oxo-2,7-diazaspiro[4.5]dec-7-yl]-5H-pyrido[4,3-b]indole-4-carboxamide | Calc'd 473.2, found 473.4 | 2.499 |
| 81 | 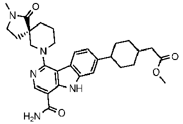 | methyl (4-{4-carbamoyl-1-[(5R)-2-methyl-1-oxo-2,7-diazaspiro[4.5]dec-7-yl]-5H-pyrido[4,3-b]indol-7-yl}cyclohexyl)acetate | Calc'd 532.3, found 532.2 | 2.519 |
| 82 | 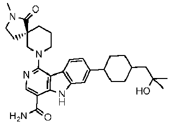 | 7-[4-(2-hydroxy-2-methylpropyl)cyclohexyl]-1-[(5R)-2-methyl-1-oxo-2,7-diazaspiro[4.5]dec-7-yl]-5H-pyrido[4,3-b]indole-4-carboxamide | Calc'd 532.3, found 532.5 | 2.355 |

### Example 83

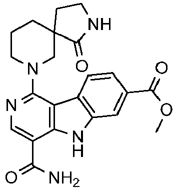

### Methyl 4-carbamoyl-1-(1-oxo-2,7-diazaspiro[4.5]decan-7-yl)-5H-pyrido[4,3-b]indole-7-carboxylate

To a solution of 7-bromo-1-(1-oxo-2,7-diazaspiro[4.5]decan-7-yl)-5H-pyrido[4,3-b]indole-4-carboxamide(200 mg, 0.45 mmol) and CH₃COONa (111 mg, 1.36 mmol) in MeOH/DMF (5 mL/1 mL) was added Pd(OAc)₂ (10 mg, 0.045 mmol) and DPPF (25 mg, 0.045 mmol). The mixture was stirred under CO (50 psi) at 80°C for 14 h. The mixture was cooled to ambient temperature and filtered, and then the filtrate was concentrated *in vacuo* and purified by prep-HPLC to afford methyl4-carbamoyl-1-(1-oxo-2,7-diazaspiro[4.5]decan-7-yl)-5H-pyrido[4,3-b]indole-7-carboxylate.MS: 422.3[M+H]⁺. ¹H NMR (400 MHz, CD₃OD) δ 8.56 (s, 1H), 8.51 (s, 1H), 8.15 (dd, *J*=1.0, 8.5 Hz, 1H), 8.09 - 8.03 (m, 1H), 3.99 (s, 4H), 3.90 - 3.78 (m, 2H), 3.65 (d, *J*=5.0 Hz, 1H), 3.40 - 3.34 (m, 1H), 3.26 - 3.20 (m, 1H), 2.26 - 2.06 (m, 5H), 1.96 - 1.83 (m, 1H) ppm.

The following examples in Table 3 were prepared using procedures similar to thosedescribed in Example 83.

**Table 3**

| **Ex. No.** | **Structure** | **Name** | **Exact Mass [M+H]+** | **Retention time (min)** |
|---|---|---|---|---|
| 84 | 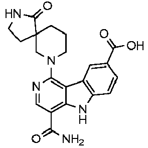 | 4-carbamoyl-1-(1-oxo-2,7-diazaspiro [4.5] dec-7-yl)-5H-pyrido[4,3-b]indole-8-carboxylic acid | Calc'd 408.2, found 408.3 | 2.004 |
| 85 | 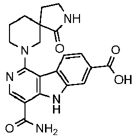 | 4-carbamoyl-1-(1-oxo-2,7-diazaspiro[4.5]dec-7-yl)-5H-pyrido[4,3-b]indole-7-carboxylic acid | Calc'd 408.2, found 408.3 | 2.054 |

### Example 86

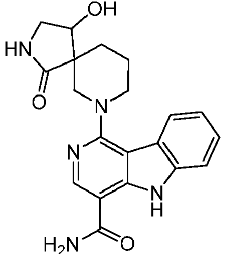

### 1-(4-hydroxy-1-oxo-2,7-diazaspiro[4.5]decan-7-yl)-5H-pyrido[4,3-b]lindole-4-carboxamide

### Step 1: 1-(4-((tert-butyldimethylsilyl)oxy)-1-oxo-2,7-diazaspiro[4.5]decan-7-yl)-5H-pyrido[4,3-b]indole-4-carboxamide

A mixture of 4-((*tert*-butyldimethylsilyl)oxy)-2,7-diazaspiro[4.5]decan-1-one (300 mg, 1.22 mmol), 1-chloro-5H-pyrido[4,3-b]indole-4-carboxamide (417 mg, 1.46 mmol) and DIPEA (630 mg, 4.88 mmol) in 1-methylpyrrolidin-2-one (4 mL) was stirred in a microwave at 150°C for 1.5 h. To the mixture was added water (10 mL) and DCM (50 mL). After partition, the aqueous layer was extracted with DCM/i-PrOH (v:v=3:1, 20 mL × 3). The combined organic layers were washed with brine (10 mL), dried over sodium sulfate, filtered and concentrated to afford the crude product, which was purified by silica gel columnchromatography (MeOH / DCM = 0% ∼ 10 %) to give 1-(4-((*tert-*butyldimethylsilyl)oxy)-1-oxo-2,7-diazaspiro[4.5]decan-7-yl)-5H-pyrido[4,3-b]indole-4-carboxamide. MS: 494.2 [M+H]⁺.

### Step 2: 1-(4-hydroxy-1-oxo-2,7-diazaspiro[4.5]decan-7₋yl)-5H-pyrido[4,3-b]indole-4-carboxamide

To a solution of 1-(4-((*tert*-butyldimethylsilyl)oxy)-1-oxo-2,7-diazaspiro[4.5]decan-7-yl)-5H-pyrido[4,3-b]indole-4-carboxamide (300 mg, 0.608 mmol) in anhydrousTHF (10 mL) was added TBAF (318 mg, 1.22 mmol)portionwise. The reaction mixture was stirred at 15 °C for 2 h, then warmed to 50°C and stirred for another 15 h. The reaction was quenched with water (30 mL) and extracted with EtOAc (30 mL×3). After concentration, the residue was purified with prep-HPLC to afford 1-(4-hydroxy-1-oxo-2,7-diazaspiro[4.5]decan-7-yl)-5H-pyrido[4,3-b]indole-4-carboxamide as a diastereomeric mixture containing all four isomers. MS: 380.1[M+H]⁺.

### Example 87

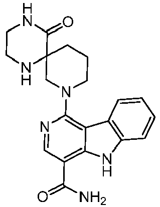

### 1-(5-oxo-1,4,8-triazaspiro[5.5]undecan-8-yl)-5H-pyrido[4,3 -b]indole-4-carboxamide

### Step 1: benzyl 2,4-dioxo-1,3,7-triazaspiro[4.5]decane-7-carboxylate

To a solution of benzyl 3-oxopiperidine-1-carboxylate (30 g, 129 mmol) in MeOH (130 mL) and H₂O (170mL) was added (NH₄)₂CO₃ (24.8 g,258 mmol) and KCN (16.7 g, 258mmol). The solution was stirred at 40 °C in a sealed tube for 48 h,and then the resulting solid was filtered and washed with water(1L).The benzyl 2,4-dioxo-1,3,7-triazaspiro[4.5]decane-7-carboxylate was obtained and dried *in vacuo.* ¹H NMR (400 MHz, CD₃OD) δ 7.35 (br. s.,5H), 5.14 - 5.07 (m, 2H), 4.60 (br. s., 1H), 3.84 (br. s., 1H), 3.49 - 3.34 (m, 1H), 3.25 - 3.10 (m, 1H), 2.20 - 1.93 (m, 1H), 1.92 - 1.53 (m, 3H) ppm.

### Step 2: 7-benzyl 1,3-di-tert-butyl 2,4-dioxo-1,3,7-triazaspiro[4.5]decane-1,3,7-tricarboxylate

A mixture of benzyl 2,4-dioxo-1,3,7-triazaspiro[4.5]decane-7-carboxylate (6 g, 19.8 mmol) di-*tert*-butyl dicarbonate (17.1g, 79.2mmol,TEA(1.94g, 19.8mmol) and DMAP(30mg) in dry DME (200mL) was stirred at 25 °Cfor 18 h. The solvent was removed. The solid was washed with isopropyl ether to afford 7-benzyl 1,3-di-*tert*-butyl 2,4-dioxo-1,3,7-triazaspiro[4.5]decane-1,3,7-tricarboxylate. ¹H NMR (400 MHz, CD₃OD) δ 7.42 - 7.27 (m, 5H), 5.23 - 4.98 (m, 2H), 4.42 - 4.17 (m, 2H), 4.01 - 3.81 (m, 1H), 2.99 - 2.80 (m, 1H), 2.76 - 2.58 (m, 1H), 2.39 - 2.19 (m, 1H), 2.06 (d, J=13.3 Hz, 1H), 1.74 (d, J=13.6 Hz, 1H), 1.60 - 1.54 (m, 18H) ppm.

### Step 3: 3-amino-1-((benzyloxy)carbonyl)piperidine-3-carboxylic acid

To a solution of 7-benzyl 1,3-di-*tert*-butyl 2,4-dioxo-1,3,7-triazaspiro[4.5]decane-1,3,7-tricarboxylate (24g, 47.7 mmol) in THF(300mL) was added 1.0N LiOH (382mL) aqueous solution, and the resulting mixture was stirred at 23 °C for 18 h. THF was removed under vacuum. HCl(382mL,1N) was added to the residue at 0 °C to adjust the pH = 6∼7. The mixture was concentrated *in vacuo* to 250mL and filtered. After drying, 3-amino-1-((benzyloxy)carbonyl)piperidine-3-carboxylic acid was obtained. ¹H NMR (400 MHz, DDMSO-*d*₆) δ 7.42 - 7.32 (m, 5H), 5.06 (br. s., 2H), 3.95 - 3.83 (m, 2H), 2.83 (br. s., 1H), 2.01 - 1.87 (m, 1H), 1.79 - 1.45 (m, 3H) ppm.

### Step 4: 1-benzyl 3-methyl 3-aminopiperidine-1,3-dicarboxylate

To a solution of 3-amino-1-((benzyloxy)carbonyl)piperidine-3-carboxylic acid (5.2 g, 18.7mmol) in MeOH (60 mL) was added methanesulfonic acid (7mL).Thesolution was refluxed for 18 h. The solvent was removed *in vacuo*, then water(50mL) was added and the pH adjusted to 7∼8 with satd. NaHCO₃ (aq.).The aqueous solution was extracted with DCM:MeOH(V:V=10:1,100 mL).The extract was washed with brine(50mL), dried over Na₂SO₄, filtered, concentrated *in vacuo* to afford 1-benzyl 3-methyl 3-aminopiperidine-1,3-dicarboxylate. ¹H NMR (400 MHz, CDCl₃) δ= 7.38 - 7.29 (m, 5H), 5.14 (d, J=7.0 Hz, 2H), 3.81 - 3.48 (m, 6H), 3.20 (br. s., 1H), 2.06 - 1.94 (m, 1H), 1.88 - 1.75 (m, 1H), 1.71 - 1.65 (m, 1H), 1.61 - 1.52 (m, 1H) ppm.

### Step 5: 1-benzyl 3-methyl 3-((2-((tert-butoxycarbonyl)amino)ethyl)amino)piperidine-1,3-dicarbo xylate

To a solution of 1-benzyl 3-methyl 3-aminopiperidine-1,3-dicarboxylate (1.3 g, 4.5mmol) in MeOH (15mL) was added *tert*-butyl (2-oxoethyl)carbamate (702mg,4.5mmol) and NaCNBH₃ (706 mg, 11.2mmol). The mixture was stirred at 26 °C under N₂ for 30 h. The solution was concentrated *in vacuo.* The residue was purified by silica gel column chromatography (THF: PE, =0%∼60%) to afford 1-benzyl 3-methyl 3-((2-((*tert-*butoxycarbonyl)amino)ethyl)amino)piperidine-1,3-dicarboxylate. ¹H NMR (400 MHz, CD₃OD)δ 7.41 - 7.26 (m, 5H), 5.12 (br. s., 2H), 4.11 (d, J=12.3 Hz, 1H), 3.88 - 3.64 (m, 5H), 3.48 - 3.32 (m, 3H), 3.19 (br. s., 2H), 2.35 - 2.03 (m, 2H), 1.93 - 1.66 (m, 2H), 1.44 (s, 9H) ppm.

### Step 6: benzyl 5-oxo-1,4,8-triazaspiro[5.5]undecane-8-carboxylate

To a solution of 1-benzyl 3-methyl 3-((2-((*tert-*butoxycarbonyl)amino)ethyl)amino)piperidine-1,3-dicarboxylate (700mg, 1.6 mmol) in MeOH(5mL) was added HCl/MeOH(4mL). The solution was stirred at 27 °C for 1 hour, then concentrated *in vacuo.* The residue was dissolved in MeOH(5mL) and water (5mL), then K₂CO₃ (1.24g,9 mmol) was added. The mixture was stirred at 27 °C for 18h. The mixture was concentrated *in vacuo* and the residue was extracted with EtOAc (50mL). The organic layers were washed with brine (30 mL), dried over Na₂SO₄, filtered, and concentrated *in vacuo.* The residue was purified by prep-HPLC to afford benzyl 5-oxo-1,4,8-triazaspiro[5.5]undecane-8-carboxylate. ¹H NMR (400 MHz, CD₃OD) δ 7.45 - 7.26 (m, 5H), 5.15 (br. s., 2H), 4.49 (d, J=10.3 Hz, 1H), 4.16 (d, J=12.8 Hz, 1H), 3.64 - 3.36 (m, 5H), 3.02 (t, J=11.5 Hz, 1H), 2.38 (dt, J=4.9, 14.2 Hz, 1H), 2.10 (d, J=13.3 Hz, 1H), 1.88 - 1.57 (m, 2H) ppm.

### Step 7: 1,4,8-triazaspiro[5.5]undecan-5-one

To a solution of benzyl 5-oxo-1,4,8-triazaspiro[5.5]undecane-8-carboxylate (80 mg, 0.26 mmol) in MeOH (5 mL) was added Pd/C (100 mg). The mixture was stirred at 25 °C for 18 h under H₂. The mixture was filtered and concentrated *in vacuo* to afford crude 1,4,8-triazaspiro[5.5]undecan-5-one. MS: 304.0 [M+H]⁺.

### Step 8: 1-(5-oxo-1,4,8-triazaspiro[5.5]undecan-8-yl)-5H-pyrido[4,3-b]indole-4-carboxamide

To a solution of 1,4,8-triazaspiro[5.5]undecan-5-one (30 mg, 0.18mmol) in NMP (2 mL) was added 1-chloro-5H-pyrido[4,3-b]indole-4-carboxamide (44 mg, 0.18mmol) and DIEA (70 mg, 0.54mmol), and the mixture was heated under microwave irradiation for 1.5 h at 150 °C. The reaction solution was purified by prep-HPLC to afford 1-(5-oxo-1,4,8-triazaspiro[5.5]undecan-8-yl)-5H-pyrido[4,3-b]indole-4-carboxamide. MS: 379.3 [M+H]⁺. ¹H NMR (400 MHz, DMSO-*d*₆) δ 11.93 (s, 1H), 9.67 (br. s., 1H), 8.70 (s, 1H), 8.54 (br. s., 1H), 8.19 (br. s., 1H), 8.05 (d, J=7.8 Hz, 1H), 7.83 (d, J=8.0 Hz, 1H), 7.54 (br. s., 1H), 7.48 (t, J=7.4 Hz, 1H), 7.38 - 7.31 (m, 1H), 4.39 (d, J=14.1 Hz, 1H), 3.93 (d, J=12.5 Hz, 2H), 3.56 - 3.49 (m, 2H), 3.45 (br. s., 2H), 2.98 (t, J=11.5 Hz, 1H), 2.40 - 2.21 (m, 2H), 2.20 - 2.05 (m, 1H), 1.84 (d, J=13.1 Hz, 1H) ppm.

### Example 88

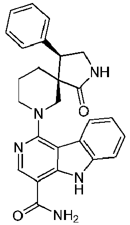

### 1-[(trans)-1-oxo-4-phenyl-2,7-diazaspiro[4.5]dec-7-yl]-5H-pyrido[4,3-b]indole-4-carboxamide Step 1: 1-tert-butyl 3-methyl 3-(2-nitro-1-phenylethyl)piperidine-1,3-dicarboxylate (isomer 1) and 1-tert-butyl 3-methyl 3-(2-nitro-1-phenylethyl)piperidine-1,3-dicarboxylate (isomer 2)

To a solution of 1-*tert*-butyl 3-methyl piperidine-1,3-dicarboxylate (10 g, 41.1 mmol) in anhydrous THF (200 mL) was added LDA (28.8 mL, 57.5 mmol) dropwise at -78 °C. The mixture was stirred at -40 °C for 40 min. Then a solution of (Z)-(2-nitrovinyl)benzene (7.35 g, 49.3 mmol) in anhydrous THF (50 mL) was added at -78 °C dropwise and the mixture was stirred at this temperature for 2 h. The mixture was quenched with satd. NH₄Cl (100 mL) slowly at -78 °C and extracted with ethyl acetate (100 mL× 3).The combined organic layers were washed with brine (30 mL), dried over sodium sulfate, filtered and concentrated to afford the crude product, which was purified by silica gel column chromatography (EtOAc/PE = 0 ∼ 25%) to give 1-*tert*-butyl 3-methyl 3-(2-nitro-1-phenylethyl)piperidine-1,3-dicarboxylate (isomer 1).¹H NMR (400 MHz, CDCl₃) δ 7.34 - 7.27 (m, 3H), 7.09 (dd, J=1.9, 7.4 Hz, 2H), 5.19 - 4.89 (m, 2H), 3.96 - 3.75 (m, 2H), 3.73 (s, 3H), 3.53 - 3.20 (m, 3H), 1.90 - 1.77 (m, 1H), 1.75 - 1.62 (m, 1.5H), 1.60 - 1.48 (m, 1.5H), 1.47 (s, 9H) ppm and 1-*tert*-butyl 3-methyl 3-(2-nitro-1-phenylethyl)piperidine-1,3-dicarboxylate (isomer 2). ¹H NMR (400 MHz, CDCl₃) = 7.34 - 7.27 (m, 3H), 7.09 (dd, *J*=2.1, 7.4 Hz, 2H), 5.03 - 4.94 (m, 1H), 4.92 - 4.86 (m, 1H), 4.07 - 3.76 (m, 1H), 3.70 (dd, *J*=4.0, 11.0 Hz, 2H), 3.64 (s, 3H), 2.94 (d, *J*=13.3 Hz, 2H), 2.27 - 2.14 (m, 1H), 1.69 (br. s., 1H), 1.45 (s, 2H), 1.39 (s, 9H) ppm.

### Step 2: 1-tert-butyl 3-methyl 3-(2-amino-1-phenylethyl)piperidine-1,3-dicarboxylate(isomer1)

To a solution of 1-*tert*-butyl 3-methyl 3-(2-nitro-1-phenylethyl)piperidine-1,3-dicarboxylate (isomer 1) (500 mg, 1.27 mmol) in anhydrous MeOH (10 mL) was added NiCl₂ (169 mg, 1.27 mmol) and the mixture was cooled with an ice bath. NaBH₄ (578 mg, 15.3 mmol) was added and the resulting suspension was stirred at 0 °C∼ 25 °C for one hour. The mixture was quenched with 10% NH₃·H₂O (50 mL) and stirred vigorously at 25 °C until the suspension dissolved to give a purple aqueous solution. The organic solvent was separated and the aqueous layer was extracted with EtOAc (50 mL × 2). The combined organic layers were washed with brine (20 mL), dried over sodium sulfate, concentrated to give 1-*tert*-butyl 3-methyl 3-(2-amino-1-phenylethyl)piperidine-1,3-dicarboxylate (isomer 1). MS: 307.0(M-55).

### Step 3:(cis)-tert-butyl 1-oxo-4-phepyl-2,7-diazaspiro[4.5]decane-7-carboxylate

A mixture of 3-(2-amino-1-phenylethyl)piperidine-1,3-dicarboxylate(isomer 1) (500 mg, 1.38 mmol ) in toluene (10 mL) was stirred at 120 °C for 3 h. The mixture was concentrated to afford the crude product, which was purified by silica gel column chromatography (THF/PE = 20% ∼ 70%) to give (cis)-*tert*-butyl 1-oxo-4-phenyl-2,7-diazaspiro[4.5]decane-7-carboxylate. ¹H NMR (400 MHz, CDCl₃) δ 7.34 - 7.26 (m, 3H), 7.25 - 7.20 (m, 2H), 6.63 - 6.19 (m, 1H), 3.87 - 3.27 (m, 5H), 3.21 - 2.77 (m, 2H), 2.23 - 1.81 (m, 4H), 1.45 - 1.07 (m, 9H) ppm.

### Step 4: (cis)-4-phepyl-2,7-diazaspiro[4.5]decan-1-one

A mixture of *tert*-butyl 1-oxo-4-phenyl-2,7-diazaspiro[4.5]decane-7-carboxylate (isomer 1) (100 mg, 0.303 mmol) in MeOH (5 mL) was added HCl-dioxane (5 mL) and the mixture was stirred at 25 °C for 2 h. The mixture was concentrated to afford (cis)-4-phenyl-2,7-diazaspiro[4.5]decan-1-one hydrochloride, which was immediately used in the next step. MS: 231.1[M+H]⁺.

### Step 5: 1-((cis)-1-oxo-4-phepyl-2,7-diazaspiro[4.5]decan-7-yl)-5H-pyrido[4.3-b]indole-4-carbox amide

Following the same procedure as step 4 of Example 1, 1-((cis)-1-oxo-4-phenyl-2,7-diazaspiro[4.5]decan-7-yl)-5H-pyrido[4,3-b]indole-4-carboxamide was obtained. MS: 440.4 [M+H]⁺. ¹H NMR (400 MHz, CD₃OD) δ 8.54 (s, 1H), 7.98 (d, J=8.0 Hz, 1H), 7.86 (d, J=8.0 Hz, 1H), 7.64 (t, J=7.4 Hz, 1H), 7.56 - 7.50 (m, 1H), 7.46 - 7.33 (m, 5H), 4.11 (d, J=12.5 Hz, 1H), 3.88 (d, J=12.8 Hz, 1H), 3.79 - 3.71 (m, 1H), 3.70 - 3.61 (m, 2H), 3.60 - 3.49 (m, 2H), 2.20 - 2.06 (m, 1H), 2.04 - 1.94 (m, 1H), 1.74 (td, J=3.3, 6.9 Hz, 1H), 1.53 (ddd, J=4.0, 9.5, 13.6 Hz, 1H) ppm.

### Example 89

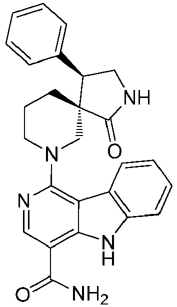

### 1-[(trans)-1-oxo-4-phenyl-2,7-diazaspiro[4.5]dec-7-yl]-5H-pyrido[4,3-b]indole-4-carboxamide

Using the 1-*tert*-butyl 3-methyl 3-(2-nitro-1-phenylethyl)piperidine-1,3-dicarboxylate (isomer 2) that was obtained from step 1 of Example 9, 1-[(trans)-1-oxo-4-phenyl-2,7-diazaspiro[4.5]dec-7-yl]-5H-pyrido[4,3-b]indole-4-carboxamide was prepared following the same procedure as Example 88. MS: 440.2 [M+H]⁺. ¹H NMR (400 MHz, CD₃OD) δ 8.33 (s, 1H), 7.86 - 7.76 (m, 2H), 7.60 (t, J=7.7 Hz, 1H), 7.48 - 7.41 (m, 1H), 6.98 (d, J=7.5 Hz, 2H), 6.87 - 6.80 (m, 1H), 6.79 - 6.69 (m, 2H), 4.09 (d, J=14.3 Hz, 1H), 3.91 (dd, J=7.3, 10.8 Hz, 1H), 3.75 (d, J=12.8 Hz, 1H), 3.67 - 3.52 (m, 3H), 3.37 (s, 1H), 2.48 - 2.34 (m, 1H), 2.27 - 2.13 (m, 3H) ppm.

### Examples 90 - 92

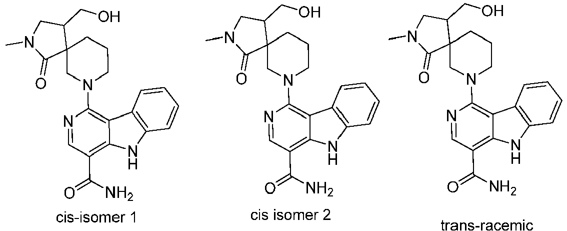

### Cis-1-(-4-(hydroxymethyl)-2-methyl-1-oxo-2,7-diazaspiro[4.5]decan-7-yl)-5H-pyrido[4.3-blindole-4-carboxamide (isomer 1), cis-1-(4-(hydroxymethyl)-2-methyl-1-oxo-2,7-diazaspiro[4.5]decan-7-yl)-5H-pyrido[4,3-b]indole-4-carboxamide (isomer 2) and trans-1-(4-(hydroxymethyl)-2-methyl-1-oxo-2,7-diazaspiro[4.5]decan-7-yl)-5H-pyrido[4,3-b]indole-4-carboxamide

### Step 1: 1-tert-butyl 3-methyl 3-(1-(benzyloxy)-3-nitropropan-2-yl)piperidine-1,3-dicarboxylate

To a solution of 1-*tert*-butyl 3-methyl piperidine-1,3-dicarboxylate (2.6 g, 13.6 mmol) in anhydrous THF (50 mL) was added LDA (8.2 mL, 16.3 mmol) dropwise at -78 °C. The mixture was stirred at -40 °C for 40 mins. Then a solution of (E)-(((3-nitroallyl)oxy)methyl)-benzene (3.3 g, 13.6 mmol) in anhydrous THF (50 mL) was added at -40 °C dropwise and the mixture was stirred at thistemperature for 2 h. The mixture was quenched withsatd.NH₄Cl (50 mL) slowly at -78 °C ∼ 0 °C and extracted with ethyl acetate (100 mL × 3). The combined organic layers were washed with brine (50 mL), dried over sodium sulfate, filtered and concentrated to afford the crude product, which was purified by silica gel column chromatography (EtOAc/PE = 0 ∼ 30%) to give 1-*tert*-butyl 3-methyl 3-(1-(benzyloxy)-3-nitropropan-2-yl)piperidine-1,3-dicarboxylate as an oil. MS: 459.1[M+H]⁺.

### Step 2: tert-butyl 4-((benzyloxy)methyl)-1-oxo-2,7-diazaspiro[4.5]decane-7-carboxylate

To a solution of 1-*tert*-butyl 3-methyl 3-(1-(benzyloxy)-3-nitropropan-2-yl)piperidine-1,3-dicarboxylate (350 mg, 0.80 mmol) in MeOH (20 mL) was added NH₃·H₂O (1 mL) and Raney Ni (50 mg). The mixture was stirred at 30 °C underH₂(20 psi) for 3 h. The mixture was filtered and all volatiles were evaporated to give *tert*-butyl 4-((benzyloxy)methyl)-1-oxo-2,7-diazaspiro[4.5]decane-7-carboxylate as an oil, which was immediately used in the next step. ¹H NMR (400 MHz, CDCl₃) δ= 7.38 - 7.27 (m, 5H), 5.70 (br. s., 1H), 4.55 - 4.45 (m, 2H), 4.16 - 3.80 (m, 2H), 3.79 - 3.71 (m, 1H), 3.53 - 3.42 (m, 2H), 3.40 - 3.28 (m, 1H), 3.07 - 2.68 (m, 2H), 2.60 - 2.47 (m, 1H), 1.88 - 1.64 (m, 4H), 1.47 - 1.44 (m, 9H) ppm.

### Step 3: tert-butyl 4-((benzyloxy)methyl)-2-methyl-1-oxo-2,7-diazaspiro[4.5]decane-7-carboxylate

To a solution of *tert*-butyl 4-((benzyloxy)methyl)-1-oxo-2,7-diazaspiro[4.5]decane-7-carboxylate (160 mg, 0.427 mmol) in anhydrous DMF (5 mL) was added NaH (60%, 23 mg, 0.555 mmol) at 0 °C under nitrogen, the mixture was stirred at 0 °C for 10 mins, then MeI (1.0 g, 7.04 mmol) was added and the mixture was stirred at 25 °C for one hour. The mixture was quenched with H₂O (2 mL) and extracted with EtOAc (10 mL × 2), the combined organic layers were washed with brine (10 mL × 3), dried over sodium sulfate, and concentrated togive crude *tert*-butyl 4-((benzyloxy)methyl)-2-methyl-1-oxo-2,7-diazaspiro[4.5]decane-7-carboxylate as a solid, which was immediately used in the next step. MS: 389.1[M+H]⁺.

### Step 4: 4-((benzyloxy)methyl)-2-methyl-2,7-diazaspiro[4.5]decan-1-one

To a mixture of *tert*-butyl 4-((benzyloxy)methyl)-2-methyl-1-oxo-2,7-diazaspiro[4.5]decane-7-carboxylate (160 mg, 0.412 mmol) in MeOH (2 mL) was added HCl-dioxane (2 mL) and the mixture was stirred at 25 °C for one hour. The mixture was concentrated to afford the crude 4-((benzyloxy)methyl)-2-methyl-2,7-diazaspiro[4.5]decan-1-one as an oil, which was immediately used in the next step. MS: 288.9[M+H]⁺.

### Step 5: 1-(4-((benzyloxy)methyl)-2-methyl-1-oxo-2,7-diazaspiro[4.5]decan-7-yl)-5H-pyrido[4,3-b]indole-4-carboxamide

A mixture of 4-((benzyloxy)methyl)-2-methyl-2,7-diazaspiro[4.5]decan-1-one (118 mg, 0.407 mmol), 1-chloro-5H-pyrido[4,3-b]indole-4-carboxamide (100 mg, 0.407 mmol) and DIPEA (0.3 mL, 1.63 mmol) in 1-methylpyrrolidin-2-one (3 mL) was stirred in a microwave at 150 °C for 2 h. To the mixture was added water (10 mL). The mixture was extracted with DCM/i-PrOH (3:1, 20 mL × 3). The combined organic layers were washed with brine (10 mL), dried over sodium sulfate, filtered and concentrated to afford the crude product, which was purified by silica gel column chromatography (THF / PE = 30% ∼ 80 %) to give 1-(4-((benzyloxy)methyl)-2-methyl-1-oxo-2,7-diazaspiro[4.5]decan-7-yl)-5H-pyrido[4,3-b]indole-4-carboxamide as a solid. MS: 498.2[M+H]⁺.

### Step 6: 1-(4-(hydroxymethyl)-2-methyl-1-oxo-2,7-diazaspiro[4.5]decan-7-yl)-5H-indeno[1,2-c]p yridine-4-carboxamide

To a solution of 1-(4-((benzyloxy)methyl)-2-methyl-1-oxo-2,7-diazaspiro[4.5]decan-7-yl)-5H-pyrido[4,3-b]indole-4-carboxamide (160 mg, 0.32 mmol) in methanol (10 mL) was added Pd(OH)₂ (50 mg). The resulting solution was stirred at 40°C under 55 psi hydrogen pressure overnight. The mixture was filtered, and the filtrate was concentrated under reduced pressure to afford 1-(4-(hydroxymethyl)-2-methyl-1-oxo-2,7-diazaspiro[4.5]decan-7-yl)-5H-indeno[1,2-c]pyridine-4-carboxamide.

### Step 7: cis-1-(4-(hydroxymethyl)-2-methyl-1-oxo-2,7-diazaspiro[4.5]decan-7₋yl)-5H-pyrido[4,3-b]indole-4-carboxamide,cis-1-(4-(hydroxymethyl)-2-methyl-1-oxo-2,7-diazaspiro[4.5]decan-7-yl )-5H-pyrido[4,3-b]indole-4-carboxamide and trans-1-(4-(hydroxymethyl)-2-methyl-1-oxo-2,7-di azaspiro[4.5]decan-7-y1)-5H-pyrido[4,3-b]indole-4-carboxamide

The mixture of the four stereoisomers of 1-(4-(hydroxymethyl)-2-methyl-1-oxo-2,7-diazaspiro[4.5]decan-7-yl)-5H-indeno[1,2-c]pyridine-4-carboxamidewas purified by chiral SFC (Column: Chiralcel OD-H 250×4.6mm I.D., 5um Mobile phase: 40% ethanol (0.05% DEA) in CO₂ Flow rate: 2.35mL/min Wavelength: 220nm) to afford three stereoisomers as shown below.

| **Ex. No.** | **Name** | Eluting sequence on SFC | ¹H NMR |
|---|---|---|---|
| 90 | cis-1-(4-(hydroxymethyl)-2-methyl-l-oxo-2, 7-diazaspiro[4.5]decan-7-yl)-5H-pyrido[4,3-b ]indole-4-carboxamide (isomer 1) | 1st | ¹H NMR (400 MHz, CD₃OD) δ 8.52 (s, 1H), 8 .01 (d, *J*=8.0 Hz, 1H), 7.82 (d, *J*=8.3 Hz, 1H), 7 .61 (t, *J*=7.7 Hz, 1H), 7.53 - 7.44 (m, 1H), 4.06 - 3.88 (m, 3H), 3.71 - 3.59 (m, 3H), 3.55 (dd, *J* =6.1, 10.9 Hz, 1H), 3.23 (dd, *J*=3.4, 10.4 Hz, 1 H), 2.78 (s, 3H), 2.46 (br. s., 1H), 2.29 (d, *J*=3*.* 3 Hz, 1H), 2.17 (d, *J*=4.0 Hz, 1H), 2.07 - 1.92 ( m, 2H) |
| 91 | cis-1-(4-(hydroxymethyl)-2-methyl-1-oxo-2, 7-diazaspiro[4.5]decan-7-yl)-5H-pyrido[4,3-b]indole-4-carboxamide (isomer 2) | 2^{nd} | ¹H NMR (400 MHz, CD₃OD) δ 8.52 (s, 1H), 8 .01 (d, *J*=8.0 Hz, 1H), 7.83 (d, *J*=8.3 Hz, 1H), 7 .61 (t, *J*=7.7 Hz, 1H), 7.53 - 7.46 (m, 1H), 4.05 - 3.90 (m, 3H), 3.71 - 3.59 (m, 3H), 3.55 (dd, *J* =6.3, 11.0 Hz, 1H), 3.23 (dd, *J*=3.4, 10.4 Hz, 1 H), 2.79 (s, 3H), 2.46 (br. s., 1H), 2.28 (br. s., 1 H), 2.18 (br. s., 1H), 2.07 - 1.92 (m, 2H) |
| 92 (trans racemic mixture) | trans-1-(4-(hydroxymethyl)-2-methyl-1-oxo-2,7-diazaspiro[4.5]dec an-7-yl)-5H-pyrido[4,3-b]indole-4-carboxamide | 3rd | ¹H NMR (400 MHz, CD₃OD) δ 8.42 (s, 1H), 7 .96 (d, *J*=8.2 Hz, 1H), 7.81 (d, *J*=7.8 Hz, 1H), 7 .57 (t, *J*=7.4 Hz, 1H), 7.50 - 7.41 (m, 1H), 4.14 - 3.97 (m, 3H), 3.86 (dd, *J*=7.2, 10.8 Hz, 1H), 3 .77 - 3.65 (m, 2H), 3.36 - 3.31 (m, 1H), 3.20 - 3 .12 (m, 1H), 2.68 (s, 3H), 2.43 - 2.28 (m, 2H), 2.01 (d, *J*=6.7 Hz, 2H), 1.97 - 1.89 (m, 1H) |

### Biological Activity

The Btk inhibitor compounds of the invention having Formula (I) inhibit the Btk kinase activity.

The term IC50 means the concentration of the test compound that is required for 50% inhibition of its maximum effect *in vitro.*

### Btk enzyme activity Assay Methods

BTK enzymatic activity was determined with the LANCE (Lanthanide Chelate Excite) TR-FRET (Time-resolved fluorescence resonance energy transfer) assay. In this assay, the potency (IC₅₀) of each compound was determined from an eleven point (1:3 serial dilution; final compound concentration range in assay from 1 µM to 0.017 nM) titration curve using the following outlined procedure. To each well of a black non-binding surface Corning 384-well microplate (Corning Catalog #3820), 5 nL of compound (2000 fold dilution in final assay volume of 10 µL) was dispensed, followed by the addition of 7.5 µL of 1x kinase buffer (50 mM Hepes 7.5, 10 mM MgCl₂, 0.01% Brij-35, 1 mM EGTA, 0.05% BSA & 1 mM DTT) containing 5.09 pg/µL (66.67 pM) of BTK enzyme (recombinant protein from baculovirus-transfected *Sf9* cells: full-length BTK, 6HIS-tag cleaved). Following a 60 minute compound & enzyme incubation, each reaction was initiated by the addition of 2.5 µL 1x kinase buffer containing 8 µM biotinylated "A5" peptide (Biotin-EQEDEPEGDYFEWLE-NH2) (SEQ.ID.NO.: 1), and 100 µM ATP. The final reaction in each well of 10 µL consists of 50 pM *h*BTK, 2 µM biotin-A5-peptide, and 25 µM ATP. Phosphorylation reactions were allowed to proceed for 120 minutes. Reactions were immediately quenched by the addition of 20 uL of 1x quench buffer (15 mM EDTA, 25 mM Hepes 7.3, and 0.1% Triton X-100) containing detection reagents (0.626 nM of LANCE-Eu-W1024-anti-phosphoTyrosine antibody, PerkinElmer and 86.8 nM of Streptavidin-conjugated Dylight 650, Dyomics/ThermoFisher Scientific). After 60 minutes incubation with detection reagents, reaction plates were read on a PerkinElmer EnVision plate reader using standard TR-FRET protocol. Briefly, excitation of donor molecules (Eu-chelate:anti-phospho-antibody) with a laser light source at 337 nm produces energy that can be transferred to Dylight-650 acceptor molecules if this donor:acceptor pair is within close proximity. Fluorescence intensity at both 665 nm (acceptor) and 615 nm (donor) are measured and a TR-FRET ratio calculated for each well (acceptor intensity/donor intensity). IC₅₀ values were determined by 4 parameter robust fit of TR-FRET ratio values vs. (Log₁₀) compound concentrations.

The following Table 4 provides specific IC₅₀ values for all the examples. The IC₅₀ values set forth below were determined according to Assay method described above.

**Table 4: BTK binding potency**

| **Example No.** | **IC₅₀ (nM)** | | **Example No.** | **IC₅₀ (nM)** |
|---|---|---|---|---|
| 1 | 149 | | 45 | 204.5 |
| 2 | 12.9 | | 46 | 377.4 |
| 3 | 108.7 | | 47 | 72.6 |
| 4 | 17 | | 48 | 47.6 |
| 5 | 492.3 | | 49 | 87.3 |
| 6 | 853.3 | | 50 | 58.7 |
| 7 | 34.2 | | 51 | 87.9 |
| 8 | 12.6 | | 52 | 0.96 |
| 9 | 384 | | 53 | 1.4 |
| 10 | 19.4 | | 54 | 1.2 |
| 11 | 31.1 | | 55 | 1.3 |
| 12 | 35.0 | | 56 | 3.0 |
| 13 | 163.5 | | 57 | 2.1 |
| 14 | 26.0 | | 58 | 1.4 |
| 15 | 37.4 | | 59 | 1.6 |
| 16 | 98.7 | | 60 | 76.9 |
| 17 | 538.4 | | 61 | 81.8 |
| 18 | 117.3 | | 62 | 59.7 |
| 19 | 22.0 | | 63 | 6.3 |
| 20 | 58.6 | | 64 | 0.63 |
| 21 | 434.8 | | 65 | 16.5 |
| 22 | 21.0 | | 66 | 124.6 |
| 23 | 161.3 | | 67 | 13.9 |
| 24 | 9.0 | | 68 | 3.9 |
| 25 | 535.6 | | 69 | 17.5 |
| 26 | 160.1 | | 70 | 5.4 |
| 27 | 232.1 | | 71 | 0.83 |
| 28 | 579 | | 72 | 0.63 |
| 29 | 270.6 | | 73 | 0.74 |
| 30 | 28.0 | | 74 | 8.9 |
| 31 | 96.8 | | 75 | 9.5 |
| 32 | 244.3 | | 76 | 0.64 |
| 33 | 212.1 | | 77 | 0.55 |
| 34 | 17.2 | | 78 | 59.9 |
| 35 | 55.2 | | 79 | 63 |
| 36 | 9533 | | 80 | 12.7 |
| 37 | 46.43 | | 81 | 5.5 |
| 38 | 420.3 | | 82 | 2.9 |
| 39 | 120 | | 83 | 57.2 |
| 40 | 121.3 | | 84 | 48.8 |
| 41 | 10.7 | | 85 | 447.7 |
| 42 | 1.4 | | 86 | 170.4 |
| 43 | 7.4 | | 87 | 82.8 |
| 44 | 1.6 | | 88 | 77.8 |
| | | | 89 | 668.5 |
| | | | 90 | 115 |
| | | | 91 | 1000 |
| | | | 92 | 64 |

## Claims

1. A compound of Formula (I) 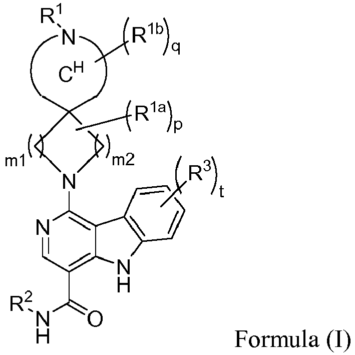 wherein:
ring C^{H} is a 4- to 6-membered saturated heterocyclic ring, wherein said heterocyclic ring optionally contains one additional heteroatom group which is N(H) or O;
the subscripts m1 and m2 are independently 0, 1,2, or 3, with the proviso that 2 ≤ m1+m2 ≤ 5;
R¹ is:
(a.) H;
(b.) C₁-₃alkyl, wherein said C₁-₃alkyl of R¹ is unsubstituted or substituted by -C(O)O-R^{1e}, wherein R^{1e} is H or C₁₋₆alkyl;
(c.) -C(O)O-R^{1c} wherein R^{1c} is C₁₋₆alkyl;
(d.) -C(O)-R^{1d} wherein R^{1d} is C₁₋₃alkyl or phenyl, wherein said phenyl of R^{1d} is unsubstituted or substituted by 1 to 2 substituents selected from C₁₋₆alkyl, halo, or C₁₋₃alkoxy; or
(e.) phenyl, wherein said phenyl of R¹ is unsubstituted or substituted by 1 to 2 substituents selected from C₁₋₆alkyl, halo, or C₁₋₃alkoxy;
R^{1a} is C₁-₃alkyl, C₁-₃fluoroalkyl, C₁-₃alkoxy or fluoro;
R^{1b} is hydroxyl, C₁-₃alkyl, C₁-₃hydroxyalkyl, C₁-₃fluoroalkyl, C₁-₃alkoxy, fluoro, or phenyl; or optionally two R^{1b} moieties when substituted on a common ring carbon atom, together with said carbon atom form a carbonyl;
R² is H or OH;
R³ is
(a.) halo;
(b.) C₁-₃alkyl;
(c.) -C(H)(OH)-CH₂OH;
(d.) -CO2R^{3a}, wherein R^{3a} is H or C₁-₃alkyl;
(e.) ring Cⁱ, wherein Cⁱ is
(i.) phenyl,
(ii.) 3- to 6-membered cycloalkyl;
(iii.) 5- to 6-membered heteroaryl containing 1 or 2 heteroatoms selected from N, O, or S; or
(iv.) 5- to 6-membered saturated heterocyclyl containing 1 to 2 N atoms;
wherein ring Cⁱ is unsubstituted or substituted by 1 to 4 R^{3b}, wherein R^{3b} is selected from C₁-₆alkyl, C₁-₆fluoroalkyl, C₁-₆hydroxyalkyl, C₁₋₃alkoxy, halo, -C(O)O-C₁-₆alkyl, -C(O)OH, -C(O)-C₁₋₆alkyl, -C(O)N(H)-C₁₋₃alkyl, -CH₂C(O)OH, -CH₂C(O)O-C₁₋₃alkyl, -S(O)₂-C₁₋₃alkyl, or -C(H)(OH)-C₁-₃fluoroalkyl;
or optionally two R^{3b} moieties when substituted on a ring common carbon atom, together with said carbon atom form a carbonyl;
the subscript p is 0, 1 or 2;
the subscript q is 0,1, 2, 3, or 4;
the subscript t is 0 or 1; or
a pharmaceutically acceptable salt thereof.

2. The compound of claim 1 or a pharmaceutically acceptable salt thereof, wherein2 ≤ m1+m2 ≤ 4.

3. The compound of claim 1 or a pharmaceutically acceptable salt thereof, wherein ring C^{H} is unsubstituted or substituted pyrrolidine or piperidine.

4. The compound of claim 1 or a pharmaceutically acceptable salt thereof, wherein the group

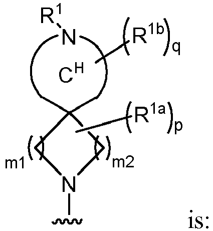

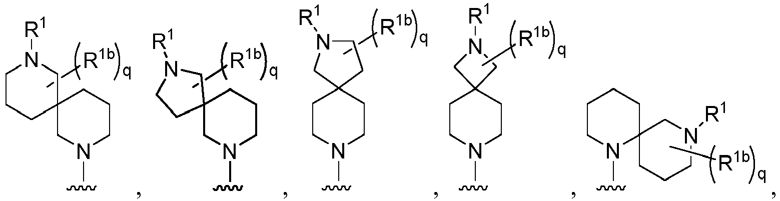

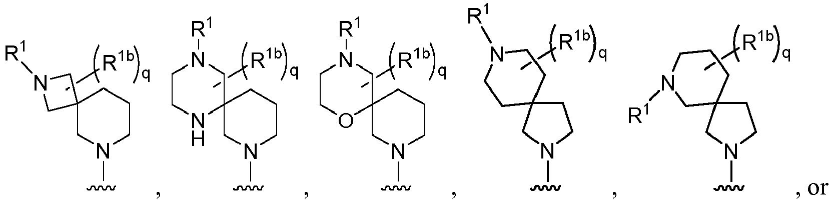

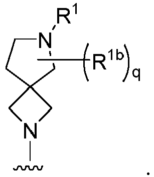

5. The compound of claim 1 or a pharmaceutically acceptable salt thereof, wherein R¹ is H, methyl, -C(O)CH₃, unsubstituted phenyl, or phenyl substituted by 1 to 2 fluoro or
C₁₋₄alkyl.

6. The compound of claim 1 or a pharmaceutically acceptable salt thereof, wherein R^{1b} is hydroxyl, phenyl, -CH₂OH, or optionally two R^{1b} moieties when substituted on a common ring carbon atom, together with said carbon atom form a carbonyl.

7. The compound of claim 1 or a pharmaceutically acceptable salt thereof, wherein R² is H.

8. The compound of claim 1 or a pharmaceutically acceptable salt thereof, wherein the subscript t is 0.

9. The compound of claim 1 or a pharmaceutically acceptable salt thereof, wherein the subscript t is 1.

10. The compound of claim 9 or a pharmaceutically acceptable salt thereof, wherein R³ is substituted on the 7- or 8-positions of the illustrated gamma-carboline ring of Formula (I).

11. The compound of claim 9 or a pharmaceutically acceptable salt thereof, wherein R³ is fluoro, -C(O)OH, -C(O)OCH₃, -C(H)(OH)-CH₂OH, or a group selected from:

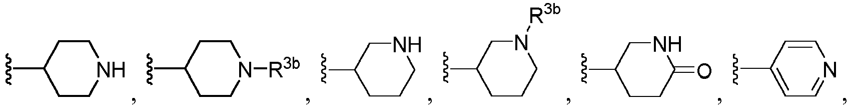

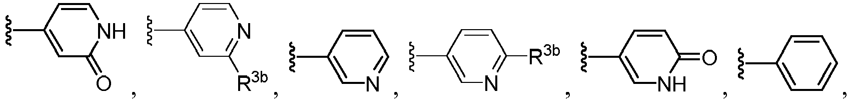

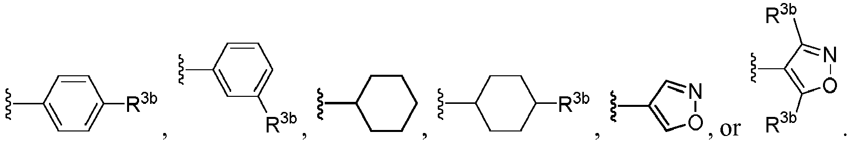

12. The compound of claim 11 or a pharmaceutically acceptable salt thereof, wherein R^{3b} is -CH₃, -CH₂CH₃, -C(H)(CH₃)₂, -OCH₃, -C(O)OH, -C(O)OCH₃,-C(O)OC(CH₃)₃,
-C(O)N(H)CH₃, -CH₂C(O)CH₃, -CH₂C(OH)(CH₃)₂, -C(H)(OH)(CF₃), -S(O)₂CH₃, or-C(O)CH₃.

13. The compound of claim 1 which is:
1-[(6R)-8-(4-fluorophenyl)-7-oxo-2,8-diazaspiro[5.5]undec-2-yl]-5H-pyrido[4,3-b]indole-4-carboxamide;
1-[(6R)-8-(4-fluorophenyl)-7-oxo-2,8-diazaspiro[5.5]undec-2-yl]-5H-pyrido[4,3-b]indole-4-carboxamide;
1-[4-(4-fluorophenyl)-5-oxo-1-oxa-4,8-diazaspiro[5.5]undec-8-yl]-5H-pyrido[4,3-b]indole-4-carboxamide;
1-[8-(4-fluorophenyl)-7-oxo-2,8-diazaspiro[5.5]undec-2-yl]-5H-pyrido[4,3-b]indole-4-carboxamide;
1-{8-[4-(1-methylethyl)phenyl]-7-oxo-2,8-diazaspiro[5.5]undec-2-yl}-5H-pyrido[4,3-b]indole-4-carboxamide;
8-fluoro-1-[(6R)-8-(4-fluorophenyl)-7-oxo-2,8-diazaspiro[5.5]undec-2-yl]-5H-pyrido[4,3-b]indole-4-carboxamide;
8-fluoro-1-[(6S)-8-(4-fluorophenyl)-7-oxo-2,8-diazaspiro[5.5]undec-2-yl]-5H-pyrido[4,3-b]indole-4-carboxamide;
4-carbamoyl-1-[(6R)-8-(4-fluorophenyl)-7-oxo-2,8-diazaspiro[5.5]undec-2-yl]-5H-pyrido[4,3-b]indole-8-carboxylic acid;
N-hydroxy-1-(1-oxo-2,7-diazaspiro[4.5]dec-7-yl)-5H-pyrido[4,3-b]indole-4-carboxamide;
1-(3-oxo-2,8-diazaspiro[4.5]dec-8-yl)-5H-pyrido[4,3-b]indole-4-carboxamide;
1-(1-oxo-2,8-diazaspiro[4.5]dec-8-yl)-5H-pyrido[4,3-b]indole-4-carboxamide;
1-(2,8-diazaspiro[4.5]dec-8-yl)-5H-pyrido[4,3-b]indole-4-carboxamide;
*tert*-butyl 8-(4-carbamoyl-5H-pyrido[4,3-b]indol-1-yl)-2,8-diazaspiro[4.5]decane-2-carboxylate;
1-(2-acetyl-2,8-diazaspiro[4.5]dec-8-yl)-5H-pyrido[4,3-b]indole-4-carboxamide;
1-(2,8-diazaspiro[4.5]dec-2-yl)-5H-pyrido[4,3-b]indole-4-carboxamide;
1-(7-oxa-2-azaspiro[3.5]non-2-yl)-5H-pyrido[4,3-b]indole-4-carboxamide;
*tert*-butyl 7-(4-carbamoyl-5H-pyrido[4,3-b]indol-1-yl)-2,7-diazaspiro[3.5]nonane-2-carboxylate;
1-[(4S,5R)-4-hydroxy-1-oxo-2,7-diazaspiro[4.5]dec-7-yl]-5H-pyrido[4,3-b]indole-4-carboxamide;
1-[(4R,5S)-4-hydroxy-1-oxo-2,7-diazaspiro[4.5]dec-7-yl]-5H-pyrido[4,3-b]indole-4-carboxamide;
1-(2,7-diazaspiro[3.5]non-7-yl)-5H-pyrido[4,3-b]indole-4-carboxamide;
1-(2-acetyl-2,7-diazaspiro[3.5]non-7-yl)-5H-pyrido[4,3-b]indole-4-carboxamide;
*tert*-butyl 2-(4-carbamoyl-5H-pyrido[4,3-b]indol-1-yl)-2,6-diazaspiro[3.4]octane-6-carboxylate;
1-[(4S,5S)-4-hydroxy-1-oxo-2,7-diazaspiro[4.5]dec-7-yl]-5H-pyrido[4,3-b]indole-4-carboxamide;
1-[(4S,5S)-4-hydroxy-1-oxo-2,7-diazaspiro[4.5]dec-7-yl]-5H-pyrido[4,3-b]indole-4-carboxamide;
1-[(4R,5R)-4-hydroxy-1-oxo-2,7-diazaspiro[4.5]dec-7-yl]-5H-pyrido[4,3-b]indole-4-carboxamide;
1-{8-[(4-*tert*-butylphenyl)carbonyl]-1,8-diazaspiro[5.5]undec-1-yl}-5H-pyrido[4,3-b]indole-4-carboxamide;
1-(2,6-diazaspiro[3.4]oct-2-yl)-5H-pyrido[4,3-b]indole-4-carboxamide;
1-(6-acetyl-2,6-diazaspiro[3.4]oct-2-yl)-5H-pyrido[4,3-b]indole-4-carboxamide;
*tert*-butyl 6-(4-carbamoyl-5H-pyrido[4,3-b]indol-1-yl)-2,6-diazaspiro[3.5]nonane-2-carboxylate;
1-(2-acetyl-2,6-diazaspiro[3.5]non-6-yl)-5H-pyrido[4,3-b]indole-4-carboxamide;
1-(7-oxo-2,8-diazaspiro[5.5]undec-2-yl)-5H-pyrido[4,3-b]indole-4-carboxamide;
methyl 3-[8-(4-carbamoyl-5H-pyrido[4,3-b]indol-1-yl)-1-oxo-2,8-diazaspiro[5.5]undec-2-yl]propanoate;
1-[2-(4-fluorophenyl)-1-oxo-2,7-diazaspiro[4.5]dec-7-yl]-5H-pyrido[4,3-b]indole-4-carboxamide;
1-[7-(4-fluorophenyl)-6-oxo-2,7-diazaspiro[4.5]dec-2-yl]-5H-pyrido[4,3-b]indole-4-carboxamide;
1-(1-oxo-2,7-diazaspiro[4.5]dec-7-yl)-5H-pyrido[4,3-b]indole-4-carboxamide;
1-(8-methyl-7-oxo-2,8-diazaspiro[5.5]undec-2-yl)-5H-pyrido[4,3-b]indole-4-carboxamide;
1-[2-(4-*tert*-butylphenyl)-1-oxo-2,7-diazaspiro[4.5]dec-7-yl]-5H-pyrido[4,3-b]indole-4-carboxamide;
1-(8-oxo-2,9-diazaspiro[5.5]undec-2-yl)-5H-pyrido[4,3-b]indole-4-carboxamide;
1-[9-(4-*tert*-butylphenyl)-8-oxo-2,9-diazaspiro[5.5]undec-2-yl]-5H-pyrido[4,3-b]indole-4-carboxamide;
1-[8-(1-methylethyl)-7-oxo-2,8-diazaspiro[5.5]undec-2-yl]-5H-pyrido[4,3-b]indole-4-carboxamide;
1-[(5R)-1-oxo-2,7-diazaspiro[4.5]dec-7-yl]-5H-pyrido[4,3-b]indole-4-carboxamide;
1-[(5S)-1-oxo-2,7-diazaspiro[4.5]dec-7-yl]-5H-pyrido[4,3-b]indole-4-carboxamide;
1-(1-oxo-2,7-diazaspiro[4.5]dec-7-yl)-7-piperidin-4-yl-5H-pyrido[4,3-b]indole-4-carboxamide;
*tert*-butyl 4-[4-carbamoyl-1-(1-oxo-2,7-diazaspiro[4.5]dec-7-yl)-5H-pyrido[4,3-b]indol-7-yl]piperidine-1-carboxylate;
1-(1-oxo-2,7-diazaspiro[4.5]dec-7-yl)-7-piperidin-3-yl-5H-pyrido[4,3-b]indole-4-carboxamide;
1-(1-oxo-2,7-diazaspiro[4.5]dec-7-yl)-8-piperidin-4-yl-5H-pyrido[4,3-b]indole-4-carboxamide;
*tert*-butyl 4-[4-carbamoyl-1-(1-oxo-2,7-diazaspiro[4.5]dec-7-yl)-5H-pyrido[4,3-b]indol-8-yl]piperidine-1-carboxylate;
8-[1-(methylcarbamoyl)piperidin-4-yl]-1-(1-oxo-2,7-diazaspiro[4.5]dec-7-yl)-5H-pyrido[4,3-b]indole-4-carboxamide;
{4-[4-carbamoyl-1-(1-oxo-2,7-diazaspiro[4.5]dec-7-yl)-5H-pyrido[4,3-b]indol-8-yl]piperidin-1-yl}acetic acid;
8-(1-ethylpiperidin-4-yl)-1-(1-oxo-2,7-diazaspiro[4.5]dec-7-yl)-5H-pyrido[4,3-b]indole-4-carboxamide;
8-[1-(1-methylethyl)piperidin-4-yl]-1-(1-oxo-2,7-diazaspiro[4.5]dec-7-yl)-5H-pyrido[4,3-b]indole-4-carboxamide;
methyl 4-[4-carbamoyl-1-(1-oxo-2,7-diazaspiro[4.5]dec-7-yl)-5H-pyrido[4,3-b]indol-8-yl]piperidine-1-carboxylate;
7-[1-(1-methylethyl)piperidin-4-yl]-1-(1-oxo-2,7-diazaspiro[4.5]dec-7-yl)-5H-pyrido[4,3-b]indole-4-carboxamide;
7-[1-(methylcarbamoyl)piperidin-4-yl]-1-(1-oxo-2,7-diazaspiro[4.5]dec-7-yl)-5H-pyrido[4,3-b]indole-4-carboxamide;
7-[1-(2-hydroxy-2-methylpropyl)piperidin-4-yl]-1-(1-oxo-2,7-diazaspiro[4.5]dec-7-yl)-5H-pyrido[4,3-b]indole-4-carboxamide;
7-(1-methylpiperidin-4-yl)-1-(1-oxo-2,7-diazaspiro[4.5]dec-7-yl)-5H-pyrido[4,3-b]indole-4-carboxamide;
methyl 4-[4-carbamoyl-1-(1-oxo-2,7-diazaspiro[4.5]dec-7-yl)-5H-pyrido[4,3-b]indol-7-yl]piperidine-1-carboxylate;
7-[1-(methylsulfonyl)piperidin-4-yl]-1-(1-oxo-2,7-diazaspiro[4.5]dec-7-yl)-5H-pyrido[4,3-b]indole-4-carboxamide;
7-(1-acetylpiperidin-4-yl)-1-(1-oxo-2,7-diazaspiro[4.5]dec-7-yl)-5H-pyrido[4,3-b]indole-4-carboxamide;
{4-[4-carbamoyl-1-(1-oxo-2,7-diazaspiro[4.5]dec-7-yl)-5H-pyrido[4,3-b]indol-7-yl]piperidin-1-yl}acetic acid;
8-(1-methylpiperidin-4-yl)-1-(1-oxo-2,7-diazaspiro[4.5]dec-7-yl)-5H-pyrido[4,3-b]indole-4-carboxamide;
8-(1-acetylpiperidin-4-yl)-1-(1-oxo-2,7-diazaspiro[4.5]dec-7-yl)-5H-pyrido[4,3-b]indole-4-carboxamide;
8-[1-(2-hydroxy-2-methylpropyl)piperidin-4-yl]-1-(1-oxo-2,7-diazaspiro[4.5]dec-7-yl)-5H-pyrido[4,3-b]indole-4-carboxamide;
1-[(5S)-1-oxo-2,7-diazaspiro[4.5]dec-7-yl]-7-piperidin-4-yl-5H-pyrido[4,3-b]indole-4-carboxamide;
1-[(5R)-1-oxo-2,7-diazaspiro[4.5]dec-7-yl]-7-piperidin-4-yl-5H-pyrido[4,3-b]indole-4-carboxamide;
8-(1,2-dihydroxyethyl)-1-(1-oxo-2,7-diazaspiro[4.5]dec-7-yl)-5H-pyrido[4,3-b]indole-4-carboxamide;
8-[1-(methylsulfonyl)piperidin-4-yl]-1-(1-oxo-2,7-diazaspiro[4.5]dec-7-yl)-5H-pyrido[4,3-b]indole-4-carboxamide;
7-(6-methoxypyridin-3-yl)-1-[(5R)-1-oxo-2,7-diazaspiro[4.5]dec-7-yl]-5H-pyrido[4,3-b]indole-4-carboxamide;
1-[(5R)-1-oxo-2,7-diazaspiro[4.5]dec-7-yl]-7-(6-oxo-1,6-dihydropyridin-3-yl)-5H-pyrido[4,3-b]indole-4-carboxamide;
7-(2-methoxypyridin-4-yl)-1-[(5R)-1-oxo-2,7-diazaspiro[4.5]dec-7-yl]-5H-pyrido[4,3-b]indole-4-carboxamide;
1-[(5R)-1-oxo-2,7-diazaspiro[4.5]dec-7-yl]-7-(2-oxo-1,2-dihydropyridin-4-yl)-5H-pyrido[4,3-b]indole-4-carboxamide;
7-[1-(1-methylethyl)piperidin-4-yl]-1-[(5R)-1-oxo-2,7-diazaspiro[4.5]dec-7-yl]-5H-pyrido[4,3-b]indole-4-carboxamide;
7-[1-(2-hydroxy-2-methylpropyl)piperidin-4-yl]-1-[(5R)-R)-1-oxo-2,7-diazaspiro[4.5]dec-7-yl]-5H-pyrido[4,3-b]indole-4-carboxamide;
1-[(5R)-1-oxo-2,7-diazaspiro[4.5]dec-7-yl]-7-(6-oxopiperidin-3-yl)-5H-pyrido[4,3-b]indole-4-carboxamide;
7-(6-methoxypyridin-3-yl)-1-[(5R)-2-methyl-1-oxo-2,7-diazaspiro[4.5]dec-7-yl]-5H-pyrido[4,3-b]indole-4-carboxamide;
7-(2-methoxypyridin-4-yl)-1-[(5R)-2-methyl-1-oxo-2,7-diazaspiro[4.5]dec-7-yl]-5H-pyrido[4,3-b]indole-4-carboxamide;
7-[1-(1-methylethyl)piperidin-4-yl]-1-[(5 R)-2-methyl-1 -oxo-2,7-diazaspiro[4.5]dec-7-yl]-5H-pyrido[4,3-b]indole-4-carboxamide;
7-[1-(2-hydroxy-2-methylpropyl)piperidin-4-yl]-1-[(5R)-2-methyl-1-oxo-2,7-diazaspiro[4.5]dec-7-yl]-5H-pyrido[4,3-b]indole-4-carboxamide;
1-[(5R)-2-methyl-1-oxo-2,7-diazaspiro[4.5]dec-7-yl]-7-[4-(2,2,2-trifluoro-1-hydroxyethyl)phenyl]-5H-pyrido[4,3-b]indole-4-carboxamide;
1-[(5R)-2-methyl-1-oxo-2,7-diazaspiro[4.5]dec-7-yl]-7-[3-(2,2,2-trifluoro-1-hydroxyethyl)phenyl]-5H-pyrido[4,3-b]indole-4-carboxamide;
7-(3,5-dimethylisoxazol-4-yl)-1-[(5R)-2-methyl-1-oxo-2,7-diazaspiro[4.5]dec-7-yl]-5H-pyrido[4,3-b]indole-4-carboxamide;
methyl(4-{4-carbamoyl-1-[(5R)-2-methyl-1-oxo-2,7-diazaspiro[4.5]dec-7-yl]-5H-pyrido[4,3-b]indol-7-yl}cyclohexyl)acetate;
7-[4-(2-hydroxy-2-methylpropyl)cyclohexyl]-1-[(5R)-2-methyl-1-oxo-2,7-diazaspiro[4.5]dec-7-yl]-5H-pyrido[4,3-b]indole-4-carboxamide;
methyl 4-carbamoyl-1-(1-oxo-2,7-diazaspiro[4.5]dec-7-yl)-5H-pyrido[4,3-b]indole-7-carboxylate;
4-carbamoyl-1-(1-oxo-2,7-diazaspiro[4.5]dec-7-yl)-5H-pyrido[4,3-b]indole-8-carboxylic acid;
4-carbamoyl-1-(1-oxo-2,7-diazaspiro[4.5]dec-7-yl)-5H-pyrido[4,3-b]indole-7-carboxylic acid;
1-(4-hydroxy-1-oxo-2,7-diazaspiro[4.5]dec-7-yl)-5H-pyrido[4,3-b]indole-4-carboxamide;
1-(5-oxo-1,4,8-triazaspiro[5.5]undec-8-yl)-5H-pyrido[4,3-b]indole-4-carboxamide;
1-[(4R,5R)-1-oxo-4-phenyl-2,7-diazaspiro[4.5]dec-7-yl]-5H-pyrido[4,3-b]indole-4-carboxamide;
1-[(4R,5S)-1-oxo-4-phenyl-2,7-diazaspiro[4.5]dec-7-yl]-5H-pyrido[4,3-b]indole-4-carboxamide;
1-[(4S,5S)-4-(hydroxymethyl)-2-methyl-1-oxo-2,7-diazaspiro[4.5]dec-7-yl]-5H-pyrido[4,3-b]indole-4-carboxamide;
1-[(4R,5S)-4-(hydroxymethyl)-2-methyl-1-oxo-2,7-diazaspiro[4.5]dec-7-yl]-5H-pyrido[4,3-b]indole-4-carboxamide; or
a pharmaceutically acceptable salt thereof.

14. A pharmaceutical composition comprising the compound of claim 1 or a pharmaceutically acceptable salt thereof and a pharmaceutically acceptable carrier.

15. The pharmaceutical composition of claim 14, which further comprises at least one additional therapeutically active agent.

16. The compound of claim 1 or a pharmaceutically acceptable salt thereof for use in therapy.

17. The compound of claim 1 or a pharmaceutically acceptable salt thereof for use in treating autoimmune and inflammatory diseases.

## Patentansprüche

1. Eine Verbindung der Formel (I) 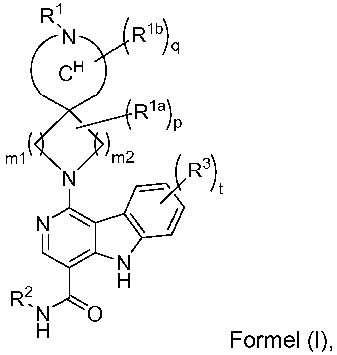 wobei:
Ring C^{H} ein 4- bis 6-gliedriger gesättigter heterocyclischer Ring ist, wobei der heterocyclische Ring gegebenenfalls eine zusätzliche Heteroatomgruppe enthält, die N(H) oder O ist,
die Indizes m1 und m2 unabhängig 0, 1, 2 oder 3 sind, mit der Maßgabe, dass 2 ≤ m1 + m2 ≤ 5,
R¹ ist:
(a.) H,
(b.) C₁₋₃-Alkyl, wobei das C₁₋₃-Alkyl von R¹ unsubstituiert oder substituiert ist durch -C(O)O-R^{1e}, wobei R^{1e} H oder C₁₋₆-Alkyl ist,
(c.) -C(O)O-R^{1c}, wobei R^{1c} C₁₋₆-Alkyl ist,
(d.) -C(O)-R^{1d}, wobei R^{1d} C₁₋₃-Alkyl oder Phenyl ist, wobei das Phenyl von R^{1d} unsubstituiert oder substituiert ist durch 1 bis 2 Substituenten, ausgewählt aus C₁₋₆-Alkyl, Halogen oder C₁₋₃-Alkoxy, oder
(e.) Phenyl, wobei das Phenyl von R¹ unsubstituiert oder substituiert ist durch 1 bis 2 Substituenten, ausgewählt aus C₁₋₆-Alkyl, Halogen oder C₁₋₃-Alkoxy,
R^{1a} C₁₋₃-Alkyl, C₁₋₃-Fluoralkyl, C₁₋₃-Alkoxy oder Fluor ist,
R^{1b} Hydroxyl, C₁₋₃-Alkyl, C₁₋₃-Hydroxyalkyl, C₁₋₃-Fluoralkyl, C₁₋₃-Alkoxy, Fluor oder Phenyl ist, oder gegebenenfalls zwei R^{1b}-Reste, wenn sie sich als Substituenten an einem gemeinsamen Ring-Kohlenstoffatom befinden, zusammen mit dem Kohlenstoffatom ein Carbonyl bilden,
R² H oder OH ist,
R³ ist
(a.) Halogen,
(b.) C₁₋₃-Alkyl,
(c.) -C(H)(OH)-CH₂OH,
(d.) -CO₂R^{3a}, wobei R^{3a} H oder C₁₋₃-Alkyl ist,
(e.) Ring Cⁱ, wobei Cⁱ ist
(i.) Phenyl,
(ii.) 3- bis 6-gliedriges Cycloalkyl,
(iii.) 5- bis 6-gliedriges Heteroaryl, das 1 oder 2 Heteroatome, ausgewählt aus N, O oder S, enthält, oder
(iv.) 5- bis 6-gliedriges gesättigtes Heterocyclyl, das 1 bis 2 N-Atome enthält,
wobei Ring Cⁱ unsubstituiert oder substituiert ist durch 1 bis 4 R^{3b}, wobei R^{3b} ausgewählt ist aus C₁₋₆-Alkyl, C₁₋₆-Fluoralkyl, C₁₋₆-Hydroxyalkyl, C₁₋₃-Alkoxy, Halogen, -C(O)O-C₁₋₆-Alkyl, -C(O)OH, -C(O)-C₁₋₆-Alkyl, -C(O)N(H)-C₁₋₃-Alkyl, -CH₂C(O)OH, -CH₂C(O)O-C₁₋₃-Alkyl,-S(O)₂-C₁₋₃-Alkyl oder -C(H)(OH)-C₁₋₃-Fluoralkyl,
oder gegebenenfalls zwei R^{3b}-Reste, wenn sie sich als Substituenten an einem gemeinsamen Ring-Kohlenstoffatom befinden, zusammen mit dem Kohlenstoffatom ein Carbonyl bilden,
der Index p 0, 1 oder 2 ist,
der Index q 0, 1, 2, 3 oder 4 ist,
der Index t 0 oder 1 ist, oder
ein pharmazeutisch annehmbares Salz davon.

2. Die Verbindung nach Anspruch 1 oder ein pharmazeutisch annehmbares Salz davon, wobei 2 ≤ m1 + m2 ≤ 4.

3. Die Verbindung nach Anspruch 1 oder ein pharmazeutisch annehmbares Salz davon, wobei Ring C^{H} unsubstituiertes oder substituiertes Pyrrolidin oder Piperidin ist.

4. Die Verbindung nach Anspruch 1 oder ein pharmazeutisch annehmbares Salz davon, wobei die Gruppe 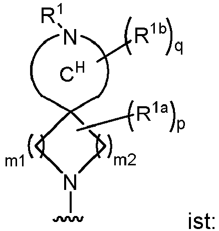 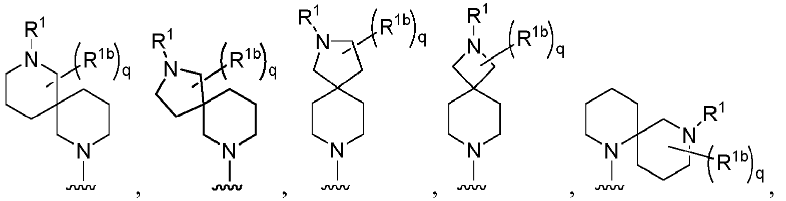 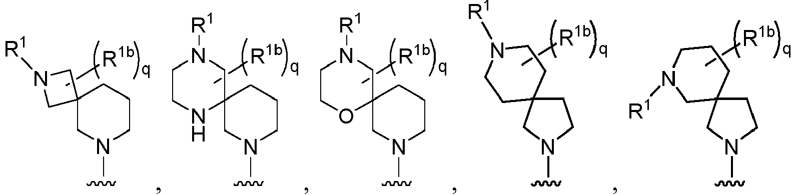 oder

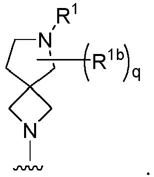

5. Die Verbindung nach Anspruch 1 oder ein pharmazeutisch annehmbares Salz davon, wobei R¹ H, Methyl, -C(O)CH₃, unsubstituiertes Phenyl oder Phenyl, das durch 1 bis 2 Fluor oder C₁₋₄-Alkyl substituiert ist, ist.

6. Die Verbindung nach Anspruch 1 oder ein pharmazeutisch annehmbares Salz davon, wobei R^{1b} Hydroxyl, Phenyl, -CH₂OH ist, oder gegebenenfalls zwei R^{1b}-Reste, wenn sie sich als Substituenten an einem gemeinsamen Ring-Kohlenstoffatom befinden, zusammen mit dem Kohlenstoffatom ein Carbonyl bilden.

7. Die Verbindung nach Anspruch 1 oder ein pharmazeutisch annehmbares Salz davon, wobei R² H ist.

8. Die Verbindung nach Anspruch 1 oder ein pharmazeutisch annehmbares Salz davon, wobei der Index t 0 ist.

9. Die Verbindung nach Anspruch 1 oder ein pharmazeutisch annehmbares Salz davon, wobei der Index t 1 ist.

10. Die Verbindung nach Anspruch 9 oder ein pharmazeutisch annehmbares Salz davon, wobei R³ sich als Substituent an den 7- oder 8-Positionen des gezeigten gamma-Carbolin-Rings der Formel (I) befindet.

11. Die Verbindung nach Anspruch 9 oder ein pharmazeutisch annehmbares Salz davon, wobei R³ Fluor, -C(O)OH, -C(O)OCH₃, -C(H)(OH)-CH₂OH oder eine Gruppe, ausgewählt aus 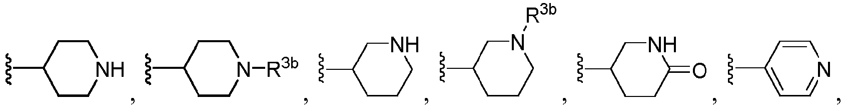 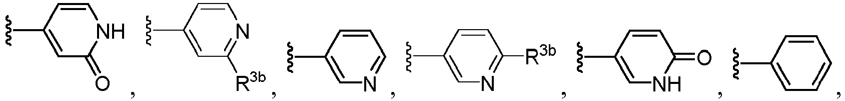 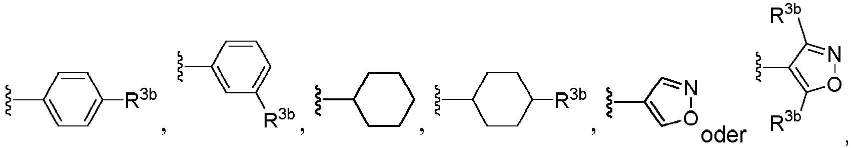 ist.

12. Die Verbindung nach Anspruch 11 oder ein pharmazeutisch annehmbares Salz davon, wobei R^{3b} -CH₃, -CH₂CH₃, -C(H)(CH₃)₂, -OCH₃, -C(O)OH, -C(O)OCH₃, -C(O)OC(CH₃)₃,-C(O)N(H)CH₃, -CH₂C(O)CH₃, -CH₂C(OH)(CH₃)₂, -C(H)(OH)(CF₃), -S(O)₂CH₃ oder -C(O)CH₃ ist.

13. Die Verbindung nach Anspruch 1, die ist:
1-[(6R)-8-(4-Fluorphenyl)-7-oxo-2,8-diazaspiro[5.5]undec-2-yl]-5H-pyrido[4,3-b]indol-4-carboxamid,
1-[(6R)-8-(4-Fluorphenyl)-7-oxo-2,8-diazaspiro[5.5]undec-2-yl]-5H-pyrido[4,3-b]indol-4-carboxamid,
1-[4-(4-Fluorphenyl)-5-oxo-1-oxa-4,8-diazaspiro[5.5]undec-8-yl]-5H-pyrido[4,3-b]indol-4-carboxamid,
1-[8-(4-Fluorphenyl)-7-oxo-2,8-diazaspiro[5.5]undec-2-yl]-5H-pyrido[4,3-b]indol-4-carboxamid,
1-{8-[4-(1-Methylethyl)phenyl]-7-oxo-2,8-diazaspiro[5.5]undec-2-yl}-5H-pyrido[4,3-b]indol-4-carboxamid,
8-Fluor-1-[(6R)-8-(4-fluorphenyl)-7-oxo-2,8-diazaspiro[5.5]undec-2-yl]-5H-pyrido[4,3-b]indol-4-carboxamid,
8-Fluor-1-[(6S)-8-(4-fluorphenyl)-7-oxo-2,8-diazaspiro[5.5]undec-2-yl]-5H-pyrido[4,3-b]indol-4-carboxamid,
4-Carbamoyl-1-[(6R)-8-(4-fluorphenyl)-7-oxo-2,8-diazaspiro[5.5]undec-2-yl]-5H-pyrido[4,3-b]indol-8-carbonsäure,
N-Hydroxy-1-(1-oxo-2,7-diazaspiro[4.5]dec-7-yl)-5H-pyrido[4,3-b]indol-4-carboxamid,
1-(3-Oxo-2,8-diazaspiro[4.5]dec-8-yl)-5H-pyrido[4,3-b]indol-4-carboxamid,
1-(1-Oxo-2,8-diazaspiro[4.5]dec-8-yl)-5H-pyrido[4,3-b]indol-4-carboxamid,
1-(2,8-Diazaspiro[4.5]dec-8-yl)-5H-pyrido[4,3-b]indol-4-carboxamid,
*terf*-Butyl-8-(4-carbamoyl-5H-pyrido[4,3-b]indol-1-yl)-2,8-diazaspiro[4.5]decan-2-carboxylat,
1-(2-Acetyl-2,8-diazaspiro[4.5]dec-8-yl)-5H-pyrido[4,3-b]indol-4-carboxamid,
1-(2,8-Diazaspiro[4.5]dec-2-yl)-5H-pyrido[4,3-b]indol-4-carboxamid,
1-(7-Oxo-2-azaspiro[3.5]non-2-yl)-5H-pyrido[4,3-b]indol-4-carboxamid,
*tert*-Butyl-7-(4-carbamoyl-5H-pyrido[4,3-b]indol-1-yl)-2,7-diazaspiro[3.5]nonan-2-carboxylat,
1-[(4S,5R)-4-Hydroxy-1-oxo-2,7-diazaspiro[4.5]dec-7-yl]-5H-pyrido[4,3-b]indol-4-carboxamid,
1-[(4R,5S)-4-Hydroxy-1-oxo-2,7-diazaspiro[4.5]dec-7-yl]-5H-pyrido[4,3-b]indol-4-carboxamid,
1-(2,7-Diazaspiro[3.5]non-7-yl)-5H-pyrido[4,3-b]indol-4-carboxamid,
1-(2-Acetyl-2,7-diazaspiro[3.5]non-7-yl)-5H-pyrido[4,3-b]indol-4-carboxamid,
*tert*-Butyl-2-(4-carbamoyl-5H-pyrido[4,3-b]indol-1-yl)-2,6-diazaspiro[3.4]octan-6-carboxylat,
1-[(4S,5S)-4-Hydroxy-1-oxo-2,7-diazaspiro[4.5]dec-7-yl]-5H-pyrido[4,3-b]indol-4-carboxamid,
1-[(4S,5S)-4-Hydroxy-1-oxo-2,7-diazaspiro[4.5]dec-7-yl]-5H-pyrido[4,3-b]indol-4-carboxamid,
1-[(4R,5R)-4-Hydroxy-1 -oxo-2,7-diazaspiro[4.5]dec-7-yl]-5H-pyrido[4,3-b]indol-4-carboxamid,
1-{8-[(4-*tert*-Butylphenyl)carbonyl]-1,8-diazaspiro[5.5]undec-1-yl}-5H-pyrido[4,3-b]indol-4-carboxamid,
1-(2,6-Diazaspiro[3.4]oct-2-yl)-5H-pyrido[4,3-b]indol-4-carboxamid,
1-(6-Acetyl-2,6-diazaspiro[3.4]oct-2-yl)-5H-pyrido[4,3-b]indol-4-carboxamid,
*tert*-Butyl-6-(4-carbamoyl-5H-pyrido[4,3-b]indol-1-yl)-2,6-diazaspiro[3.5]nonan-2-carboxylat,
1-(2-Acetyl-2,6-diazaspiro[3.5]non-6-yl)-5H-pyrido[4,3-b]indol-4-carboxamid,
1-(7-Oxo-2,8-diazaspiro[5.5]undec-2-yl)-5H-pyrido[4,3-b]indol-4-carboxamid,
Methyl-3-[8-(4-carbamoyl-5H-pyrido[4,3-b]indol-1-yl)-1-oxo-2,8-diazaspiro[5.5]undec-2-yl]propanoat,
1-[2-(4-Fluorphenyl)-1-oxo-2,7-diazaspiro[4.5]dec-7-yl]-5H-pyrido[4,3-b]indol-4-carboxamid,
1-[7-(4-Fluorphenyl)-6-oxo-2,7-diazaspiro[4.5]dec-2-yl]-5H-pyrido[4,3-b]indol-4-carboxamid,
1-(1-Oxo-2,7-diazaspiro[4.5]dec-7-yl)-5H-pyrido[4,3-b]indol-4-carboxamid,
1-(8-Methyl-7-oxo-2,8-diazaspiro[5.5]undec-2-yl)-5H-pyrido[4,3-b]indol-4-carboxamid,
1-[2-(4-*tert*-Butylphenyl)-1-oxo-2,7-diazaspiro[4.5]dec-7-yl]-5H-pyrido[4,3-b]indol-4-carboxamid,
1-(8-Oxo-2,9-diazaspiro[5.5]undec-2-yl)-5H-pyrido[4,3-b]indol-4-carboxamid,
1-[9-(4-*tert*-Butylphenyl)-8-oxo-2,9-diazaspiro[5.5]undec-2-yl]-5H-pyrido[4,3-b]indol-4-carboxamid,
1-[8-(1-Methylethyl)-7-oxo-2,8-diazaspiro[5.5]undec-2-yl]-5H-pyrido[4,3-b]indol-4-carboxamid,
1-[(5R)-1-Oxo-2,7-diazaspiro[4,5]dec-7-yl]-5H-pyrido[4,3-b]indol-4-carboxamid,
1-[(5S)-1-Oxo-2,7-diazaspiro[4.5]dec-7-yl]-5H-pyrido[4,3-b]indol-4-carboxamid,
1-(1-oxo-2,7-diazaspiro[4.5]dec-7-yl)-7-piperidin-4-yl-5H-pyrido[4,3-b]indol-4-carboxamid,
*tert*-Butyl-4-[4-carbamoyl-1-(1-oxo-2,7-diazaspiro[4.5]dec-7-yl)-5H-pyrido[4,3-b]indol-7-yl]piperidin-1-carboxylat,
1-(1-Oxo-2,7-diazaspiro[4.5]dec-7-yl)-7-piperidin-3-yl-5H-pyrido[4,3-b]indol-4-carboxamid,
1-(1-Oxo-2,7-diazaspiro[4.5]dec-7-yl)-8-piperidin-4-yl-5H-pyrido[4,3-b]indol-4-carboxamid,
*tert*-Butyl-4-[4-carbamoyl-1-(1-oxo-2,7-diazaspiro[4.5]dec-7-yl)-5H-pyrido[4,3-b]indol-8-yl]piperidin-1-carboxalat,
8-[1-(Methylcarbamoyl)piperidin-4-yl]-1-(1-oxo-2,7-diazaspiro[4.5]dec-7-yl)-5H-pyrido[4,3-b]indol-4-carboxamid,
{4-[4-Carbamoyl-1-(1-oxo-2,7-diazaspiro[4.5]dec-7-yl)-5H-pyrido[4,3-b]indol-8-yl]piperidin-1-yl}essigsäure,
8-(1-Ethylpiperidin-4-yl)-1-(1-oxo-2,7-diazaspiro[4.5]dec-7-yl)-5H-pyrido[4,3-b]indol-4-carboxamid,
8-[1-(1-Methylethyl)piperidin-4-yl]-1-(1-oxo-2,7-diazaspiro[4.5]dec-7-yl)-5H-pyrido[4,3-b]indol-4-carboxamid,
Methyl-4-[4-carbamoyl-1-(1-oxo-2,7-diazaspiro[4.5]dec-7-yl)-5H-pyrido[4,3-b]indol-8-yl]piperidin-1-carboxylat,
7-[1-(1-Methylethyl)piperidin-4-yl]-1-(1-oxo-2,7-diazaspiro[4.5]dec-7-yl)-5H-pyrido[4,3-b]indol-4-carboxamid,
7-[1-(Methylcarbamoyl)piperidin-4-yl]-1-(1-oxo-2,7-diazaspiro[4.5]dec-7-yl)-5H-pyrido[4,3-b]indol-4-carboxamid,
7-[1-(2-Hydroxy-2-methylpropyl)piperidin-4-yl]-1-(1-oxo-2,7-diazaspiro[4.5]dec-7-yl)-5H-pyrido[4,3-b]indol-4-carboxamid,
7-(1-Methylpiperidin-4-yl)-1-(1-oxo-2,7-diazaspiro[4.5]dec-7-yl)-5H-pyrido[4,3-b]indol-4-carboxamid,
Methyl-4-[4-carbamoyl-1-(1-oxo-2,7-diazaspiro[4.5]dec-7-yl)-5H-pyrido[4,3-b]indol-7-yl]piperidin-1-carboxylat,
7-[1-(Methylsulfonyl)piperidin-4-yl]-1-(1-oxo-2,7-diazaspiro[4.5]dec-7-yl)-5H-pyrido[4,3-b]indol-4-carboxamid,
7-(1-Acetylpiperidin-4-yl)-1-(1-oxo-2,7-diazaspiro[4.5]dec-7-yl)-5H-pyrido[4,3-b]indol-4-carboxamid,
{4-[4-Carbamoyl-1-(1-oxo-2,7-diazaspiro[4.5]dec-7-yl)-5H-pyrido[4,3-b]indol-7-yl]piperidin-1-yl}essigsäure,
8-(1-Methylpiperidin-4-yl)-1-(1-oxo-2,7-diazaspiro[4.5]dec-7-yl)-5H-pyrido[4,3-b]indol-4-carboxamid,
8-(1-Acetylpiperidin-4-yl)-1-(1-oxo-2,7-diazaspiro[4.5]dec-7-yl)-5H-pyrido[4,3-b]indol-4-carboxamid,
8-[1-(2-Hydroxy-2-methylpropyl)piperidin-4-yl]-1-(1-oxo-2,7-diazaspiro[4.5]dec-7-yl)-5H-pyrido[4,3-b]indol-4-carboxamid,
1-[(5S)-1-Oxo-2,7-diazaspiro[4,5-dec-7-yl]-7-piperidin-4-yl-5H-pyrido[4,3-b]indol-4-carboxamid,
1-[(5R)-1-Oxo-2,7-diazaspiro[4.5]dec-7-yl]-7-piperidin-4-yl-5H-pyrido[4,3-b]indol-4-carboxamid,
8-(1,2-Dihydroxyethyl)-1-(1-oxo-2,7-diazaspiro[4.5]dec-7-yl)-5H-pyrido[4,3-b]indol-4-carboxamid,
8-[1-(Methylsulfonyl)piperidin-4-yl]-1-(1-oxo-2,7-diazaspiro[4.5]dec-7-yl)-5H-pyrido[4,3-b]indol-4-carboxamid,
7-(6-Methoxypyridin-3-yl)-1-[(5R)-1-oxo-2,7-diazaspiro[4.5]dec-7-yl]-5H-pyrido[4,3-b]indol-4-carboxamid,
1-[(5R)-1-Oxo-2,7-diazaspiro[4.5]dec-7-yl]-7-(6-oxo-1,6-dihydropyridin-3-yl)-5H-pyrido[4,3-b]indol-4-carboxamid,
7-(2-Methoxypyridin-4-yl)-1-[(5R)-1-oxo-2,7-diazaspiro[4.5]dec-7-yl]-5H-pyrido[4,3-b]indol-4-carboxamid,
1-[(5R)-1-Oxo-2,7-diazaspiro[4.5]dec-7-yl]-7-(2-oxo-1,2-dihydropyridin-4-yl)-5H-pyrido[4,3-b]indol-4-carboxamid,
7-[1-(1-Methylethyl)piperidin-4-yl]-1-[(5R)-1-oxo-2,7-diazaspiro[4.5]dec-7-yl]-5H-pyrido[4,3-b]indol-4-carboxamid,
7-[1-(2-Hydroxy-2-methylpropyl)piperidin-4-yl]-1-[(5R)-1-oxo-2,7-diazaspiro[4.5]dec-7-yl]-5H-pyrido[4,3-b]indol-4-carboxamid,
1-[(5R)-1-Oxo-2,7-diazaspiro[4.5]dec-7-yl]-7-(6-oxopiperidin-3-yl)-5H-pyrido[4,3-b]indol-4-carboxamid,
7-(6-Methoxypyridin-3-yl)-1-[(5R)-2-methyl-1-oxo-2,7-diazaspiro[4.5]dec-7-yl]-5H-pyrido[4,3-b]indol-4-carboxamid,
7-(2-Methoxypyridin-4-yl)-1-[(5R)-2-methyl-1-oxo-2,7-diazaspiro[4.5]dec-7-yl]-5H-pyrido[4,3-b]indol-4-carboxamid,
7-[1-(1-Methylethyl)piperidin-4-yl]-1-[(5R)-2-Methyl-1-oxo-2,7-diazaspiro[4.5]dec-7-yl]-5H-pyrido[4,3-b]indol-4-carboxamid,
7-[1-(2-Hydroxy-2-methylpropyl)piperidin-4-yl]-1-[(5R)-2-methyl-1-oxo-2,7-diazaspiro[4.5]dec-7-yl]-5H-pyrido[4,3-b]indol-4-carboxamid,
1-[(5R)-2-Methyl-1-oxo-2,7-diazaspiro[4.5]dec-7-yl]-7-[4-(2,2,2-trifluor-1-hydroxyethyl)phenyl]-5H-pyrido[4,3-b]indol-4-carboxamid,
1-[(5R)-2-Methyl-1-oxo-2,7-diazaspiro[4.5]dec-7-yl]-7-[3-(2,2,2-trifluor-1-hydroxyethyl)phenyl]-5H-pyrido[4,3-b]indol-4-carboxamid,
7-(3,5-Dimethylisoxazol-4-yl)-1-[(5R)-2-methyl-1-oxo-2,7-diawzaspiro[4.5]dec-7-yl]-5H-pyrido[4,3-b]indol-4-carboxamid,
Methyl-(4-{4-carbamoyl-1-[(5R)-2-methyl-1-oxo-2,7-diazaspiro[4.5]dec-7-yl]-5H-pyrido[4,3-b]indol-7-yl}cyclohexyl)acetat,
7-[4-(2-Hydroxy-2-methylpropyl)cyclohexyl]-1-[(5R)-2-methyl-1-oxo-2,7-diazaspiro[4.5]dec-7-yl]-5H-pyrido[4,3-b]indol-4-carboxamid,
Methyl-4-carbamoyl-1-(1-oxo-2,7-diazaspiro[4.5]dec-7-yl)-5H-pyrido[4,3-b]indol-7-carboxylat,
4-Carbamoyl-1-(1-oxo-2,7-diazaspiro[4.5]dec-7-yl)-5H-pyrido[4,3-b]indol-8-carbonsäure,
4-Carbamoyl-1-(1-oxo-2,7-diazaspiro[4.5]dec-7-yl)-5H-pyrido[4,3-b]indol-7-carbonsäure,
1-(4-Hydroxy-1 -oxo-2,7-diazaspiro[4.5]dec-7-yl)-5H-pyrido[4,3-b]indol-4-carboxamid,
1-(5-Oxo-1,4,8-triazaspiro[5.5]undec-8-yl)-5H-pyrido[4,3-b]indol-4-carboxamid,
1-[(4R,5R)-1-Oxo-4-phenyl-2,7-diazaspiro[4.5]dec-7-yl]-5H-pyrido[4,3-b]indol-4-carboxamid,
1-[(4R,5S)-1-Oxo-4-phenyl-2,7-diazaspiro[4.5]dec-7-yl]-5H-pyrido[4,3-b]indol-4-carboxamid,
1-[(4S,5S)-4-(Hydroxymethyl)-2-methyl-1-oxo-2,7-diazaspiro[4.5]dec-7-yl]-5H-pyrido[4,3-b]indol-4-carboxamid,
1-[(4R,5S)-4-(Hydroxymethyl)-2-methyl-1-oxo-2,7-diazaspiro[4.5]dec-7-yl]-5H-pyrido[4,3-b]indol-4-carboxamid,
oder ein pharmazeutisch annehmbares Salz davon.

14. Eine pharmazeutische Zusammensetzung, die die Verbindung nach Anspruch 1 oder ein pharmazeutisch annehmbares Salz davon und einen pharmazeutisch annehmbaren Träger umfasst.

15. Die pharmazeutische Zusammensetzung nach Anspruch 14, die ferner wenigstens ein zusätzliches therapeutisch wirksames Mittel umfasst.

16. Die Verbindung nach Anspruch 1 oder ein pharmazeutisch annehmbares Salz davon zur Verwendung in der Therapie.

17. Die Verbindung nach Anspruch 1 oder ein pharmazeutisch annehmbares Salz davon zur Verwendung bei der Behandlung von Autoimmun- und Entzündungserkrankungen.

## Revendications

1. Composé de la Formule (I): 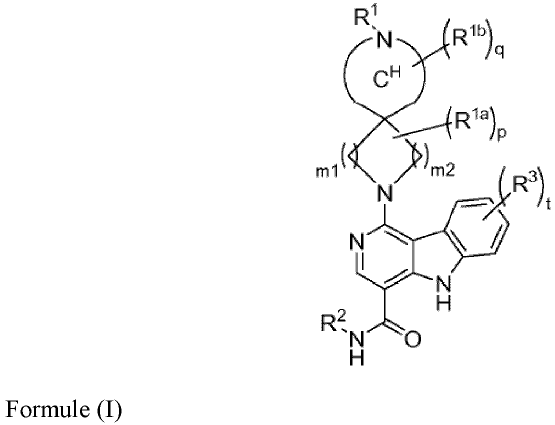 où:
le cyle C^{H} est un cycle hétérocylique saturé de 4 à 6 chaînons, où ledit cycle hétérocyclique contient optionnellement un groupe d'hétéroatome supplémentaire qui est N(H) ou O;
le ml et le m2 en indice sont indépendamment 0, 1, 2, ou 3, à condition que 2 ≤ m1 + m2 ≤ 5;
R¹ est:
(a.) H;
(b.) alkyle C₁₋₃, où ledit alkyle C₁₋₃ de R¹ est non substitué ou substitué par -C(O)OR^{1e}, où R^{1e} est H ou alkyle C₁₋₆;
(c.) -C(O)O-R^{1c} où R^{1c} est un alkyle C₁₋₆;
(d.) -C(O)O-R^{1d} où R^{1d} est un alkyle C₁₋₃ ou un phényle, où ledit phényle de R^{1d} est non substitué ou substitué par 1 à 2 substituants sélectionnés parmi alkyle C₁₋₆, halo ou alcoxy C₁₋₃; ou bien
(e.) phényle, où ledit phényle de R¹ est non substitué ou substitué par 1 à 2 substituants sélectionnés parmi alkyle C₁₋₆, halo ou alcoxy C₁₋₃;
R^{1a} est un alkyle C₁₋₃, fluoroalkyle C₁₋₃, alcoxy C₁₋₃ ou fluoro;
R^{1b} est un hydroxyle, alkyle C₁₋₃, hydroxyalkyle C₁₋₃, fluoroalkyle C₁₋₃, alcoxy C₁₋₃, fluoro ou phényle; ou bien optionnellement deux fractions R^{1b} lorsque substituées sur un atome de carbone cyclique commun forment un carbonyle avec ledit atome de carbone;
R² est H ou OH;
R³ est
(a.) halo;
(b.) alkyle C₁₋₃;
(c.) -C(H)(OH)-CH₂OH;
(d.) -CO₂R^{3a}, où R^{3a} est H ou un alkyle C₁₋₃;
(e.) un cycle Cⁱ, où Cⁱ est
(i.) phényle;
(ii.) cycloalkyle de 3 à 6 chaînons;
(iii.) hétéroaryle de 5 à 6 chaînons contenant 1 ou 2 hétéroatomes sélectionnés parmi N, O ou S; ou bien
(iv.) hétérocyclyle saturé de 5 à 6 chaînons contenant 1 à 2 atomes N;
où le cycle Cⁱ est non substitué ou substitué par 1 à 4 R^{3b}, où R^{3b} est sélectionné parmi alkyle C₁₋₆, fluoroalkyle C₁₋₆, hydroxyalkyle C₁₋₆, alcoxy C₁₋₃, halo, -C(O)O-alkyle C₁₋₆,-C(O)OH, -C(O)alkyle C₁₋₆, -C(O)N(H)-alkyle C₁₋₃, -CH₂C(O)OH, -CH₂C(O)O-alkyle C₁₋₃,-S(O)₂-alkyle C₁₋₃, ou -C(H)(OH)-fluoroalkyle C₁₋₃;
ou bien optionnellement deux fractions R^{3b} lorsque substituées sur un atome de carbone cyclique commun, forment un carbonyle avec ledit atome de carbone;
p en indice est 0, 1 ou 2;
q en indice est 0, 1, 2, 3 ou 4;
t en indice est 0 ou 1; ou
un sel pharmaceutiquement acceptable de celui-ci.

2. Composé selon la revendication 1 ou un sel pharmaceutiquement acceptable de celui-ci, dans lequel 2 ≤ m1 + m2 ≤ 4.

3. Composé selon la revendication 1 ou un sel pharmaceutiquement acceptable de celui-ci, dans lequel le cycle C^{H} est un pyrrolidine ou pipéridine non substitué ou substitué.

4. Composé selon la revendication 1 ou un sel pharmaceutiquement acceptable de celui-ci, dans lequel le groupe

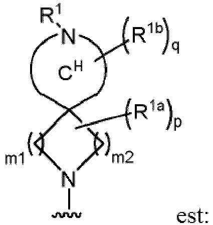

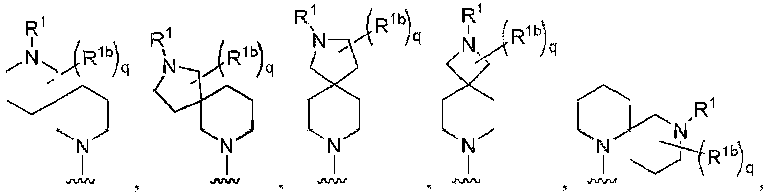

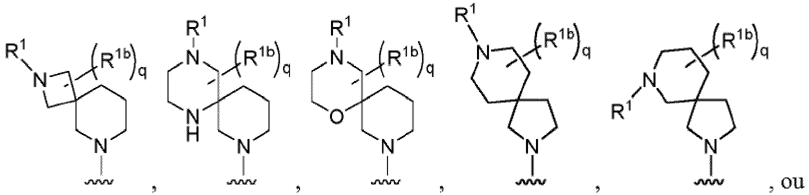

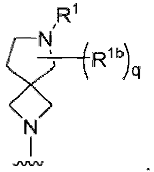

5. Composé selon la revendication 1 ou un sel pharmaceutiquement acceptable de celui-ci, dans lequel R¹ est H, méthyle, -C(O)CH₃, phényle non substitué ou phényle substitué par 1 à 2 fluoro ou alkyle C₁₋₄.

6. Composé selon la revendication 1 ou un sel pharmaceutiquement acceptable de celui-ci, dans lequel R^{1b} est un hydroxyle, phényle, -CH₂OH, ou bien optionnellement deux fractions R^{1b} lorsque substituées sur un atome de carbone cyclique commun forment un carbonyle avec ledit atome de carbone.

7. Composé selon la revendication 1 ou un sel pharmaceutiquement acceptable de celui-ci, dans lequel R² est H.

8. Composé selon la revendication 1 ou un sel pharmaceutiquement acceptable de celui-ci, dans lequel le t en indice est 0.

9. Composé selon la revendication 1 ou un sel pharmaceutiquement acceptable de celui-ci, dans lequel le t en indice est 1.

10. Composé selon la revendication 9 ou un sel pharmaceutiquement acceptable de celui-ci, dans lequel R³ est substitué sur la 7^{e} ou 8^{e} position du cycle gamma-carboline illustré de la Formule (I).

11. Composé selon la revendication 9 ou un sel pharmaceutiquement acceptable de celui-ci, dans lequel R³ est un fluoro, -C(O)OH, -C(O)OCH₃, -C(H)(OH)-CH₂OH, ou un groupe sélectionné parmi:

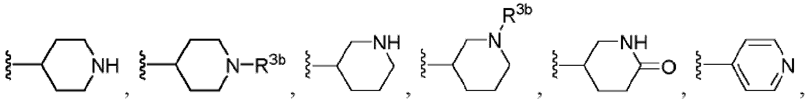

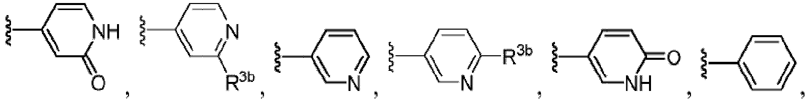

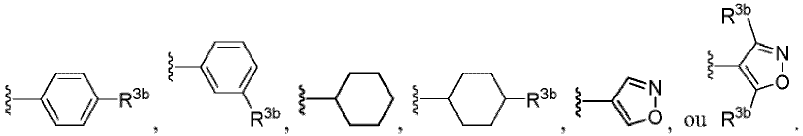

12. Composé selon la revendication 11 ou un sel pharmaceutiquement acceptable de celui-ci, dans lequel R^{3b} est -CH₃, -CH₂CH₃, -C(H)(CH₃)₂, -OCH₃, -C(O)OH, -C(O)OCH₃,-C(O)OC(CH₃)₃, -C(O)N(H)CH₃, -CH₂C(O)CH₃, -CH₂C(OH)(CH₃)₂, -C(H)(OH)(CF₃),-S(O)₂CH₃ ou -C(O)CH₃.

13. Composé selon la revendication 1 qui est du:
1-[(6R)-8-(4-fluorophényl)-7-oxo-2,8-diazaspiro[5.5]undéc-2-yl]-5H-pyrido[4,3-b]indole-4-carboxamide;
1-[(6R)-8-(4-fluorophényl)-7-oxo-2,8-diazaspiro[5.5]undéc-2-yl]-5H-pyrido[4,3-b]indole-4-carboxamide;
1-[4-(4-fluorophényl)-5-oxo-1-oxa-4,8-diazaspiro[5.5]undéc-8-yl]-5H-pyrido[4,3-b]indole-4-carboxamide;
1-[8-(4-fluorophényl)-7-oxo-2,8-diazaspiro[5.5]undéc-2-yl]-5H-pyrido[4,3-b]indole-4-carboxamide;
1-{8-[4-(1-méthyléthyl)phényl]-7-oxo-2,8-diazaspiro[5.5]undéc-2-yl}-5H-pyrido[4,3-b]indole-4-carboxamide;
8-fluoro-1-[(6R)-8-(4-fluorophényl)-7-oxo-2,8-diazaspiro[5.5]undéc-2-yl]-5H-pyrido[4,3-b]indole-4-carboxamide;
8-fluoro-1-[(6S)-8-(4-fluorophényl)-7-oxo-2,8-diazaspiro[5.5]undéc-2-yl]-5H-pyrido[4,3-b]indole-4-carboxamide;
Acide 4-carbamoyl-1-[(6R)-8-(4-fluorophényl)-7-oxo-2,8-diazaspiro[5.5]undéc-2-yl]-5H-pyrido[4,3-b]indole-8-carboxylique;
N-hydroxy-1-(1-oxo-2,7-diazaspiro[4.5]déc-7-yl)-5H-pyrido[4,3-b]indole-4-carboxamide;
1-(3-oxo-2,8-diazaspiro[4.5]déc-8-yl)-5H-pyrido[4,3-b]indole-4-carboxamide;
1-(1-oxo-2,8-diazaspiro[4.5]déc-8-yl)-5H-pyrido[4,3-b]indole-4-carboxamide;
1-(2,8-diazaspiro[4.5]déc-8-yl)-5H-pyrido[4,3-b]indole-4-carboxamide;
8-(4-carbamoyl-5H-pyrido[4,3-b]indol-1-yl)-2,8-diazaspiro[4.5]decane-2-carboxylate de *tert*-butyle;
1-(2-acétyl-2,8-diazaspiro[4.5]déc-8-yl)-5H-pyrido[4,3-b]indole-4-carboxamide;
1-(2,8-diazaspiro[4.5]déc-2-yl)-5H-pyrido[4,3-b]indole-4-carboxamide;
1-(7-oxa-2-azaspiro[3.5]non-2-yl)-5H-pyrido[4,3-b]indole-4-carboxamide;
7-(4-carbamoyl-5H-pyrido[4,3-b]indol-1-yl)-2,7-diazaspiro[3.5]nonane-2-carboxylate de *tert*-butyle;
1-[(4S,5R)-4-hydroxy-1-oxo-2,7-diazaspiro[4.5]déc-7-yl]-5H-pyrido[4,3-b]indole-4-carboxamide;
1-[(4R,5S)-4-hydroxy-1-oxo-2,7-diazaspiro[4.5]déc-7-yl]-5H-pyrido[4,3-b]indole-4-carboxamide;
1-(2,7-diazaspiro[3.5]non-7-yl)-5H-pyrido[4,3-b]indole-4-carboxamide;
1-(2-acétyl-2,7-diazaspiro[3.5]non-7-yl)-5H-pyrido[4,3-b]indole-4-carboxamide;
2-(4-carbamoyl-5H-pyrido[4,3-b]indol-1-yl)-2,6-diazaspiro[3.4]octane-6-carboxylate de *tert*-butyle;
1-[(4S,5S)-4-hydroxy-1-oxo-2,7-diazaspiro[4.5]déc-7-yl]-5H-pyrido[4,3-b]indole-4-carboxamide;
1-[(4S,5S)-4-hydroxy-1-oxo-2,7-diazaspiro[4.5]déc-7-yl]-5H-pyrido[4,3-b]indole-4-carboxamide;
1-[(4R,5R)-4-hydroxy-1-oxo-2,7-diazaspiro[4.5]déc-7-yl]-5H-pyrido[4,3-b]indole-4-carboxamide;
1-{8-[(4-*tert*-butylphényl)carbonyl]-1,8-diazaspiro[5.5]undéc-1-yl}-5H-pyrido[4,3-b]indole-4-carboxamide;
1-(2,6-diazaspiro[3.4]oct-2-yl)-5H-pyrido[4,3-b]indole-4-carboxamide;
1-(6-acétyl-2,6-diazaspiro[3.4]oct-2-yl)-5H-pyrido[4,3-b]indole-4-carboxamide;
6-(4-carbamoyl-5H-pyrido[4,3-b]indol-1-yl)-2,6-diazaspiro[3.5]nonane-2-carboxylate de *tert*-butyle;
1-(2-acétyl-2,6-diazaspiro[3.5]non-6-yl)-5H-pyrido[4,3-b]indole-4-carboxamide;
1-(7-oxo-2,8-diazaspiro[5.5]undéc-2-yl)-5H-pyrido[4,3-b]indole-4-carboxamide;
3-[8-(4-carbamoyl-5H-pyrido[4,3-b]indol-1-yl)-1-oxo-2,8-diazaspiro[5.5]undéc-2-yl]propanoate de méthyle;
1-[2-(4-fluorophényl)-1-oxo-2,7-diazaspiro[4.5]déc-7-yl]-5H-pyrido[4,3-b]indole-4-carboxamide;
1-[7-(4-fluorophényl)-6-oxo-2,7-diazaspiro[4.5]déc-2-yl]-5H-pyrido[4,3-b]indole-4-carboxamide;
1-(1-oxo-2,7-diazaspiro[4.5]déc-7-yl)-5H-pyrido[4,3-b]indole-4-carboxamide;
1-(8-méthyl-7-oxo-2,8-diazaspiro[5.5]undéc-2-yl)-5H-pyrido[4,3-b]indole-4-carboxamide;
1-[2-(4-*tert*-butylphényl)-1-oxo-2,7-diazaspiro[4.5]déc-7-yl]-5H-pyrido[4,3-b]indole-4-carboxamide;
1-(8-oxo-2,9-diazaspiro[5.5]undéc-2-yl)-5H-pyrido[4,3-b]indole-4-carboxamide;
1-[9-(4-*tert*-butylphényl)-8-oxo-2,9-diazaspiro[5.5]undéc-2-yl]-5H-pyrido[4,3-b]indole-4-carboxamide;
1-[8-(1-méthyléthyl)-7-oxo-2,8-diazaspiro[5.5]undéc-2-yl]-5H-pyrido[4,3-b]indole-4-carboxamide;
1-[(5R)-1-oxo-2,7-diazaspiro[4.5]déc-7-yl]-5H-pyrido[4,3-b]indole-4-carboxamide;
1-[(5S)-1-oxo-2,7-diazaspiro[4.5]déc-7-yl]-5H-pyrido[4,3-b]indole-4-carboxamide;
1-(1-oxo-2,7-diazaspiro[4.5]déc-7-yl)-7-pipéridin-4-yl-5H-pyrido[4,3-b]indole-4-carboxamide;
4-[4-carbamoyl-1-(1-oxo-2,7-diazaspiro[4.5]déc-7-yl)-5H-pyrido[4,3-b]indol-7-yl]pipéridine-1-carboxylate de *tert*-butyle;
1-(1-oxo-2,7-diazaspiro[4.5]déc-7-yl)-7-pipéridin-3-yl-5H-pyrido[4,3-b]indole-4-carboxamide;
1-(1-oxo-2,7-diazaspiro[4.5]déc-7-yl)-8-pipéridin-4-yl-5H-pyrido[4,3-b]indole-4-carboxamide;
4-[4-carbamoyl-1-(1-oxo-2,7-diazaspiro[4.5]déc-7-yl)-5H-pyrido[4,3-b]indol-8-yl]pipéridine-1-carboxylate de *tert*-butyle;
8-[1-(méthylcarbamoyl)pipéridin-4-yl]-1-(1-oxo-2,7-diazaspiro[4.5]déc-7-yl)-5H-pyrido[4,3-b]indole-4-carboxamide;
Acide {4-[4-carbamoyl-1-(1-oxo-2,7-diazaspiro[4.5]déc-7-yl)-5H-pyrido[4,3-b]indol-8-yl]pipéridin-1-yl}acétique;
8-(1-éthylpipéridin-4-yl)-1-(1-oxo-2,7-diazaspiro[4.5]déc-7-yl)-5H-pyrido[4,3-b]indole-4-carboxamide;
8-[1-(1-méthyléthyl)pipéridin-4-yl]-1-(1-oxo-2,7-diazaspiro[4.5]déc-7-yl)-5H-pyrido[4,3-b]indole-4-carboxamide;
4-[4-carbamoyl-1-(1-oxo-2,7-diazaspiro[4.5]déc-7-yl)-5H-pyrido[4,3-b]indol-8-yl]pipéridine-1-carboxylate de méthyle;
7-[1-(1-méthyléthyl)pipéridin-4-yl]-1-(1-oxo-2,7-diazaspiro[4.5]déc-7-yl)-5H-pyrido[4,3-b]indole-4-carboxamide;
7-[1-(méthylcarbamoyl)pipéridin-4-yl]-1-(1-oxo-2,7-diazaspiro[4.5]déc-7-yl)-5H-pyrido[4,3-b]indole-4-carboxamide;
7-[1-(2-hydroxy-2-méthylpropyl)pipéridin-4-yl]-1-(1-oxo-2,7-diazaspiro[4.5]déc-7-yl)-5H-pyrido[4,3-b]indole-4-carboxamide;
7-(1-méthylpipéridin-4-yl)-1-(1-oxo-2,7-diazaspiro[4.5]déc-7-yl)-5H-pyrido[4,3-b]indole-4-carboxamide;
4-[4-carbamoyl-1-(1-oxo-2,7-diazaspiro[4.5]déc-7-yl)-5H-pyrido[4,3-b]indol-7-yl]pipéridine-1-carboxylate de méthyle;
7-[1-(méthylsulfonyl)pipéridin-4-yl]-1-(1-oxo-2,7-diazaspiro[4.5]déc-7-yl)-5H-pyrido[4,3-b]indole-4-carboxamide;
7-(1-acétylpipéridin-4-yl)-1-(1-oxo-2,7-diazaspiro[4.5]déc-7-yl)-5H-pyrido[4,3-b]indole-4-carboxamide;
Acide {4-[4-carbamoyl-1-(1-oxo-2,7-diazaspiro[4.5]déc-7-yl)-5H-pyrido[4,3-b]indol-7-yl]pipéridin-1-yl}acétique;
8-(1-méthylpipéridin-4-yl)-1-(1-oxo-2,7-diazaspiro[4.5]déc-7-yl)-5H-pyrido[4,3-b]indole-4-carboxamide;
8-(1-acétylpipéridin-4-yl)-1-(1-oxo-2,7-diazaspiro[4.5]déc-7-yl)-5H-pyrido[4,3-b]indole-4-carboxamide;
8-[1-(2-hydroxy-2-méthylpropyl)pipéndin-4-yl]-1-(1-oxo-2,7-diazaspiro[4.5]déc-7-yl)-5H-pyrido[4,3-b]indole-4-carboxamide;
1-[(5S)-1-oxo-2,7-diazaspiro[4.5]déc-7-yl]-7-pipéridin-4-yl-5H-pyrido[4,3-b]indole-4-carboxamide;
1-[(SR)-1-oxo-2,7-diazaspiro[4.5]déc-7-yl]-7-pipéridin-4-yl-5H-pyrido[4,3-b]indole-4-carboxamide;
8-(1,2-dihydroxyéthyl)-1-(1-oxo-2,7-diazaspiro[4.5]déc-7-yl)-5H-pyrido[4,3-b]indole-4-carboxamide;
8-[1-(méthylsulfonyl)pipéridin-4-yl]-1-(1-oxo-2,7-diazaspiro[4.5]déc-7-yl)-5H-pyrido[4,3-b]indole-4-carboxamide;
7-(6-méthoxypyridin-3-yl)-1-[(5R)-1-oxo-2,7-diazaspiro[4.5]déc-7-yl]-5H-pyrido[4,3-b]indole-4-carboxamide;
1-[(5R)-1-oxo-2,7-diazaspiro[4.5]déc-7-yl]-7-(6-oxo-1,6-dihydropyridin-3-yl)-5H-pyrido[4,3-b]indole-4-carboxamide;
7-(2-méthoxypyridin-4-yl)-1-[(5R)-1-oxo-2,7-diazaspiro[4.5]déc-7-yl]-5H-pyrido[4,3-b]indole-4-carboxamide;
1-[(5R)-1-oxo-2,7-diazaspiro[4.5]déc-7-yl]-7-(2-oxo-1,2-dihydropyridin-4-yl)-5H-pyrido[4,3-b]indole-4-carboxamide;
7-[1-(1-méthyléthyl)pipéridin-4-yl]-1-[(5R)-1-oxo-2,7-diazaspiro[4.5]déc-7-yl]-5H-pyrido[4,3-b]indole-4-carboxamide;
7-[1-(2-hydroxy-2-méthylpropyl)pipéridin-4-yl]-1-[(5R)-1-oxo-2,7-diazaspiro[4.5]déc-7-yl]-5H-pyrido[4,3-b]indole-4-carboxamide;
1-[(5R)-1-oxo-2,7-diazaspiro[4.5]déc-7-yl]-7-(6-oxopipéridin-3-yl)-4H-pyrido[4,3-b]indole-4-carboxamide;
7-(6-méthoxypyridin-3-yl)-1-[(5R)-2-méthyl-1-oxo-2,7-diazaspiro[4.5]déc-7-yl]-5H-pyrido[4,3-b]indole-4-carboxamide;
7-(2-méthoxypyridin-4-yl)-1-[(5R)-2-méthyl-1-oxo-2,7-diazaspiro[4.5]déc-7-yl]-5H-pyrido[4,3-b]indole-4-carboxamide;
7-[1-(1-méthyléthyl)pipéridin-4-yl]-1-[(5R)-2-méthyl-1-oxo-2,7-diazaspiro[4.5]déc-7-yl]-5H-pyrido[4,3-b]indole-4-carboxamide;
7-[1-(2-hydroxy-2-méthylpropyl)pipéridin-4-yl]-1-[(5R)-2-méthyl-1-oxo-2,7-diazaspiro[4.5]déc-7-yl]-5H-pyrido[4,3-b]indole-4-carboxamide;
1-[(5R)-2-méthyl-1-oxo-2,7-diazaspiro[4.5]déc-7-yl]-7-[4-(2,2,2-trifluoro-1-hydroxyéthyl)phényl]-5H-pyrido[4,3-b]indole-4-carboxamide;
1-[(5R)-2-méthyl-1-oxo-2,7-diazaspiro[4.5]déc-7-yl]-7-[3-(2,2,2-trifluoro-1-hydroxyéthyl)phényl]-5H-pyrido[4,3-b]indole-4-carboxamide;
7-(3,5-diméthylisoxazol-4-yl)-1-[(5R)-2-méthyl-1-oxo-2,7-diazaspiro[4.5]déc-7-yl]-5H-pyrido[4,3-b]indole-4-carboxamide;
(4-{4-carbamoyl-1-[(5R)-2-méthyl-1-oxo-2,7-diazaspiro[4.5]déc-7-yl]-5H-pyrido[4,3-b]indol-7-yl}cyclohexyl)acétate de méthyle;
7-[4-(2-hydroxy-2-méthylpropyl)cyclohexyl]-1-[(5R)-2-méthyl-1-oxo-2,7-diazaspiro[4.5]déc-7-yl]-5H-pyrido[4,3-b]indole-4-carboxamide,
4-carbamoyl-1-(1-oxo-2,7-diazaspiro[4.5]déc-7-yl)-5H-pyrido[4,3-b]indole-7-carboxylate de méthyle;
Acide 4-carbamoyl-1-(1-oxo-2,7-diazaspiro[4.5]déc-7-yl)-5H-pyrido[4,3-b]indole-8-carboxylique;
Acide 4-carbamoyl-1-(1-oxo-2,7-diazaspiro[4.5]déc-7-yl)-5H-pyrido[4,3-b]indole-7-carboxylique;
1-(4-hydroxy-1-oxo-2,7-diazaspiro[4.5]déc-7-yl)-5H-pyrido[4,3-b]indole-4-carboxamide;
1-(5-oxo-1,4,8-triazaspiro[5.5]undéc-8-yl)-5H-pyrido[4,3-b]indole-4-carboxamide;
1-[(4R,5R)-1-oxo-4-phényl-2,7-diazaspiro[4.5]déc-7-yl]-5H-pyrido[4,3-b]indole-4-carboxamide;
1-[(4R,5S)-1-oxo-4-phényl-2,7-diazaspiro[4.5]déc-7-yl]-5H-pyrido[4,3-b]indole-4-carboxamide;
1-[(4S,5S)-4-(hydroxyméthyl)-2-méthyl-1-oxo-2,7-diazaspiro[4.5]déc-7-yl]-5H-pyrido[4,3-b]indole-4-carboxamide;
1-[(4R,5S)-4-(hydroxyméthyl)-2-méthyl-1-oxo-2,7-diazaspiro[4.5]déc-7-yl]-5H-pyrido[4,3-b]indole-4-carboxamide; ou
un sel pharmaceutiquement acceptable de ceux-ci.

14. Composition pharmaceutique comprenant le composé selon la revendication 1 ou un sel pharmaceutiquement acceptable de celui-ci et un véhicule pharmaceutiquement acceptable.

15. Composition pharmaceutique selon la revendication 14, qui comprend en outre au moins un agent thérapeutiquement actif supplémentaire.

16. Composé selon la revendication 1 ou un sel pharmaceutiquement acceptable de celui-ci, à utiliser en thérapie.

17. Composé selon la revendication 1 ou un sel pharmaceutiquement acceptable de celui-ci à utiliser dans le traitement de maladies auto-immunes ou inflammatoires.


### SEQUENCE LISTING

<110> Merck Sharp & Dohme Corp.
<120> AZACARBAZOLE BTK INHIBITORS
<130> 24057-WO-PCT2
<150> PCT/CN2015/076040
   <151> 2015-04-08
<160> 1
<170> Patent In version 3.5
<210> 1
   <211> 15
   <212> PRT
   <213> Artificial Sequence
<220>
   <223> Completely Synthetic Amino Acid Sequence
<400> 1
   Glu Gln Glu Asp Glu Pro Glu Gly Asp Tyr Phe Glu Trp Leu Glu
   1               5                   10                  15